(19) Europäisches Patentamt
European Patent Office
Office européen des brevets

(11) Veröffentlichungsnummer: **0 407 955 A1**

# (12) EUROPÄISCHE PATENTANMELDUNG

(21) Anmeldenummer: **90113130.0**

(22) Anmeldetag: **10.07.90**

(51) Int. Cl.[5]: **C07D 495/14**, C07D 495/04, C07C 255/40, A61K 31/55, //(C07D495/14,333:00,243:00, 235:00),(C07D495/14,333:00, 249:00,243:00)

(30) Priorität: **12.07.89 DE 3922944**
**07.04.90 DE 4011345**

(43) Veröffentlichungstag der Anmeldung:
**16.01.91 Patentblatt 91/03**

(84) Benannte Vertragsstaaten:
**AT BE CH DE DK ES FR GB GR IT LI LU NL SE**

(71) Anmelder: **BOEHRINGER INGELHEIM KG**
**Postfach 200**
**D-6507 Ingelheim am Rhein(DE)**
(84) **BE**

Anmelder: **BOEHRINGER INGELHEIM INTERNATIONAL GmbH**
**Postfach 20**
**D-6507 Ingelheim am Rhein(DE)**
(84) **GB**

(72) Erfinder: **Weber, Karl-Heinz, Dr.**
**Kaiser-Karl-Strasse 11**
**D-6535 Gau-Algesheim(DE)**
Erfinder: **Stransky, Werner, Dr.**
**Im Hippel 24**
**D-6535 Gau-Algesheim(DE)**
Erfinder: **Walther, Gerhard, Dr.**

**Verstorben(DE)**
Erfinder: **Küfner-Mühl, Ulrike, Dr.**
**Am Rübenacker 5**
**D-6500 Mainz(DE)**
Erfinder: **Heuer, Hubert, Dr.**
**Am Sportfeld 74**
**D-6501 Schwabenheim(DE)**
Erfinder: **Birke, Franz, Dr.**
**Albrecht-Dürer-Strasse 23**
**D-6507 Ingelheim am Rhein(DE)**
Erfinder: **Muacevic, Gojko, Dr.**
**In der Dörrwiese 13**
**D-6507 Ingelheim am Rhein(DE)**
Erfinder: **Bechtel, Wolf-Dietrich, Dr.**
**Mühlstrasse 3**
**D-6531 Appenheim(DE)**

## (54) Neue Hetrazepine mit PAF-antagonistischer Wirkung, Verfahren zu ihrer Herstellung und ihre Verwendung als Arzneimittel.

(57) Die Erfindung betrifft Hetrazepinoide PAF-Antagonisten mit ungewöhnlichen Substitutionsmuster, Verfahren zu ihrer Herstellung sowie ihrer Verwendung als Arzneimittel.

EP 0 407 955 A1

# NEUE HETRAZEPINE MIT PAF-ANTAGONISTISCHER WIRKUNG, VERFAHREN ZU IHRER HERSTELLUNG UND IHRE VERWENDUNG ALS ARZNEIMITTEL

Die vorliegende Erfindung betrifft neue Diazepine, Verfahren zu ihrer Herstellung sowie ihre Verwendung als Arzneimittel mit PAF-antagonistischer Wirkung.

In den vergangenen Jahren - insbesondere seit 1984 -wurden zahlreiche Verbindungen synthetisiert die als PAF-Antagonisten bezeichnet werden. Der Begriff PAF-Antagonist ist mittlerweile in der Literatur eingehend beschrieben und definiert.

Eine Substanzklasse, die sogenannten Thienodiazepine der allgemeinen Formel 1

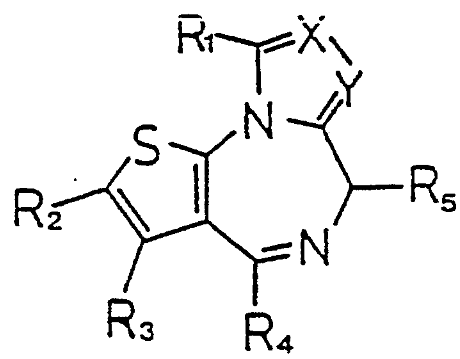

1

erwiesen sich hierbei als besonders wirksame Verbindungen. Trotz vielfacher Substitutionsmuster und unterschiedlichster funktioneller Gruppen weisen nahezu alle bekannten PAF-Antagonisten des genannten Stukturtyps eine Phenylgruppe bzw. eine ortho-Chlorphenylgruppe in der 4- bzw. 6-Position ($R_4$) des zentralen 1,4-Diazpinringes auf. Sowohl der unsubstituierte Phenylring wie auch die bisher bekannten anders substituierten Phenyle - wie beispielsweise methyl- oder methoxy-substituiert -weisen eine geringere Wirkungsstärke auf. Überraschenderweise wurde nunmehr ein Strukturelement gefunden, das - als Substituent $R_4$ in der 4- bzw. 6-Position eines PAF-Antagonisten mit einem zentral angeordneten 1,4-Diazepin die Wirkungsstärke des PAF-Antagonisten entscheidend verbessert.

Der neue Substituent $R_4$ entspricht der allgemeinen Formel

$-S_K - W_L - T_M - V_N - U_M - P_N - Z_\alpha$

worin

S eine Einfachbindung, verzweigtes oder unverzweigtes Alkyl mit 1 bis 8 - bevorzugt 1 bis 4 - Kohlenstoffatom(en), gegebenenfalls durch Halogen und/oder Hydroxy substituiert, verzweigtes oder unverzweigtes Alkenyl mit 2 bis 8 Kohlenstoffatomen - bevorzugt mit 2 bis 4 Kohlenstoffatomen, gegebenenfalls durch Halogen und/oder Hydroxy substituiert, verzweigtes oder unverzweigtes Alkinyl mit 2 bis 8 Kohlenstoffatomen - bevorzugt mit 2 bis 4 Kohlenstoffatomen, gegebenenfalls durch Halogen und/oder Hydroxy substituiert;

W einen gegebenenfalls substituierter aromatischer Ring mit 6 bis 10 Kohlenstoffatomen, der gegebenenfalls ein oder mehrere Heteroatome aus der Gruppe Stickstoff, Sauerstoff oder Schwefel enthalten kann, einen gegebenenfalls substituierten carbocyclischen Ring mit 3 bis 7 Kohlenstoffatomen, der auch eine Doppelbindung enthalten kann;

T verzweigtes oder unverzweigtes Alkyl gegebenenfalls substituiert durch Halogen oder Hydroxy mit 1 bis 8 - bevorzugt 1 bis 4 -Kohlenstoffatom(en); verzweigtes oder unverzweigtes Alkenyl mit 2 bis 8 Kohlenstoffatomen - bevorzugt 2 bis 4 Kohlenstoffatomen - gegebenenfalls durch Halogen und/oder Hydroxy substituiert; verzweigtes oder unverzweigtes Alkinyl mit 2 bis 8 Kohlenstoffatomen - bevorzugt 2 bis 4 Kohlenstoffatomen - gegebenenfalls durch Halogen und/oder Hydroxy substituiert; einen Rest der Formel

$- (F)_{0-1} - E - (F)_{0-1} -$

$E = O, S, N-R_6$,

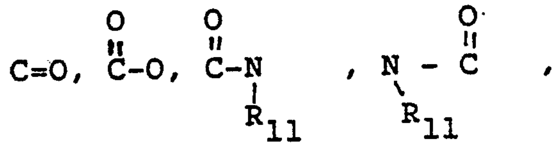

worin $R_6$ wie zuvor definiert, bevorzugt Wasserstoff, $C_1$ - $C_4$-Alkyl, oder

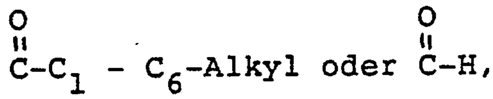

und $R_{11}$ wie zuvor definiert, bevorzugt Wasserstoff oder $C_1$ - $C_4$ -Alkyl;

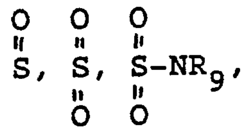

worin $R_9$ wie zuvor definiert,
F eine verzweigte oder unverzweigte gegebenenfalls substituierte Alkylgruppe mit 1 bis 8 - bevorzugt 1 bis 4 - Kohlenstoffatom(en), eine gegebenenfalls substituierte Alkenylgruppe mit 2 bis 8 -bevorzugt 2 bis 4 - Kohlenstoffatomen, eine gegebenenfalls substituierte Alkinylgruppe mit 2 bis 8 - bevorzugt 2 bis 4 Kohlenstoffatomen;
V einen gegebenenfalls substituierten aromatischen Ring mit 6 bis 10 Kohlenstoffatomen, der gegebenenfalls ein oder mehrere Heteroatome aus der Gruppe Stickstoff, Sauerstoff oder Schwefel enthalten kann, einen gegebenenfalls substituierten carbocyclischen Ring mit 3 bis 7 Kohlenstoffatomen, der auch eine Doppelbindung enthalten kann,
ein cyclisches Amid oder Imid, das 5, 6 oder 7 Ringglieder aufweisen kann und in dem gegebenenfalls ein Kohlenstoffatom durch ein weiteres Heteroatom - wie z.B. Stickstoff, Sauerstoff oder Schwefel - ersetzt sein kann und das gegebenenfalls durch einen oder mehrere Arylrest(e) anneliert sein kann;
U verzweigtes oder unverzweigtes Alkyl gegebenenfalls substituiert durch Halogen oder Hydroxy mit 1 bis 8 - bevorzugt 1 bis 4 -Kohlenstoffatom(en); verzweigtes oder unverzweigtes Alkenyl mit 2 bis 8 Kohlenstoffatomen - bevorzugt 2 bis 4 Kohlenstoffatomen - gegebenenfalls durch Halogen und/oder Hydroxy substituiert, verzweigtes oder unverzweigtes Alkinyl mit 2 bis 8 Kohlenstoffatomen - bevorzugt 2 bis 4 Kohlenstoffatomen - gegebenenfalls durch Halogen und/oder Hydroxy substituiert, einen Rest der Formel
$-(F)_{0-1}-E-(F)_{0-1}-$
E = O, S, N-$R_6$,

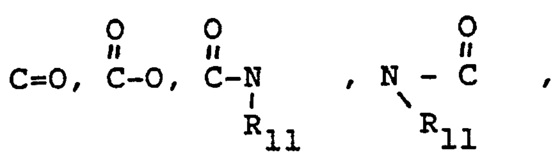

worin $R_6$ wie zuvor definiert, bevorzug Wasserstoff, $C_1$ - $C_4$-Alkyl, oder

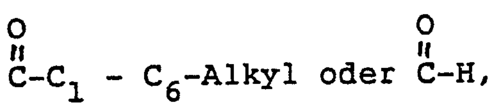

und $R_{11}$ wie zuvor definiert, bevorzugt Wasserstoff oder $C_1$ - $C_4$ -Alkyl;

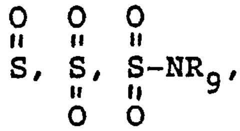

worin $R_9$ wie zuvor definiert ist,

F eine verzweigte oder unverzweigte gegebenenfalls substituierte Alkylgruppe mit 1 bis 8 - bevorzugt 1 bis 4 - Kohlenstoffatom(en), eine gegebenenfalls substituierte Alkenylgruppe mit 2 bis 8 -bevorzugt 2 bis 4 - Kohlenstoffatomen, eine gegebenenfalls substituierte Alkinylgruppe mit 2 bis 8 - bevorzugt 2 bis 4 Kohlenstoffatomen;

P einen gegebenenfalls substituierten aromatischen Ring mit 6 bis 10 Kohlenstoffatomen, der gegebenenfalls ein oder mehrere Heteroatome aus der Gruppe Stickstoff, Sauerstoff oder Schwefel enthalten kann, einen gegebenenfalls substituierten carbocyclischen Ring mit 3 bis 7 Kohlenstoffatomen, der auch eine Doppelbindung enthalten kann, wobei W, V und/oder P ein- oder mehrfach durch Z substituiert sein kann, ein cyclisches Amid oder Imid, das 5, 6 oder 7 Ringglieder aufweisen kann und in dem gegebenenfalls ein Kohlenstoffatom durch ein weiteres Heteroatom wie z.B. Stickstoff, Sauerstoff oder Schwefel - ersetzt sein kann und das gegebenenfalls durch einen oder mehrere Arylrest(e) anneliert sein kann;

Z Wasserstoff, Halogen, Hydroxy, Nitro, Cyano, Trifluormethyl, gegebenenfalls durch Halogen oder Hydroxy substituiertes, verzweigtes oder unverzweigtes Alkyl mit 1 bis 8 - bevorzugt 1 bis 4 - Kohlenstoffatom(en), gegebenenfalls substituiertes, verzweigtes Alkoxy mit 1 bis 8 bevorzugt 1 bis 4 Kohlenstoffatom(en), einen Rest der Formel

$$-(F')_{0-1} - E' - (F')_{0-1} - H,\ (F')_{0-1} - NO_2,$$

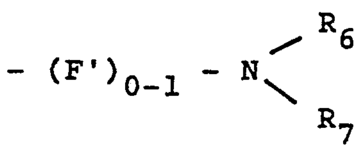 , 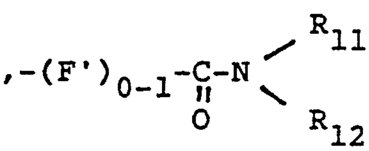

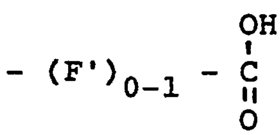

,

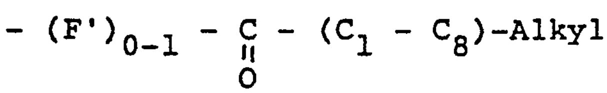

bevorzugt $C_1 - C_4$-Alkyl

worin F′ eine verzweigte oder unverzweigte Alkylgruppe mit 1 bis 4 Kohlenstoffatomen, bevorzugt 1 bis 2 Kohlenstoffatomen und

E′ = O, S, N-$R_6$,

C = O, 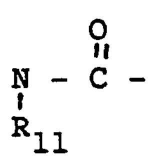 , 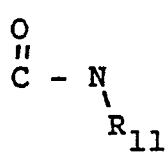

wobei bei $\alpha > 1$ alle Z gleich, teilweise gleich oder verschieden sein können,
K = 0, 1,
L = 0, 1, 2,
M = 0, 1,
N = 0, 1
und $\alpha$ = 1, 2, 3, 4, 5 bedeuten können und E die unter T angegebene Bedeutung hat, wobei die Summe K + L + M + N ungleich 0 ist und
$-S_K - W_L - T_M - V_N - U_M - P_N - Z_\alpha$
insgesamt nicht einen Phenylrest, einen Phenylrest, der mit Halogen, $CH_3$, $CF_3$ und $NO_2$ substituiert ist, einen Pyridyl- oder einen Thienylrest bedeuten kann.

Gegenstand der Erfindung sind somit PAF-Antagonisten mit einem 1,4-Diazepingerüst, das in der 4- bzw. 6-Position den zuvor definierten Substituenten $R_4$ trägt.

Die neuen Hetrazepine entsprechen der allgemeinen Formel 1

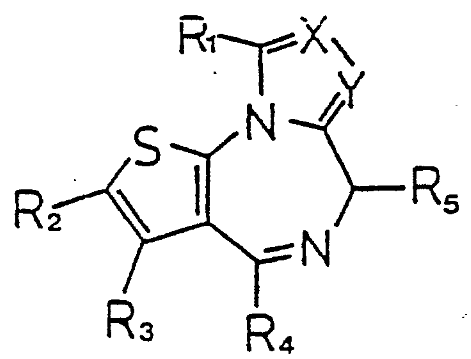

1

worin
$R_1$ Wasserstoff, eine verzweigte oder unverzweigte Alkylgruppe mit 1 bis 4 Kohlenstoffatom(en), bevorzugt Methyl, die gegebenenfalls durch Hydroxy oder Halogen substituiert sein kann, eine Cyclopropylgruppe, eine Cyclobutylgruppe, eine Cyclopentylgruppe, eine verzweigte oder unverzweigte Alkoxygruppe mit 1 bis 4 Kohlenstoffatomen, bevorzugt Methoxy, Halogen, bevorzugt Chlor oder Brom;
$R_2$ den Rest - G - $R_a$
worin
für n > O
$R_a$ Wasserstoff, Halogen, Hydroxy,

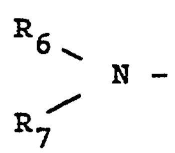

,

wobei $R_6$ und $R_7$, die gleich oder verschieden sein können, Wasserstoff, Phenyl, substituiertes Phenyl, eine verzweigte oder unverzweigte Alkyl-, Alkenyl- oder Alkinylgruppe mit 1 - 10 Kohlenstoffatom(en), bevorzugt 1 - 4 Kohlenstoffatom(en), die gegebenenfalls durch Halogen, Hydroxy, Phenyl, substituiertes Phenyl, oder durch einen C-verknüpften Heterocyclus substituiert sein kann, wobei die Kohlenstoffkette durch Stickstoff (auch NH), Sauerstoff oder Schwefel unterbrochen sein kann, eine verzweigte oder unverzweigte Alkylcarbonylgruppe mit 1-6 Kohlenstoffatom(en), [gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann], eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl,
eine Alkylsulfonylgruppe mit 1 bis 4 Kohlenstoffatomen, einen gegebenenfalls substituierten $C_3$ bis $C_7$

Cycloalkylrest, einen gegebenenfalls substituierten $C_5$ -$C_7$-Cycloalkenylrest, oder

$R_6$ oder $R_7$ ein gegebenenfalls ein- oder mehrfach durch verzweigtes oder unverzweigtes Alkyl mit 1 bis 4 Kohlenstoffatom(en) substituierter, gesättigter oder ungesättigter über ein Kohlenstoffatom gebundener 5-, 6- oder 7-gliedriger heterocyclischer Ring,,oder

$R_6$ und $R_7$ bilden zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatom(en), bevorzugt Methyl, substituiert sein kann;

$R_a$ eine Arylsulfonyloxygruppe, bevorzugt Tolylsulfonyloxy oder Phenylsulfonyloxy, gegebenenfalls ein- oder mehrfach durch verzweigte oder unverzweigte Alkyl- und/oder Alkoxygruppen mit 1 bis 4 Kohlenstoffatom(en) substituiert,

$R_a$ eine verzweigte oder unverzweigte Alkylsulfonyloxygruppe mit 1 bis 4 Kohlenstoffatom(en),

$R_a$ eine Arylcarbonyloxygruppe, bevorzugt Phenylcarbonyloxy, gegebenenfalls ein- oder mehrfach durch verzweigte oder unverzweigte Alkyl- und/oder Alkoxygruppen mit 1 bis 4 Kohlenstoffatom(en) substituiert, eine verzweigte oder unverzweigte Alkylcarbonyloxygruppe mit 1 bis 12, bevorzugt 1 bis 8 Kohlenstoffatom-(en), wobei die Alkylkette durch Stickstoff, Sauerstoff oder Schwefel unterbrochen sein kann;

$R_a$ 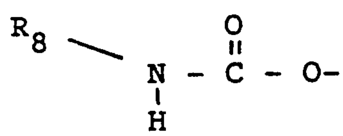 , 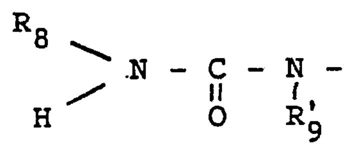

wobei $R_8$ eine verzweigte oder unverzweigte Alkyl-, Alkenyl- oder Alkinylgruppe mit 1 bis 4 Kohlenstoffatomen, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatom(en) substituierte Arylgruppe, $R'_9$ Wasserstoff oder eine verzweigte oder unverzweigte Alkylgruppe mit 1 bis 4 Kohlenstoffatom(en),

$R_a$ 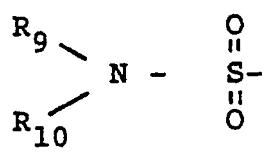

wobei $R_9$ und $R_{10}$, die gleich oder verschieden sein können, Wasserstoff, eine verzweigte oder unverzweigte Alkylgruppe mit 1 bis 10 Kohlenstoffatom(en), bevorzugt 1 bis 4 einen gegebenenfalls substituierten $C_3$ - $C_7$-Cycloalkylrest, einen gegebenenfalls substituierten $C_5$ - $C_7$-Cycloalkenylrest, oder $R_9$ und $R_{10}$ zusammen mit dem Stickstoffatom einen gegebenenfalls ein- oder mehrfach durch verzweigte oder unverzweigte Alkylgruppen mit 1 bis 4 Kohlenstoffatom(en) substituierten 5-, 6-oder 7-Ring, der als weitere Heteroatome Stickstoff, Sauerstoff oder Schwefel enthalten kann, wobei jedes weitere Stickstoffatom durch eine Alkylgruppe mit 1 bis 4 Kohlenstoffatom(en), bevorzugt Methyl, substituiert ist;

$R_a$ eine verzweigte oder unverzweigte Alkoxygruppe mit 1 bis 4 Kohlenstoffatomen;

eine Aryloxygruppe, bevorzugt Phenyloxy oder substituiertes Phenyloxy;

$R_a$ einen C-verknüpften 5 bis 7-gliedrigen nicht aromatischen Heterocyclus;

$R_a$ einen Imidorest, einen Benzimidazoylrest, ein cyclisches Amid oder Imid, das 5, 6 oder 7 Ringglieder aufweisen kann und in dem gegebenenfalls ein Kohlenstoffatom durch ein weiteres Heteroatom - wie z.B. Stickstoff, Sauerstoff oder Schwefel - ersetzt sein kann und das gegebenenfalls durch einen oder mehrere Arylrest(e) anneliert sein kann;

$R_a$ für n größer gleich 0: Wasserstoff, Halogen,

-CH=O, -CN, COOH;

$R_a$ Alkyloxycarbonylgruppe mit 1 - 10, bevorzugt 1 -4 Kohlenstoffatom(en), gegebenenfalls substituiert durch Hydroxy oder Halogen, bevorzugt Chlor, oder substituiert durch eine Aminogruppe, die gegebenenfalls ein- oder zweifach durch eine verzweigte oder unverzweigte Alkylgruppe mit 1 bis 6 Kohlenstoffatom-(en) substituiert sein kann, wobei die Alkylgruppe wiederum durch Halogen oder Hydroxy substituiert sein kann;

$R_a$ gegebenenfalls substituiertes Phenyloxycarbonyl,
$R_a$ einen Rest der allgemeinen Formel

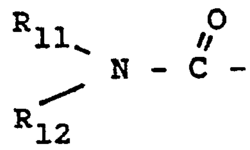
,

worin $R_{11}$ und $R_{12}$, die gleich oder verschieden sein können, Wasserstoff, Phenyl, substituiertes Phenyl, eine verzweigte oder unverzweigte Alkyl-, Alkenyl- oder Alkinylgruppe mit 1 bis 10 Kohlenstoffatom(en), bevorzugt 1 bis 4 Kohlenstoffatom(en), die gegebenenfalls {durch Halogen, Hydroxy, Nitro, Amino, eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann, Morpholin, Piperazin, N-Alkylpiperazin,
oder
im Fall von $R_{11}$ = Wasserstoff oder Alkyl und Y = C-$R_1$ oder Y Stickstoff und X C-Alkyl, $R_{12}$ durch eine Estergruppe oder ein Säureamid der allgemeinen Formel

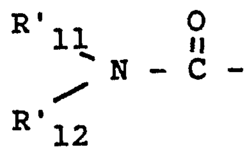

worin $R'_{11}$ und $R'_{12}$ dieselbe Bedeutung wie $R_{11}$ und $R_{12}$ - jedoch mit Ausnahme eines Säureamids haben können - substituiert sein kann,
$R_{11}$ oder $R_{12}$ ein gegebenenfalls ein- oder mehrfach durch verzweigtes oder unverzweigtes Alkyl mit 1 bis 4 Kohlenstoffatom(en) substituierter, gesättigter oder ungesättigter über ein Kohlenstoffatom gebundener 5-, 6- oder 7-gliedriger heterocyclischer Ring, $R_{11}$ oder $R_{12}$ einen gegebenenfalls substituierten $C_3$ bis $C_7$-Cycloalkylrest, einen gegebenenfalls substituierten $C_5$ bis $C_7$-Cycloalkenylrest,
oder
$R_{11}$ und $R_{12}$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatom(en) substituierten 5-, 6-oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatom(en), bevorzugt Methyl, substituiert sein kann;
$R_a$ einen Rest der allgemeinen Formel

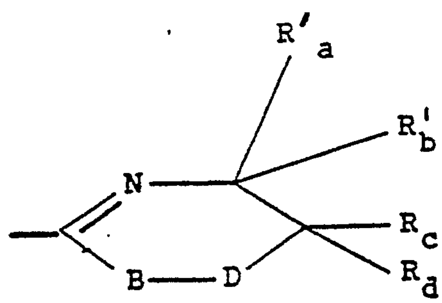
,
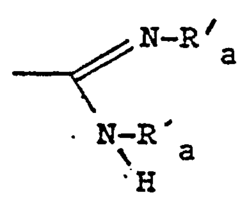

worin
B Sauerstoff, Schwefel, NH oder N-($C_1$-$C_6$-Alkyl),
D den Rest $(CR_eR_f)_n$, wobei n 0 bis 3 sein kann,
$R'_a$ Wasserstoff, gegebenenfalls durch eine Hydroxy- oder Aminogruppe substituiertes Alkyl mit 1 bis 6 Kohlenstoffatomen, $C_1$ bis $C_4$ Alkoxycarbonyl, Dialkylaminocarbonyl,
$R_b$, $R_c$, $R_d$, $R_e$, $R_f$ Wasserstoff, gegebenenfalls durch eine Hydroxy- oder Aminogruppe substituiertes Alkyl mit 1 bis 6 Kohlenstoffatomen, oder Phenyl;

$R_a$ Phenyl oder substituiertes Phenyl;

G eine verzweigte oder unverzweigte Alkyl-, Alkenyl-oder Alkinylgruppe mit n Kohlenstoffatomen, wobei G gegebenenfalls zusätzlich durch einen Arylrest oder zusätzlich durch $R_a$ substituiert sein kann;

n eine der Zahlen 0, 1, 2, 3, 4, 5, 6, 7, 8, 9 oder 10, wobei im Falle G einen Alkenyl- oder Alkinylrest bedeutet n > 1 ist und bevorzugt die Bedeutung 2, 3 und 4 besitzt;

$R_3$ Wasserstoff, Phenyl, substituiertes Phenyl oder eine verzweigte oder unverzweigte Alkylgruppe mit 1 bis 4 Kohlenstoffatom(en), bevorzugt Methyl;

oder

$R_2$ und $R_3$ zuammen einen Rest der allgemeinen Formel

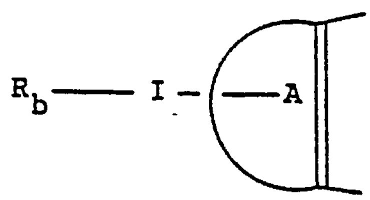

worin

A einen ankondensierten einfach ungesättigten 5-, 6-oder 7-gliedrigen Ring, wobei im Fall von m = 0 und $R_b$ = Wasserstoff ein Kohlenstoffatom durch C=0 ersetzt werden kann,

oder A einen ankondensierten Ring der Formel

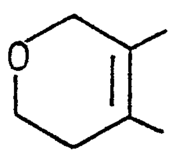 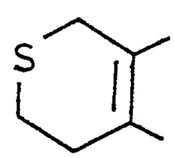 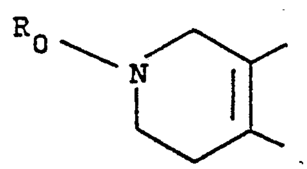

bedeutet,

wobei $R_0$ eine verzweigte oder unverzweigte Alkylgruppe mit 1 bis 18, bevorzugt mit 1 bis 4, Kohlenstoffatom(en), eine verzweigte oder unverzweigte Alkenyl- oder Alkinylgruppe mit 2 bis 18, bevorzugt 2 bis 4 Kohlenstoffatomen, eine Alkylcarbonyl- oder Alkylthiocarbonylgruppe mit 1 bis 18 bevorzugt 1 bis 4, Kohlenstoffatomen in der Alkylkette oder eine Arylcarbonyl- oder Arylthiocarbonylgruppe, eine gegebenenfalls substituierte $C_3$ bis $C_6$ Cycloalkyl- oder Cycloalkylcarbonylgruppe, eine Alkenyl- oder Alkinylcarbonylgruppe mit 2 bis 18, bevorzugt 2 bis 4 Kohlenstoffatomen, eine Alkoxycarbonylgruppe mit 1 bis 18, bevorzugt 1 bis 4 Kohlenstoffatomen, eine gegebenenfalls substituierte Phenalkyl-Phenylalkenylgruppe oder Phenalkinylgruppe mit 1 bis 6, bevorzugt 2 bis 4 Kohlenstoffatomen in der Kohlenstoffkette, eine Alkoxycarbonylalkylgruppe mit bis zu 18, bevorzugt bis zu 8 Kohlenstoffatomen, besonders bevorzugt bis zu 4, eine Alkylcarbonylalkylgruppe mit bis zu 18, bevorzugt bis zu 8 Kohlenstoffatom(en), besonders bevorzugt bis zu 4, eine Alkylcarbonylaminoalkylcarbonylgruppe mit bis zu 18, bevorzugt bis 10 Kohlenstoffatomen oder eine Aminocarbonylalkylgruppe mit bis zu 18, bevorzugt bis 4 Kohlenstoffatomen in der Alkylkette oder Wasserstoff,

$R_o$ einen Rest $R_{13}R_{14}NCO\text{-}(CH_2)_{0-3}$-, einen über $-(CH_2)_{0-3}$ verknüpften 5- oder 6-gliedrigen heterocyclischen Rest, ein über $-(CH_2)_{0-3}$, $-CO-(CH_2)_{0-3}$, $-CO-CH=CH_2$- verknüpfter $C_3$-$C_6$-Cycloalkylrest, bevorzugt Cyclopropyl;

I eine verzweigte oder unverzweigte Alkyl-, Alkenyl-oder Alkinylgruppe mit m Kohlenstoffatomen;

m 0, 1, 2, 3, 4, 5 oder 6;

$R_b$ Wasserstoff, Hydroxy, Amino, Formyl, Carboxy, Cyano, verzweigtes oder unverzweigtes Alkyloxycarbonyl mit 1 bis 18, bevorzugt 8, Kohlenstoffatom(en), wobei die Alkylkette gegebenenfalls durch Hydroxy, Amino, Nitro oder Halogen substituiert sein kann, eine gegebenenfalls substituierte Aryloxycarbonylgruppe;

$R_b$ einen Rest der allgemeinen Formel

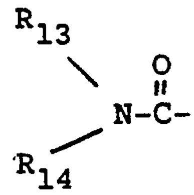

worin $R_{13}$ und $R_{14}$, die gleich oder verschieden sein können, Wasserstoff, Phenyl, substituiertes Phenyl, eine verzweigte oder unverzweigte Alkyl-, Alkenyl- oder Alkinylgruppe mit 1 bis 18 Kohlenstoffatomen, bevorzugt mit 1 bis 6, besonders bevorzugt 1 bis 4 Kohlenstoffatom(en), die gegebenenfalls durch Halogen, Hydroxy, Nitro, Amino, substituiertes Amino, $C_3$-$C_6$-Cycloalkyl, bevorzugt Cyclopropyl, Alkoxy, bevorzugt Methoxy oder im Fall von $R_{13}$ = Wasserstoff oder Alkyl, durch eine Esterfunktion oder durch ein Säureamid der allgemeinen Formel

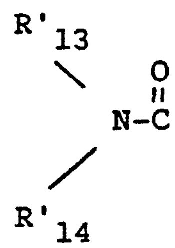

worin $R'_{13}$ und $R'_{14}$, dieselbe Bedeutung wie $R_{13}$ und $R_{14}$, jedoch mit Ausnahme eines Säureamids haben können, substituiert sein kann,

$R_{13}$ oder $R_{14}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoff verknüpfter 5-, 6-, oder 7-gliedriger heterocyclischer Ring, einen gegebenenfalls substituierten $C_3$- bis $C_7$-Cycloalkylrest, einen gegebenenfalls substituierten Cycloalkenylrest;

oder

$R_{13}$ und $R_{14}$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6-oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatom(en), bevorzugt Methyl, substituiert sein kann;

$R_b$ einen Rest der allgemeinen Formel

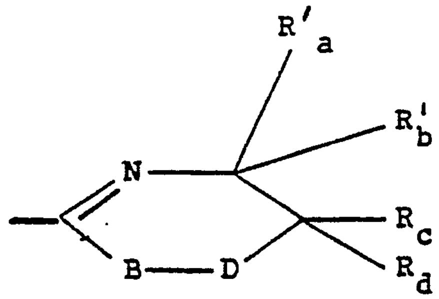

/

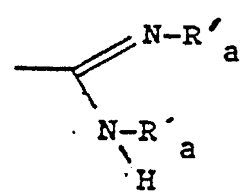

worin

B Sauerstoff, Schwefel, NH oder N-($C_1$-$C_6$-Alkyl),

D den Rest $(CR_eR_f)_n$, wobei n 0 bis 3 sein kann,

$R'_a$ Wasserstoff, gegebenenfalls durch eine Hydroxy- oder Aminogruppe substituiertes Alkyl mit 1 bis 6 Kohlenstoffatom(en), $C_1$ bis $C_4$ Alkoxycarbonyl, Dialkylaminocarbonyl,

$R_b$, $R_c$, $R_d$, $R_e$, $R_f$ Wasserstoff, gegebenenfalls durch eine Hydroxy- oder Aminogruppe substituiertes Alkyl mit 1 bis 6 Kohlenstoffatom(en), oder Phenyl;

$R_b$ 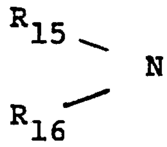

$R_{15}$ = Wasserstoff, $R_{16}$ = Wasserstoff, Alkylcarbonyl oder Alkoxycarbonyl mit 1 bis 18, bevorzugt 1 bis 6 Kohlenstoffatom(en), Aminocarbonyl, Alkylaminocarbonyl, Dialkylaminocarbonyl mit 1 bis 18 - bevorzugt 1 bis 6 Kohlenstoffatom(en) - in der Alkylkette;
$R_b$ Wasserstoff, Phenyl, substituiertes Phenyl;

$R_b$ Halogen, 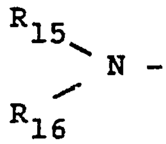 ,

wobei $R_{15}$ und $R_{16}$, die gleich oder verschieden sein können, Wasserstoff, Phenyl, substituiertes Phenyl, eine verzweigte oder unverzweigte Alkyl-, Alkenyl- oder Alkinylgruppe mit 1 - 18 Kohlenstoffatom(en), bevorzugt 1 bis 6 Kohlenstoffatom(en), die gegebenenfalls durch Halogen, Hydroxy, $C_3$-$C_6$-Cycloalkyl oder einen C-verknüpften Heterocyclus substituiert sein kann, wobei die Kohlenstoffkette durch Stickstoff, Sauerstoff oder Schwefel unterbrochen sein kann,
eine verzweigte oder unverzweigte Alkylcarbonylgruppe mit 1-6 Kohlenstoffatom(en), gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatom(en) substituierte Aminogruppe, wobei die Alkylgruppe durch Halogen oder Hydroxy substituiert sein kann, eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatom(en),
oder
$R_{15}$ oder $R_{16}$ einen gegebenenfalls ein- oder mehrfach durch verzweigtes oder unverzweigtes Alkyl mit 1 bis 4 Kohlenstoffatom(en) substituierter, gesättigter oder ungesättigter über ein Kohlenstoffatom gebundener 5-, 6- oder 7-gliedriger heterocyclischer Ring,
$R_{15}$ oder $R_{16}$ einen gegebenenfalls substituierten $C_3$ bis $C_7$-Cycloalkylrest, einen gegebenenfalls substituierten $C_5$ bis $C_7$-Cycloalkenylrest
oder
$R_{15}$ und $R_{16}$ bilden zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatom(en) substituierten 5-, 6- oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatom(en), bevorzugt Methyl, substituiert sein kann;
$R_b$ eine Arylsulfonyloxygruppe, bevorzugt Tolylsulfonyloxy oder Phenylsulfonyloxy, gegebenenfalls ein- oder mehrfach durch verzweigte oder unverzweigte Alkyl- und/oder Alkoxygruppen mit 1 bis 4 Kohlenstoffatom(en) substituiert;
$R_b$ eine verzweigte oder unverzweigte Alkylsulfonyloxygruppe mit 1 bis 4 Kohlenstoffatom(en);
$R_b$ eine Arylcarbonyloxygruppe, bevorzugt Tolylcarbonyloxy oder Phenylcarbonyloxy, gegebenenfalls ein oder mehrfach durch verzweigte oder unverzweigte Alkyl- und/oder Alkoxygruppen mit 1 bis 4 Kohlenstoffatom(en) substituiert;
$R_b$ eine verzweigte oder unverzweigte Alkylcarbonyloxygruppe mit 1 bis 18, bevorzugt 1 bis 8, Kohlenstoffatom(en), wobei die Alkylkette durch Stickstoff, Sauerstoff oder Schwefel unterbrochen sein kann;

$R_b$ 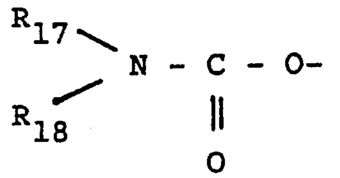 , 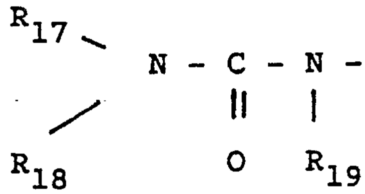

wobei $R_{17}$ Wasserstoff und $R_{18}$ eine Alkyl-, Alkenyl- oder Alkinylgruppe mit 2 bis 4 Kohlenstoffatomen, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatom(en) substituierte Arylgruppe, $R_{19}$ Wasserstoff oder eine verzweigte oder unverzweigte Alkylgruppe mit 1 bis 4 Kohlenstoffatom(en);
$R_b$ einen Imidorest,
ein cyclisches Amid oder Imid, das 5, 6 oder 7 Ringglieder aufweisen kann und in dem gegebenenfalls ein Kohlenstoffatom durch ein weiteres Heteroatom - wie z.B. Stickstoff, Sauerstoff oder Schwefel - ersetzt sein kann und das gegebenenfalls durch einen oder mehrere Arylrest(e) anneliert sein kann,
einen Benzimidazolylrest;
$R_b$ einen verzweigten oder unverzweigten Alkyloxy-bzw. Alkylthiorest mit 1 bis 18, bevorzugt 1 bis 4 Kohlenstoffatom(en), einen gegebenenfalls substituierten Phenyloxy- oder Phenylthiorest, einen über Sauerstoff oder Schwefel verknüpften, gesättigten oder ungestättigten 5- oder 6-gliedrigen heterocyclischen Ring;
$R_4$ einen Rest der allgemeinen Formel
$-S_K - W_L - T_M - V_N - U_M - P_N - Z_\alpha$
worin
S eine Einfachbindung, verzweigtes oder unverzweigtes Alkyl mit 1 bis 8 - bevorzugt 1 bis 4 - Kohlenstoffatom(en), gegebenenfalls durch Halogen und/oder Hydroxy substituiert, verzweigtes oder unverzweigtes Alkenyl mit 2 bis 8 Kohlenstoffatomen - bevorzugt mit 2 bis 4 Kohlenstoffatomen, gegebenenfalls durch Halogen und/oder Hydroxy substituiert, verzweigtes oder unverzweigtes Alkinyl mit 2 bis 8 Kohlenstoffatomen - bevorzugt mit 2 bis 4 Kohlenstoffatom(en), gegebenenfalls durch Halogen und/oder Hydroxy substituiert;
W einen gegebenenfalls substituierter aromatischer Ring mit 5 bis 10 Kohlenstoffatomen, der gegebenenfalls ein oder mehrere Heteroatom(e) aus der Gruppe Stickstoff,
Sauerstoff oder Schwefel enthalten kann, einen gegebenenfalls substituierten carbocyclischen Ring mit 3 bis 7 Kohlenstoffatomen, der auch eine Doppelbindung enthalten kann;
T eine Einfachbindung, verzweigtes oder unverzweigtes Alkyl gegebenenfalls substituiert durch Halogen oder Hydroxy mit 1 bis 8 - bevorzugt 1 bis 4 -Kohlenstoffatom(en); verzweigtes oder unverzweigtes Alkenyl mit 2 bis 8 Kohlenstoffatomen - bevorzugt 2 bis 4 Kohlenstoffatomen - gegebenenfalls durch Halogen und/oder Hydroxy substituiert; verzweigtes oder unverzweigtes Alkinyl mit 2 bis 8 Kohlenstoffatomen - bevorzugt 2 bis 4 Kohlenstoffatomen - gegebenenfalls durch Halogen und/oder Hydroxy substituiert - einen Rest der Formel
$-(F)_{0-1} - E - (F)_{0-1} -$
$E = O, S, N\text{-}R_6,$

C=O, 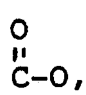 , 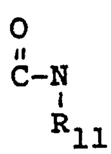 , 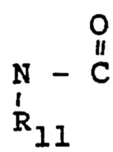 ,

worin $R_6$ wie zuvor definiert, bevorzugt Wasserstoff, $C_1 - C_4$-Alkyl, oder

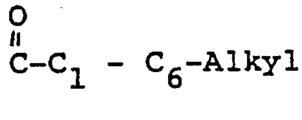 oder 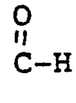 ,

und $R_{11}$ wie zuvor definiert, bevorzugt Wasserstoff oder $C_1 - C_4$ -Alkyl; oder E bedeutet

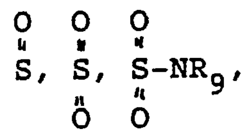

$R_9$ wie zuvor definiert ist,

F eine verzweigte oder unverzweigte gegebenenfalls substituierte Alkylgruppe mit 1 bis 8 - bevorzugt 1 bis 4 -Kohlenstoffatom(en), eine gegebenenfalls substituierte Alkenylgruppe mit 2 bis 8 -bevorzugt 2 bis 4 -Kohlenstoffatomen, eine gegebenenfalls substituierte Alkinylgruppe mit 2 bis 8 - bevorzugt 2 bis 4 Kohlenstoffatomen;

V einen gegebenenfalls substituierten aromatischen Ring mit 5 bis 10 Kohlenstoffatomen, der gegebenenfalls ein oder mehrere Heteroatome aus der Gruppe Stickstoff, Sauerstoff oder Schwefel enthalten kann, einen gegebenenfalls substituierten carbocyclischen Ring mit 3 bis 7 Kohlenstoffatomen, der auch eine Doppelbindung enthalten kann, ein cyclisches Amid oder Imid, das 5, 6 oder 7 Ringglieder aufweisen kann und in dem gegebenenfalls ein Kohlenstoffatom durch ein weiteres Heteroatom - wie z.B. Stickstoff, Sauerstoff oder Schwefel - ersetzt sein kann und das gegebenenfalls durch einen oder mehrere Arylrest(e) anneliert sein kann;

U verzweigtes oder unverzweigtes Alkyl gegebenenfalls substituiert durch Halogen oder Hydroxy mit 1 bis 8 - bevorzugt 1 bis 4 -Kohlenstoffatom(en), verzweigtes oder unverzweigtes Alkenyl mit 2 bis 8 Kohlenstoffatomen - bevorzugt 2 bis 4 Kohlenstoffatomen - gegebenenfalls durch Halogen und/oder Hydroxy substituiert; verzweigtes oder unverzweigtes Alkinyl mit 2 bis 8 Kohlenstoffatomen - bevorzugt 2 bis 4 Kohlenstoffatomen - gegebenenfalls durch Halogen und/oder Hydroxy substituiert; einen Rest der Formel

$-(F)_{0-1}-E-(F)_{0-1}-$

$E = O, S, N\text{-}R_6,$

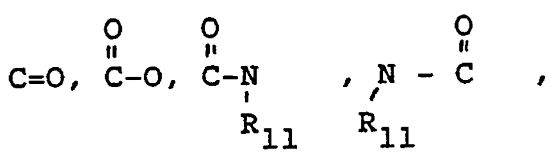

worin $R_6$ wie zuvor definiert, bevorzugt Wasserstoff, $C_1$ - $C_4$-Alkyl, oder

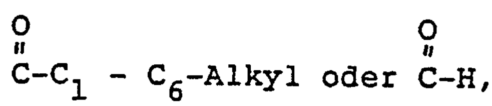

und $R_{11}$ wie zuvor definiert, bevorzugt Wasserstoff oder $C_1$ - $C_4$ -Alkyl; oder E bedeutet

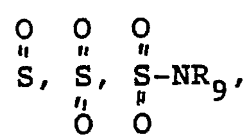

worin $R_9$ wie zuvor definiert,

F eine verzweigte oder unverzweigte gegebenenfalls substituierte Alkylgruppe mit 1 bis 8 - bevorzugt 1 bis 4 - Kohlenstoffatom(en), eine gegebenenfalls substituierte Alkenylgruppe mit 2 bis 8 -bevorzugt 2 bis 4 -Kohlenstoffatomen, eine gegebenenfalls substituierte Alkinylgruppe mit 2 bis 8 - bevorzugt 2 bis 4 Kohlenstoffatomen;

P einen gegebenenfalls substituierten aromatischen Ring mit 6 bis 10 Kohlenstoffatomen, der gegebenenfalls ein oder mehrere Heteroatome aus der Gruppe Stickstoff, Sauerstoff oder Schwefel enthalten kann, einen gegebenenfalls substituierten carbocyclischen Ring mit 3 bis 7 Kohlenstoffatomen, der auch eine Doppelbindung enthalten kann, wobei W, V und/oder P ein- oder mehrfach durch Z substituiert sein kann, ein cyclisches Amid oder Imid, das 5, 6 oder 7 Ringglieder aufweisen kann und in dem gegebenenfalls ein Kohlenstoffatom durch ein weiteres Heteroatom wie z.B. Stickstoff, Sauerstoff oder Schwefel - ersetzt sein kann und das gegebenenfalls durch einen oder mehrere Arylrest(e) anneliert sein kann;

Z Wasserstoff, Halogen, Hydroxy, Nitro, Cyano, Trifluormethyl, gegebenenfalls durch Halogen oder Hydroxy substituiertes, verzweigtes oder unverzweigtes Alkyl mit 1 bis 8 - bevorzugt 1 bis 4 - Kohlenstoffatom(en), gegebenenfalls substituiertes, verzweigtes Alkoxy mit 1 bis 8 bevorzugt 1 bis 4 Kohlenstoffatom(en), einen Rest der Formel

$$-(F')_{0-1}-E'-(F')_{0-1}-H,\ (F')_{0-1}-NO_2,$$

$$-SO_2-C_1-C_4\text{-Alkyl},$$

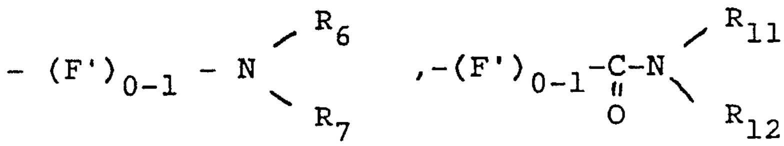

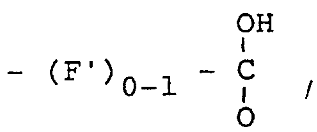

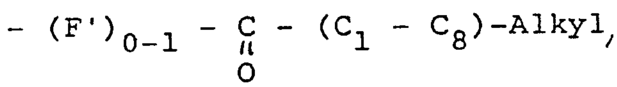

bevorzugt $C_1 - C_4$-Alkyl,

worin F′ eine verzweigte oder unverzweigte Alkylgruppe mit 1 bis 4 Kohlenstoffatom(en), bevorzugt 1 bis 2 Kohlenstoffatom(en) oder F′ = F und E′ = E oder

E′ = O, S, $N\text{-}R_6$,

C = O, 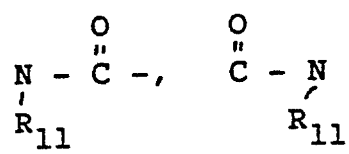

wobei bei $\alpha > 1$ alle Z gleich, teilweise gleich oder verschieden sein können,

K = 0, 1,

L = 0, 1, 2,

M = 0, 1,

N = 0, 1

und $\alpha$ = 1, 2, 3, 4, 5 bedeuten können und E die unter T angegebene Bedeutung hat, wobei die Summe K + L + M + N ungleich 0 ist und

$-S_K - W_L - T_M - V_N\ U_M - P_N - Z_\alpha$

insgesamt nicht einen Phenylrest, einen Phenylrest, der mit Halogen, $CH_3$, $CF_3$ und $NO_2$ substituiert ist, einen Pyridyl- oder einen Thienylrest bedeuten kann;

$R_5$ Wasserstoff, $CF_3$, Hydroxy, verzweigtes oder unverzweigtes Alkyl mit 1 bis 6, bevorzugt 1 bis 4 Kohlenstoffatom(en), besonders bevorzugt Methyl, gegebenenfalls ist die Alkylgruppe durch Hydroxy, Halogen, $C_1$- bis $C_4$-Alkylsulfonyloxy, bevorzugt Methylsulfonyloxy,

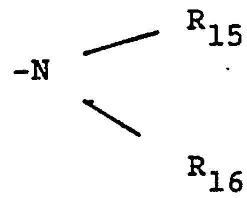

worin $R_{15}$ und $R_{16}$ die zuvor genannte Bedeutung aufweisen können, oder

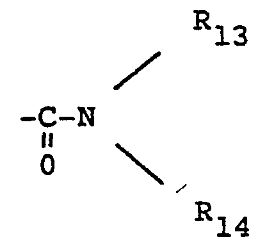

worin $R_{13}$ und $R_{14}$ die zuvor genannte Bedeutng aufweisen können, substituiert;
Alkylcarbonyloxy mit 1 bis 4 Kohlenstoffatom(en) in den verzweigten oder unverzweigten Alkylkette, Alkoxycarbonyl mit 1 bis 4 Kohlenstoffatom(en) in der verzweigten oder unverzweigten Alkylkette, Carboxyalkyl mit 1 bis 4 Kohlenstoffatom(en) in der verzweigten oder unverzweigten Alkylkette, Alkoxycarbonylalkyl mit 1 bis 4 Kohlenstoffatom(en) in den verzweigten oder unverzweigten Alkylresten
X/Y unabhängig voneinander C-$R_1$, oder N, aber nicht beide C-$R_1$, mit $R_1$ bevorzugt Wasserstoff oder Methyl,
oder Y die Gruppe C-COOR′, wobei R′ Alkyl oder Wasserstoff bedeutet und X Stickstoff bedeutet;
gegebenenfalls in Form ihrer Racemate, ihrer Enantiomere, ihrer Diastereomere und ihrer Gemische sowie gegebenenfalls ihre physiologisch unbedenklichen Säureadditionssalze.

Bevorzugte Verbindungen der allgemeinen Formel I sind solche, in denen $R_2$ und $R_3$ einen Rest wie für die allgemeine Formel 1 eingangs definiert und worin
$R_1$ Ethyl, Trifluormethyl, Methoxy, Ethoxy oder Halogen, besonders bevorzugt Chlor oder Brom; besonders bevorzugt Methyl;
$R_a$ Wasserstoff, Chlor, Brom, Jod, Hydroxy, $C_1$-$C_4$-Alkoxycarbonyl, Phenyl, substituiertes Phenyl; $C_3$-$C_6$-Cycloalkyl;
wobei in $R_4$ bevorzugt
S eine Einfachbindung (K = 0), verzweigtes oder unverzweigtes $C_1$-$C_4$-Alkyl, verzweigtes oder unverzweigtes $C_2$-$C_4$-Alkenyl, verzweigtes oder unverzweigtes $C_2$-$C_4$-Alkinyl,
W einen Phenylring, einen Pyridinring, einen Thiophenring - gegebenenfalls mit Halogen, einer Hydroxygruppe, einen Cyclopropylrest, einem $C_1$-$C_4$-Alkylrest, einem $C_1$-$C_4$-Alkoxyrest untereinander gleich oder verschieden ein- oder mehrfach substituiert;
T eine Alkylkette mit bis zu 6 Gliedern, bevorzugt Ethyl, eine Alkenylkette mit 2 bis 4 Gliedern bevorzugt Vinyl, eine Alkinylkette mit 2 bis 4 Gliedern bevorzugt C≡C, die gegebenenfalls durch ein Heteroatom - wie Sauerstoff, Schwefel oder Stickstoff unterbrochen sein können,

oder 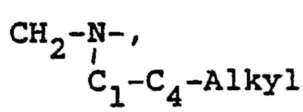 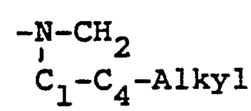

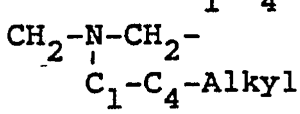

oder

den Rest 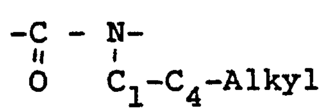 ;

V eine Phenylgruppe, eine Thienylgruppe oder eine $C_3$-$C_6$-Cycloalkylgruppe, eine substituierte Phenylgruppe,
U bevorzugt eine Einfachbindung (M = O),
oder $-CH_2-CH_2-$
wenn P eine Phenylgruppe bedeutet;
P eine Phenylgruppe, bevorzugt $P_N$ = null;
Z Wasserstoff, verzweigtes oder unverzweigtes $C_1$-$C_4$-Alkyl oder $C_1$-$C_4$-Alkoxy, Trifluormethyl, Halogen - bevorzugt Chlor oder Brom, CN, $NO_2$, Cyano, COH, COOH, $COOC_1$ - $C_4$-Alkyl, $CONH_2$, $CON(C_1$ - $C_4$-$Alkyl)_2$, Cyclopropyl, Amino, $C_1$-$C_4$-Alkylamino, $C_1$-$C_4$-Dialkylamino, Hydroxymethyl, $SO_2$-$C_1$-$C_4$-Alkyl, $SO_2$-$C_1$-$C_4$-Hydroxyalkyl, Hydroxy, SH, S-$C_1$-$C_4$-Alkyl,
wobei bei $\alpha > 1$ alle Z gleich, teilweise gleich oder verschieden sein können,
K = 0, 1, bevorzugt 0
L = 0, 1, 2, bevorzugt 1
M = 0, 1, bevorzugt 1
N = 0, 1, bevorzugt 1, aber $P_N$ bevorzugt 0
und $\alpha$ = 1, 2, 3, bedeuten können, wobei die Summe K + L + M + N ungleich 0 ist und
$- S_K - W_L - T_M - V_N - U_M-P_N- Z_\alpha$
insgesamt nicht einen Phenylrest, einen Phenylrest, der mit Halogen, $CH_3$, $CF_3$ und $NO_2$ substituiert ist, einen Pyridyl- oder einen Thienylrest bedeutet.
$R_5$ Wasserstoff, Hydroxy, Methyl oder Trifluormethyl;
X,Y unabhängig voneinander C-$R_1$ oder N, aber nicht gleichzeitig beide C-$R_1$, bevorzugt beide N oder X = C-$R_1$ ($R_1$ = H, Methyl) Y = N,
G wie zuvor definiert ist,
n eine der Zahlen 1, 2, 3, 4, 5, 6, 7, 8, 9 oder 10, bevorzugt 0, 1 oder 2, bedeuten können sowie gegebenenfalls ihre physiologisch unbedenklichen Säureadditionssalze.

Besonders bevorzugt sind Verbindungen der allgemeinen Formel 1 in denen $R_2$ und $R_3$ ein Rest der allgemeinen Formel

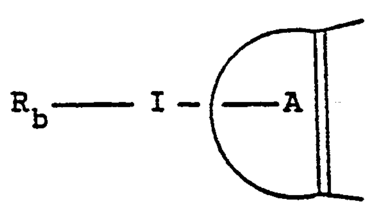

bilden, worin A ein ankondensierter einfach ungesättigter 5- oder 6-gliedriger Ring bedeutet, oder einen Rest der allgemeinen Formel

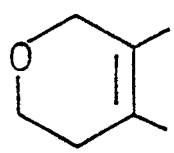

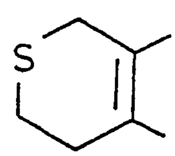

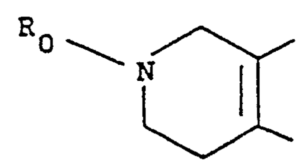

worin $R_o$ = Wasserstoff, $C_1$-$C_4$-Alkyl, $C_3$ bis $C_6$-Cycloalkyl, $C_3$ bis $C_6$-Cycloalkylcarbonyl
I eine, unverzweigte Alkylgruppe mit m Kohlenstoffatomen
m O, 1, 2, 3 oder 4;
X/Y unabhängig voneinander C-$R_1$ oder N, aber nicht beide C-$R_1$, bevorzugt beide N oder X C-$R_1$ und Y N ($R_1$ = H, Methyl);
oder
Y C-COOR′, wobei R′ Wasserstoff oder Niederalkyl und X Stickstoff bedeutet;
$R_1$ Wasserstoff, Hydroxymethyl, Chlormethyl, Trifluormethyl, Cyclopropyl, Ethyl, Methoxy, Ethoxy, Chlor oder Brom, bevorzugt Methyl;
$R_b$ Wasserstoff, Hydroxy, Amino, Aminocarbonyl, Carboxy, Cyano, Alkyloxycarbonyl mit 1 bis 6 Kohlenstoffatom(en), ein Säureamid der Formel $R_{13}R_{14}NCO$, worin $R_{13}$ und $R_{14}$ ein $C_1$ bis $C_4$-Alkylrest oder $R_{13}$ und $R_{14}$ zusammen mit dem Stickstoffatom einen gegebenenfalls substituierten 6-gliedrigen

Heterocyclus, der als weiteres Heteroatom Sauerstoff, Schwefel oder Stickstoff enthalten kann,
worin in $R_4$ besonders bevorzugt
S eine Einfachbindung (K = 0), eine Methylengruppe oder eine Ethylengruppe;
W einen Phenylring, einen Thiophenring, der gegebenenfalls mit Halogen, einer Hydroxygruppe, einem $C_1$-$C_4$-Alkylrest, einem $C_1$-$C_4$-Alkoxyrest, einen Cyclopropylrest, untereinander gleich oder verschieden, ein- oder mehrfach substituiert sein kann;
T eine Alkylkette mit bis zu 4 Gliedern, eine Alkenylkette mit 2 bis 4 Gliedern, eine Alkinylkette mit 2 bis 4 Gliedern, die gegebenenfalls durch ein Heteroatom wie Sauerstoff oder Schwefel unterbrochen ist;

oder

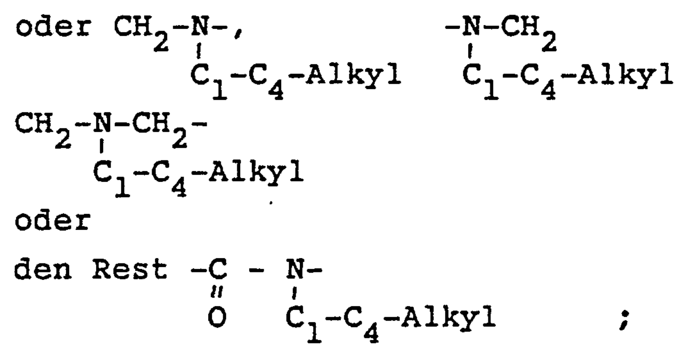

oder
den Rest
;

V eine Phenylgruppe; eine substituierte Phenylgruppe, $C_3$-$C_6$-Cycloalkylgruppe, eine Thienylgruppe
U bevorzugt eine Einfachbindung (M = O), oder
$-CH_2-CH_2-$
wenn P eine Phenygruppe bedeutet;
P eine Phenylgruppe, bevorzugt $P_N$ = null;
Z Wasserstoff, verzweigtes oder unverzweigtes $C_1$-$C_4$-Alkyl oder $C_1$-$C_4$-Alkoxy, Trifluormethyl, Halogen - bevorzugt Chlor oder Brom, CN, $NO_2$, Cyano, COH, COOH, $COOC_1$-$C_4$-Alkyl, $CONH_2$, $CON(C_1$-$C_4$-$Alkyl)_2$, Cyclopropyl, Amino, $C_1$-$C_4$-Alkylamino, $C_1$-$C_4$-Dialkylamino, Hydroxymethyl, $SO_2$-$C_1$-$C_4$-Alkyl, $C_1$-$C_4$-Dialkylamino, Hydroxymethyl, $SO_2$-$C_1$-$C_4$-Alkyl, $SO_2$-$C_1$-$C_4$-Hydroxyalkyl, Hydroxy, SH, S-$C_1$-$C_4$-Alkyl, und
K = 0, 1, bevorzugt O,
L = 0, 1, bevorzugt 1,
M = 0, 1, bevorzugt 1,
N = 0, 1, bevorzugt 1, aber $P_N$ bevorzugt 0
und $\alpha$ = 1, 2, 3,
bedeuten kann, wobei die Summe K + L + M + N ungleich 0 ist und
$R_5$ Wasserstoff, Hydroxy, Methyl oder Trifluormethyl bedeuten kann.

Besonders bevorzugt sind Verbindungen der allgemeinen Formel I, worin $R_4$ einen der folgenden Reste aufweist
cis-4[(4-Trifluormethylphenyl)vinyl]phenyl,
trans-4[(4-Trifluormethylphenyl)vinyl]phenyl,
trans-4[(4-Chlorphenyl)aminocarbonyl]phenyl,
4[2-(4-Trifluormethylphenyl)ethinyl]phenyl,
4[(4-Chlorphenyl)N-methylaminocarbonyl]phenyl,
4[(4-Chlorphenyl)thiomethylen]phenyl,
4[(4-Chlorphenyl)aminomethylen]phenyl
4-[(4-Chlorphenyl)methylenoxy]phenyl,
4-[(4-Chlorphenyl)oxymethylen]phenyl,
4[2-(4-Methylphenyl)ethyl]phenyl,
4[2-(4-iso-Butylphenyl)ethyl]phenyl,
4[2-(Phenyl)ethyl]phenyl,
4-Benzylphenyl,
3-Benzylphenyl,
4[2(3,4,5-Trimethoxyphenyl)ethyl]phenyl
4-iso-Butylphenyl,
4-[2-(4-Phenethylphenyl)ethyl]phenyl,
4-[2-(4-Methoxyphenyl)ethyl]phenyl,

4-Methoxycarbonylphenyl,
4-Hydroxymethylphenyl,
4-Carboxyphenyl,
4[(4-Chlorphenyl)aminocarbonyl]phenyl,
4-Morpholinocarbonylphenyl,
Biphenyl,
4-(4-Phenylbutyl)phenyl,
4-((N-Phenyl-N-acetylamino)methyl)phenyl,
4-((N-4-Chlorphenyl-N-methylamino)methyl)phenyl,
4[2-(4-Chlorphenyl)ethyl]phenyl,
4[2-(4-Triluormethylphenyl)ethyl]phenyl,
4[2-(4-Methoxyphenyl)ethyl]phenyl,
4[2-(3,4,5-Trimethoxyphenyl)ethyl]phenyl,
4[2-(4-Isobutylphenyl)ethyl]phenyl,
4-Methoxycarbonylphenyl,
4-Hydroxymethylphenyl,
4-Carboxyphenyl,
4[(4-Chlorphenyl)N-methyl-aminocarbonyl]phenyl,
4[(4-Cyclopropylphenyl)-N-propyl-aminocarbonyl]phenyl,
4[(3-Trifluormethylphenyl)-N-ethylaminomethylen]phenyl,
4[(3-Trifluormethylphenyl)-N-butylaminomethylen]phenyl,
4(N-Ethyl-N-benzyl-amino)phenyl,
4[(4-Cyclopropylphenyl)-N-butylaminomethylen]phenyl,
4[2(Thien-2-yl)ethyl]phenyl,
4[2(4-Cyclopropylphenyl)ethyl]phenyl.

Die erfindungsgemäßen Hetrazepine können ein C-Atom mit einem Asymmetriezentrum aufweisen und können je nach Substitutionsmuster auch mehrere Asymmetriezentren besitzen und daher in verschiedenen stereochemischen Formen existieren.

Die bei der Synthese gegebenenfalls anfallenden Gemische optisch isomerer Verbindungen können durch Bildung von Diastereomeren und nachfolgender an sich bekannte Verfahren, wie z.B. durch Kristallisation, durch chromatographische oder enzymatische Trennverfahren in die einzelnen optischen Isomeren getrennt werden. Gegenstand der Erfindung sind die einzelnen Isomeren, deren Mischungen sowie die entsprechenden physiologisch geeigneten Säureadditionssalze mit anorganischen oder organischen Säuren. Bevorzugt sind bespielsweise Salze mit Chlorwasserstoffsäure, Bromwasserstoffsäure, Schwefelsäure, Phosphorsäure, Methansulfonsäure, Ethansulfonsäure Toluolsulfonsäure, Benzolsulfonsäure, Milchsäure, Malonsäure, Bernsteinsäure, Maleinsäure, Fumarsäure, Äpfelsäure, Weinsäure, Zitronensäure oder Benzoesäure.

Soweit nicht im einzelnen abweichende Angaben gemacht werden, werden die allgemeinen Definitionen im folgenden Sinn gebraucht:

Alkyl steht im allgemeinen für einen unverzweigten oder verzweigten Kohlenwasserstoffrest mit 1 bis 18 Kohlenstoffatom(en), der gegebenenfalls mit einem Halogenatom oder mehreren Halogenatomen - vorzugsweise Fluor - substituiert sein kann, die untereinander gleich oder verschieden sein können, wobei Niederalkylreste für einen verzweigten oder unverzweigten Kohlenwasserstoffrest mit 1 bis etwa 4 Kohlenstoffatom(en) bevorzugt sind.

Als Alkylgruppen (auch soweit sie Bestandteile anderer Reste sind) werden bevorzugt, soweit nichts anderes angegeben ist, Methyl, Ethyl, Propyl, iso-Propyl, n-Propyl, n-Butyl, iso-Butyl und tert.-Butyl. Von den langkettigen Alkylresten sind n-Hexadecanyl und n-Octadecanyl bevorzugt.

Alkenyl steht im allgemeinen für einen geradkettigen oder verzweigten Kohlenwasserstoffrest mit 2 bis 18 Kohlenstoffatomen und mit einer oder mehreren, bevorzugt mit einer oder zwei Doppelbindung(en), der gegebenenfalls mit einem Halogenatom oder mehreren Halogenatomen -vorzugsweise Fluor - substituiert sein kann, die untereinander gleich oder verschieden sein können. Bevorzugt ist ein Niederalkenylrest mit 2 bis etwa 4 Kohlenstoffatomen und einer oder zwei Doppelbindung(en). Als Beispiele seien Vinyl, Allyl, Propenyl, Isopropenyl, Pentenyl und Isopentenyl genannt.

Alkinyl steht im allgemeinen für einen geradkettigen oder verzweigten Kohlenwasserstoffrest mit 2 bis 18 Kohlenstoffatomen und mit einer oder mehreren, bevorzugt mit einer oder zwei Dreifachbindung(en), der gegebenenfalls substituiert sein kann.

Bevorzugt ist ein Niederalkinylrest mit 2 bis 4 Kohlenstoffatomen und einer oder zwei Dreifachbindung-(en), der gegebenenfalls mit einem Halogenatom oder mehreren Halogenatomen substituiert sein kann, die

untereinander gleich oder verschieden sein können. Als Beispiele seien Ethinyl, Propargyl und 2-Butinyl genannt.

Cycloalkyl steht im allgemeinen für einen gesättigten oder ungesättigten cyclischen Kohlenwasserstoffrest mit 3 bis 9 Kohlenstoffatomen, der gegebenenfalls mit einem Halogenatom oder mehreren Halogenatomen einer Hydroxygruppe, einer Methylgruppe substituiert sein kann, die untereinander gleich oder verschieden sein können. Bevorzugt sind cyclische Kohlenwasserstoffe mit 3 bis 6 Kohlenstoffatomen. Als Beispiele seien Cyclopropyl, Cyclobutyl, Cyclopentyl, Cyclopentenyl, Cyclohexyl, Cyclohexenyl, Cycloheptyl, Cycloheptenyl, Cycloheptadienyl und Cyclooctyl, Cyclooctenyl, Cyclooctadienyl, Cyclononinyl genannt.

Aryl steht im allgemeinen für einen aromatisohen Rest mit 6 bis 10 Kohlenstoffatomen - auch in Zusammensetzungen, wobei der Aromat mit einer oder mehreren Niederalkylgruppe(n), Alkoxygruppe(n), Nitrogruppe(n), Aminogruppe(n) und/oder einem oder mehreren Halogenatom(en) - untereinander gleich oder verschieden -substituiert sein kann. Bevorzugte Arylreste sind Phenyl, Naphthyl und Biphenyl. Besonders bevorzugt ist ein Phenylrest, der gegebenenfalls substituiert sein kann.

Eine substituierte Phenylgruppe kann beispielsweise einen oder mehrere der nachfolgend genannten Substituenten tragen: $C_1$-$C_6$-Alkyl, $C_1$-$C_6$-Alkoxy, Halogen, Amino, Alkylamino, Dialkylamino, $CF_3$, $C_3$-$C_6$-Cycloalkyl, Cyano, $NO_2$, COH, COOH, $COOC_1$-$C_4$-Alkyl, Cyclopropyl, Hydroxy, SH, S-$C_1$-$C_4$-Alkyl, Hydroxymethyl.

Beispielhaft für substituiertes Phenyl werden genannt: 3-Chlorphenyl, 4-Chlorphenyl, 3-Bromphenyl, 4-Bromphenyl, 4-Fluormethyl, 2-Chlorphenyl, 2-Bromphenyl, 3-Fluorphenyl, 2,3-Dichlorphenyl, 2-Methylphenyl, 4-Methylphenyl, 3-Ethylphenyl, 4-Propylphenyl, 4-Isopropylphenyl, 4-Butylphenyl, 4-tert-Butylphenyl, 4-Iso-butylphenyl, 4-Pentylphenyl, 2,4-Dimethylphenyl, 2-Trifluormethylphenyl, 3-Trifluormethylphenyl, 4-Trifluormethylphenyl, 2-Methoxyphenyl, 4-Methoxyphenyl, 3-Ethoxyphenyl, 2-Propoxyphenyl, 4-Butoxyphenyl, 2,4-Dimethoxyphenyl, 3,4,5-Trimethoxyphenyl.

Aralkyl steht im allgemeinen für einen über ein Alkylenkette gebundenen Arylrest mit 7 bis 14 Kohlenstoffatomen, wobei der Aromat mit einer oder mehreren Niederalkylgruppe(n), Alkoxygruppe(n), Alkoxycarbonylgruppe(n), Hydroxygruppe(n), Cyanogruppe(n), Nitrogruppe(n), Aminogruppe(n) und/oder einem oder mehreren Halogenatom(en) - untereinander gleich oder verschieden - substituiert sein kann. Bevorzugt werden Aralkylreste mit 1 bis 6 Kohlenstoffatomen im Aliphatischen Teil und 6 bis 10 Kohlenstoffatomen im aromatischen Teil. Als bevorzugte Aralkylreste seien genannt: Benzyl, Naphthylmethyl, Phenethyl und Phenylpropyl.

Alkoxy steht im allgemeinen für einen über ein Sauerstoffatom gebundenen geradkettigen oder verzweigten Kohlenwasserstoffrest mit 1 bis 18 Kohlenstoffatomen. Bevorzugt ist Niederalkoxyrest mit 1 bis etwa 6 Kohlenstoffatomen. Besonders bevorzugt ist ein Alkoxyrest mit 1 bis 4 Kohlenstoffatomen. Als Beispiele seien Methoxy, Ethoxy, Propoxy, Isopropoxy, Butoxy, Isobutoxy, tert.-Butoxy, Pentoxy, Isopentoxy, Hexoxy, Isohexoxy, Heptoxy, Isoheptoxy, Octoxy oder Isooctoxy genannt.

Die Angabe der Zahl der Kohlenstoffatome bezieht sich, sofern nichts anderes angegeben, auf die Länge der Alkyl-, Alkenyl- oder Alkinylkette ohne die Carbonylfunktion.

Besonders bevorzugt sind anellierte 6-gliedrige Ringe A, worin die Seitenkette -I-$R_b$ in der 8- oder 9-Position des Hetrazepins oder anellierte 5-gliedrige Ringe A, worin die Seitenkette -I-$R_b$ in der 8-Position des Hetrazepins substituiert ist.

Als Heterocyclus im Rahmen der oben angegebenen Definition steht im allgemeinen für einen 5- bis 6-gliedrigen Ring, der als Heteroatome Sauerstoff, Schwefel und/oder Stickstoff enthalten kann und an den ein weiterer aromatischer Ring bevorzugt ein Phenylring ankondensiert sein kann. Bevorzugt sind 5- und 6-gliedrige aromatische Ringe, die einen Sauerstoff, einen Schwefel und/oder bis zu zwei Stickstoffatomen enthalten und die gegebenenfalls benzokondensiert sind. Als besondere Heteroarylreste seien beispielsweise Thienyl, Furyl, Pyridyl, Pyrimidyl, Pyrazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazolyl, Pyrazolyl und Indolyl genannt.

Der Heterocyclus kann durch Halogen, Hydroxy und/oder verzweigtes oder unverzweigtes Alkyl mit 1 bis 4 Kohlenstoffatom(en) substituiert sein.

Beispiele für gegebenenfalls substituierte gesättigte oder ungesättigte heterocyclische 5-, 6- oder 7-gliedrige Ringe bzw. Heteroarylreste sind:

Pyrrol, Pyrrolin, Pyrrolidin, 2-Methylpyrrolidin, 3-Methylpyrrolidin, Piperidin - gegebenenfalls durch $C_1$-$C_4$ Alkyl ein- oder mehrfach substituiert -Piperazin, N-Methylpiperazin, N-Ethylpiperazin, N-n-Propylpiperazin, N-Benzylpiperazin, Morpholin, Thiomorpholin, Imidazol, Imidazolin, Imidazolidin, Triazol, Pyrazol, Pyrazolin, Pyrazolidin, Triazin, 1, 2, 3, 4 - Tetrazin, 1, 2, 3, 5 - Tetrazin, 1, 2, 4, 5 - Tetrazin - wobei die genannten Heterocyclen durch Alkyl mit 1 bis 4 Kohlenstoffatomen - bevorzugt Methyl - substituiert sein können;

Als heterocyclische Reste, die über ein Kohlenstoffatom verknüpft sein können, werden beispielsweise

Thiophen, 2-Methylthiophen, Furan, Tetrahydrofuran, 2-Methyltetrahydrofuran, Tetrahydrofuran, 2-Hydroxymethylfuran, α-Pyran, γ-Pyran, 1,3-Dioxolan, 1,2-Oxathiolan, 1,2-Oxathiepan, Tetrahydro-pyran, Thiolan, 1,3-Dithian, 1,3-Dithiolan, 1,3-Dithiolen, genannt, wobei der Heterocyclus durch $C_1$-$C_4$ Alkyl, $C_1$-$C_4$ Alkoxy, Halogen substituiert sein kann.

Als Heterocyclus im Rahmen der zuvor angegebenen Definition steht im allgemeinen für einen 5- bis 6-gliedrigen Ring, der als Heteroatome Sauerstoff, Schwefel und/oder Stickstoff enthalten kann, wie zum Beispiel Thienyl, Furyl, Pyridyl, Pyrimidyl, Pyrazinyl, Pyrazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazolyl, Pyrazolyl und Indolyl genannt.

Der Heterocyclus kann durch Halogen, Hydroxy und/oder verzweigtes oder unverzweigtes Alkyl mit 1 bis 4 Kohlenstoffatomen verzweigtes oder unverzweigtes Alkoxy mit 1 bis 4 Kohlenstoffatomen substituiert sein.

Bekanntlich handelt es sich bei PAF (Plättchen Aktivierender Faktor) um das Phospholipid Acetylglyceryl-ether-phosphoryl-cholin (AGEPC), das als potenter Lipidmediator bekannt ist, der von tierischen und menschlichen proinflammatorischen Zellen freigesetzt wird. Unter solchen Zellen finden sich hauptsächlich basophile und neutrophile Granulozyten, Makrophagen (aus Blut und Gewebe) sowie Thrombozyten, die an Entzündungsreaktionen beteiligt sind.

PAF zeigt im pharmakologischen Experiment Bronchokonstriktion, Blutdrucksenkung, Auslösung einer Thrombozytenaggregation sowie eine proinflammatorische Wirkung.
Diese experimentell nachweisbaren Wirkungen des PAF weisen direkt oder indirekt auf mögliche Funktionen dieses Mediators in der Anaphylaxie, in der Pathophysiologie des Asthma bronchiale und allgemein in der Entzündung hin.

PAF-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, zu behandeln. Beispiele für die Indikationen eines PAF-Antagonisten sind Entzündungsprozesse des Tracheobronchialbaumes (akute und chronische Bronchitis, Asthma bronchiale) oder der Niere (Glomerulonephritis), der Gelenke (rheumatische Erkrankungen), anaphylaktische Zustände, Allergien und Entzündungen im Bereich der Schleimhäute und der Haut (z.B. Psoriasis) sowie durch Sepsis, Endotoxine oder Verbrennungen bedingte Schockzustände. Weitere wichtige Indikationen für einen PAF-Antagonisten sind Läsionen und Entzündungen im Bereich der Magen- und Darmschleimhaut, wie z.B. Gastritis, im allgemeinen Ulcus pepticum, jedoch insbesondere Ulcus ventriculi und Ulcus duodeni.

Weiterhin eignen sich die erfindungsgemäßen Verbindungen zur Behandlung bei den folgenden Indikationsstellungen:
Obstruktive Lungenerkrankungen, wie z.B. bronchiale Hyperreaktivität, entzündliche Lungenwegserkrankungen, wie z.B. chronische Bronchitis; Herz- Kreislauferkrankungen, wie z.B. Polytrauma, Anaphylaxe, Arteriosklerose, entzündliche Darmerkrankungen, EPH-Gestose (ederma-proteinuria Hypertension), Erkrankungen des extrakorporalen Kreislaufs, ischämische Erkrankungen, entzündliche und immunologische Erkrankungen, Immunmodulation bei Transplantationen von Fremdgeweben, Immunmodulation bei Leukämie, Metastasenausbreitung z.B. bei bronchialer Neoplasie, Erkrankungen des ZNS, wie z.B. Migräne, Agoraphobie (panic disorder), weiterhin erweisen sich die erfindungsgemäßen Verbindungen als Cyto- und Organoprotektiv, z.B. zur Neuroprotektion, z.B. bei Leberzirrhose, DIC (disiminierte intravasale Gerinnung); Nebenwirkungen einer Arzneimitteltherapie, z.B. anaphylaktoide Kreislaufreaktionen, Kontrastmittelzwischenfälle, Nebenwirkungen bei der Tumortherapie; haemolytik uremic syndrome; Unverträglichkeiten bei Bluttransfusionen; fulminantes Leberversagen ($CCl_4$-Intoxikation) Amanitaphalloides-Intoxikation (Knollenblätterpilzvergiftung); Symptome von parasitären Erkrankungen (z.B. Wurmerkrankungen); Autoimmunerkrankungen. Weiterhin sind folgende Indikationen von Interesse: Immunfunktion bei Aids, Diabetes, juvenile Diabetes, diabetische Retinopathie, polytraumatischer Schock, hämorrhagischer Schock, CNS: Ischämie, Multiple Sklerose, Migräne, Colitis ulcerosa, Morbus Crohn, Psoriasis, pulmonaler Hochdruck sowie chronische ischämische Herzinsuffizienz, PAF Antagonisten der allgemeinen Formel I eignen sich zur Behandlung von pahtologischen Blutgasveränderungen, wie beispielsweise respiratorische Acidose, metabolische Alkalose. In Kombination mit Anticholinergica können PAF-Antagonisten zur Verbesserung der Blutgaswerte bei Phosphorsäureestervergiftungen eingesetzt werden. Es ist bekannt, daß PAF-Antagonisten allein - oder in Kombination mit immunsuppressiv wirkenden Verbindungen (z.B. Cyclosporinen) zur Behandlung von Autoimmunerkrankungen und bei Transplantationen eingesetzt werden können.

Weiterhin wird vorgeschlagen, PAF-Antagonisten in Kombination mit Antihystaminica zu verwenden. Bezüglich der Definition der Antihistaminica wird inhaltlich auf die Europäische Patentanmeldung 345 731 Bezug genommen. Ferner ist bekannt, daß PAF-Antagonisten in Kombination mit $\beta_2$-Mimetica zur Behandlung des Astham bronchiale eingesetzt werden können.

Auch die Kombination von PAF-Antagonisten mit TNF sind vorteilhaft.

PAF assoziierte Interaktion mit Gewebshormon (autocoid hormones), Lymphokine und anderen Mediatoren.

Die neuen Hetrazepine sind sehr starke PAF-Antagonisten und anderen bekannten diazepinoiden PAF-Antagonisten in folgenden Kriterien überlegen:

- es besteht eine totale Dissoziation zwischen dem PAF-Antagonismus und den Benzodiazpin-Receptor vermittelten Effekten;
- überlegende Bindungsaffinität zum PAF-Rezeptor auf gewaschenen Humanplättchen und zeigen eine stärkere Hemmung der PAF-induzierten Plättchenaggregation;
- Darüber hinaus hemmen sie in einer überlegenen Weise die PAF-(30 ng/kg x min) induzierte Bronchokonstriktion nach oraler und parenteraler Applikation am Meerschweinchen in Kombination mit einer sehr langen Wirkdauer (größer 15 Std. nach oraler Applikation am Meerschweinchen).

Die nachfolgende Tabelle zeigt einige ausgewählte Verbindungen mit den dazugehörigen Werten zur Hemmung der PAF-induzierten Plättchenaggregation.

| Beispiel | PAF-induzierte Thrombozythenaggregation [µmol] |
|---|---|
| 1 | 0.017 |
| 2 | 0.07 |
| 3 | 0.3 |
| 4 | 0.8 |
| 5 | 0.1 |

Methodik

Gesunden männlichen und weiblichen Spendern im Alter von 18 bis 35 Jahren, die mehrere Tage vor Blutentnahme keine Medikamente (Aspirin oder andere nicht steroidale Entzündungshemmer) eingenommen hatten, wurden jeweils 200 ml Blut aus einer nicht gestauten Vene mit Hilfe einer Plastikspritze, in der sich 3.8 %-ige Natriumcitratlösung befand, entnommen. Das Verhältnis Natriumcitratlösung: Blut betrug 1:9. Das Citratblut wurde in Plastikröhrchen bei 150 x g ( = 1200 U/min) und Raumtemperatur 20 min lang zentrifugiert (Heraeus Christ Tischzentrifuge 124).

Die Messung der Thrombozytenaggregation in vitro erfolgte nach der Methode von Born und Cross (1963), wobei dem TRP unter ständigem Rühren ein Aggregationsauslöser (PAF) zugesetzt wurde. Zur Messung wurde in 1 ml Plastikküvetten, die jeweils einen kleinen Metallstift (Rührer, 1000 U/min) enthielten, 0.8 ml TRP und 0.2 ml modifizierte Tyrode-Lösung (s. unten) gegeben. Die Prüfsubstanz wurde 2 bis 3 min vor Auslösung der Aggregation in einem Volumen von 10 µl zugesetzt. Als Lösungsmittel dienten entweder DMSO und Wasser oder eine verdünnte $HC_{\omega}$-Lösung. Die Kontrollansätze enthielten das entsprechende Volumen dieser Lösungsmittel. Nach Registrierung der Ausgangsabsorption (2-3 min) wurde die Aggregation induziert. In einem Volumen von 10 µl erfolgte die Gabe von PAF ($5 \times 10^{-8}$M; Bachem Feinchemikalien) in die Küvette.

Die modifizierte Tyrode-Lösung hatte folgende Zusammensetzung: 136.9 mM NaCl; 2.68 mM KCl; 0.5 mM $MgCl_2$; 1.8 mM $CaCl_2$; 0.42 mM $NaH_2PO_4$; 5.55 mM Glucose und 11.9 mM $NaHCO_3$.

Zur Beurteilung von Substanzeffekten wurde das Maximum der ersten Aggregationswelle verwendet. Die durch den Aggregationsauslöser induzierte maximale Absorption (= maximale Aggregation = 100 %) wurde gleichzeitig in einem Parallelansatz (im 2. Kanal des Aggregometers) zu jedem Testansatz mitgeführt und als 100 % Wert verwendet. Der unter der Wirkung der Testsubstanz erreichte Aggregationswert wurde als % des Kontrollwertes (-ansatz) angegeben. Mit Hilfe dieses Verfahrens wurden Konzentrationswirkungskurven mit einem Stichprobenumfang von jeweils n = 4 erstellt und $IK_{50}$-Werte (Konzentration bei einer 50 %igen Aggregationshemmung) berechnet.

Die Herstellung der erfindungsgemäßen Verbindungen kann in Analogieverfahren gemäß dem Stand der Technik erfolgen.

In Anlehnung an die Europäische Patentschrift 194 416 und die Europäischen Patentanmeldungen 230 942 und 254 245 erfolgt die Synthese gemäß nachfolgenden Raktionen.

Ausgehend von dem entsprechend substituierten Diazepinthionen der allgemeinen Formel

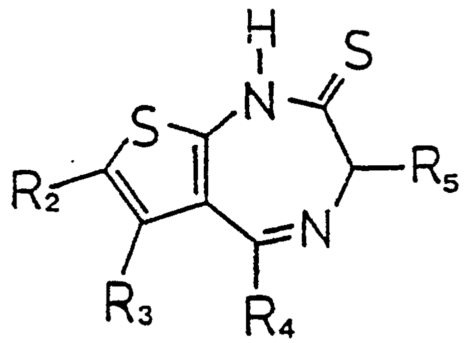

worin $R_2$, $R_3$, $R_4$ und $R_5$ wie zuvor definiert sind, erhält man die erfindungsgemäßen Verbindungen der allgemeinen Formel I dadurch, daß man

A) für den Fall, daß X und Y Stickstoff bedeutet

a) mit einem Säurehydrazid der allgemeinen Formel $R_1$-CONHNH$_2$ (III) umsetzt,

b) oder aber mit Hydrazin in eine Verbindung der allgemeinen Formel

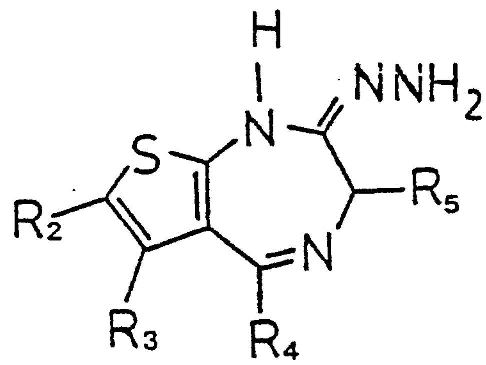

überführt und anschließend mit einem Säurehalogenid der allgemeinen Formel $R_1$-CO-Hal oder mit einem Orthoester der allgemeinen Formel $R_1$-C(OR$'$)$_3$ worin R$'$ eine niedere Alkylgruppe bedeutet, umsetzt, oder

B) Für den Fall, daß X C-H, C-Alkyl und Y Stickstoff

a) mit einem Aminoalkin der allgemeinen Formel$R'_1$ - C≡C - CH$_2$-NH$_2$

worin $R'_1$ Wasserstoff oder eine niedere Alkylgruppe bedeutet

oder aber

b) mit einem α-Aminoaldehyd-alkylacetal oder α-Aminoketon-alkylketal der allgemeinen Formel

$H_2NCH_2$-CR$_1$(OR$'$)$_2$

worin $R_1$ Wasserstoff oder eine Alkylgruppe mit 1 bis 4 Kohlenstoffatomen und R$'$ eine niedere Alkylgruppe bedeutet, umsetzt, anschließend gewünschtenfalls die erhaltenen Verbindungen mit $R_1$ = Wasserstoff in Gegenwart einer Base mit einem Halogenierungsmittel, wie z.B. mit Chlor oder Brom, zu einer Verbindung der allgemeinen Formel Ia mit $R_1$ Halogen, wie z.B. Chlor oder Brom umsetzt; anschließend gewünschtenfalls die Halogenverbindung in eine Verbindung der allgemeinen Formel Ia mit $R_1$ =Alkoxy mit 1 bis 4 Kohlenstoffatomen durch Reaktion mit dem entsprechenden Alkoholat überführt; und anschließend gegebenenfalls die so erhaltenen Verbindungen mittels an sich bekannter Trennverfahren in ihre optisch aktiven Verbindungen spaltet, und gegebenenfalls die so erhaltenen Verbindungen in ihre unbedenklichen Säureadditionssalze überführt.

Die Umsetzung des Thions II mit einem Säurehydrazid III nach dem Verfahren a) erfolgt in einem inerten organischen Lösungsmittel, wie z.B. Dioxan, Dimethylformamid, Tetrahydrofuran oder einem geeigneten Kohlenwasserstoff, wie z.B. Benzol oder Toluol bei Temperaturen zwischen der Raumtemperatur und dem Siedepunkt des Reaktionsgemisches. Die Isolierung der Endprodukte geschieht nach bekannten Methoden, wie z.B. durch Kristallisation.

Die Umsetzung des Thions II mit Hyrazin nach dem Verfahren b) erfolgt in inerten organischen Lösungsmitteln, wie beispielsweise Tetrahydrofuran, Dioxan, halogenierten Kohlenwasserstofen, wie z.B. Methylenchlorid, geeigneten Kohlenwasserstoffen, bei Temperaturen zwischen Raumtemperaturen und dem Siedepunkt des Reaktionsgmisches.

Die dabei entstehenden Hydrazin-1,4-diazepine können nach herkömmlichen Verfahren isoliert oder aber auch direkt weiterverarbeitet werden.

Die weitere Umsetzung mit einem Säurehalogenid oder Orthoester erfolgt in einem inerten organischen Lösungsmittel, wie z.B. halogenierten Kohlenwasserstoffen, cyclischen oder aliphatischen Estern, kann aber auch direkt in Substanz erfolgen. Die Isolierung des Endprodukts Ia erfolgt nach bekannten Methoden, beispielsweise durch Kristallisation. Weitere Einzelheiten des Herstellverfahrens sind dem ausführlichen Reaktionsschema sowie den Beispielen zu entnehmen.

Nach dem beanspruchten Verfahren können bevorzugt solche Verbindungen hergestellt werden, die in der Seitenkette $R_2$ = - G - $R_a$ oder $R_2/R_3$ = A-$J_m$-$R_b$ als Rest für $R_a$ bzw. $R_b$ Wasserstoff, einen Alkylrest, eine Carbonsäure oder einen Carbonsäureester, Carbonsäureamide, eine Hydroxygruppe, einen Ether- oder Thioetherrest, ein Amin, eine Alkyl-oder Arylsulfonyloxygruppe aufweisen. Andere beanspruchten Reste können vorteilhafterweise durch Folgereaktionen in Analogie zu den in den europäischen Patentanmeldungen 230 942 und 254 245 offenbarten Verfahren - auf die hiermit inhaltlich Bezug genommen wird - hergestellt werden.

Der erfindungsgemäß beanspruchte Substituent $R_4$ wird als Cyanomethylketon-Derivat auf der Stufe der Gewald-Synthese (7 - 8) in das Molekül eingeführt.

Diese sind durch Cyanmethylierung der entsprechenden Carbonsäureester - insbesondere Carbonsäurmethylester -in Gegenwart von Natriumhydrid erhältlich.

Verbindungen der allgemeinen Formel

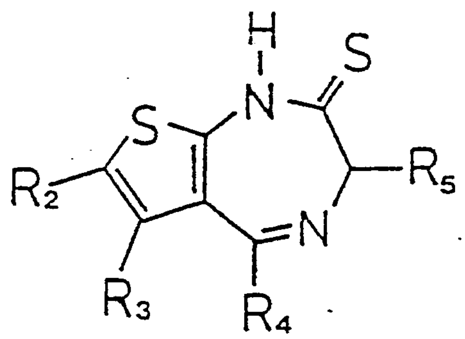

worin $R_2$, $R_3$ und $R_4$ wie zuvor definiert sind, sind wertvolle Ausgangsprodukte zur Herstellung von Hetrazepinen mit PAF-antagonistischer Wirkung und werden als solche beansprucht.

Cyanomethylketone der allgemeinen Formel

$R_4$ - $CH_2$ - CN

sind ebenfalls wertvolle Ausgangsprodukte zur Synthese pharmakologisch wirksamer Verbindungen und werden als solche beansprucht.

Der Aufbau des Grundgerüstes der erfindungsgemäßen Verbindungen erfolgt auf an sich bekannte Weise [EP-OS 254 245 und EP-PS 194 416] und ist dem folgenden Reaktionsschema retrosynthetisch skizziert; jedoch ohne das Verfahren auf die gezeigten Reste einzuschränken.

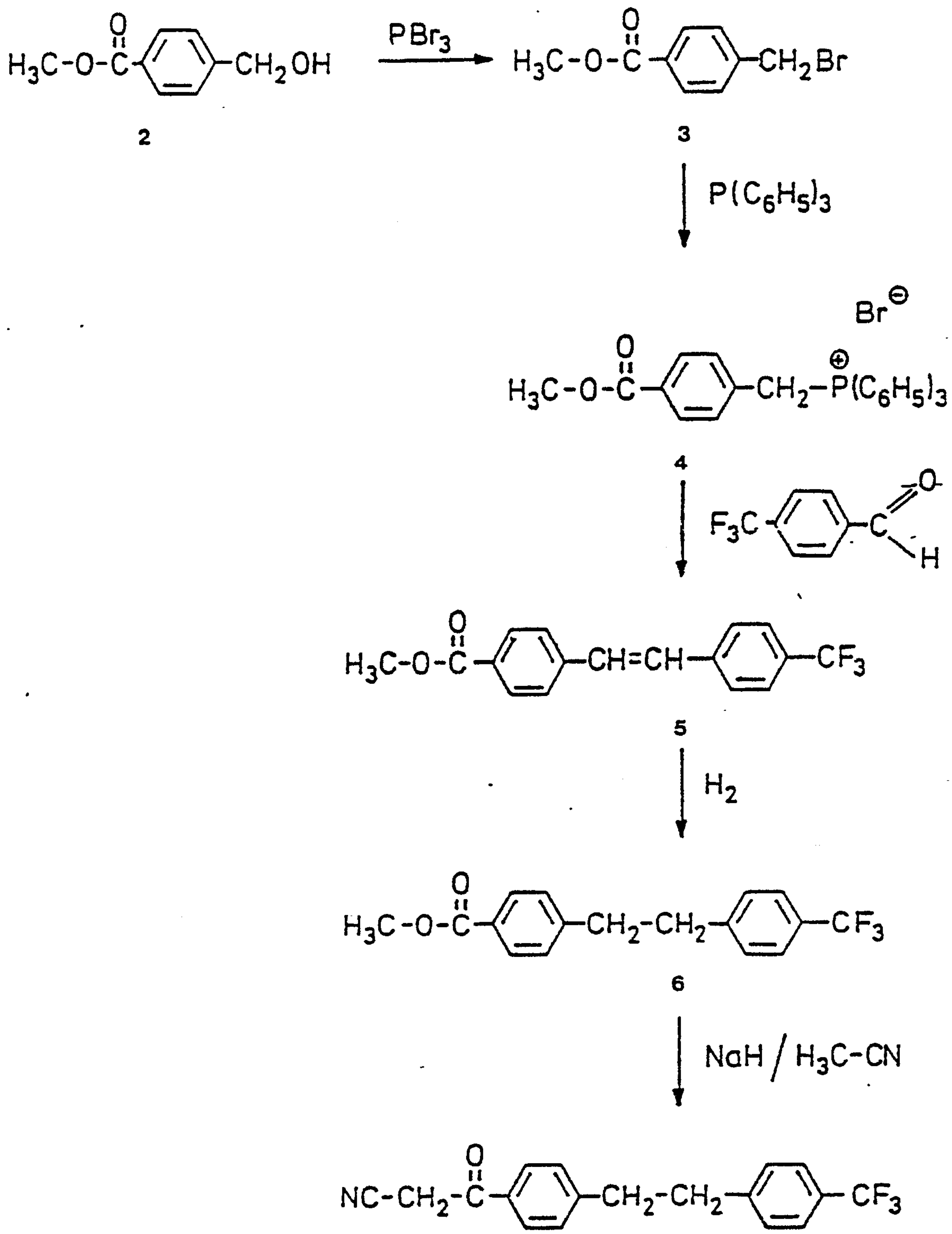


2

3

4

5

6

7

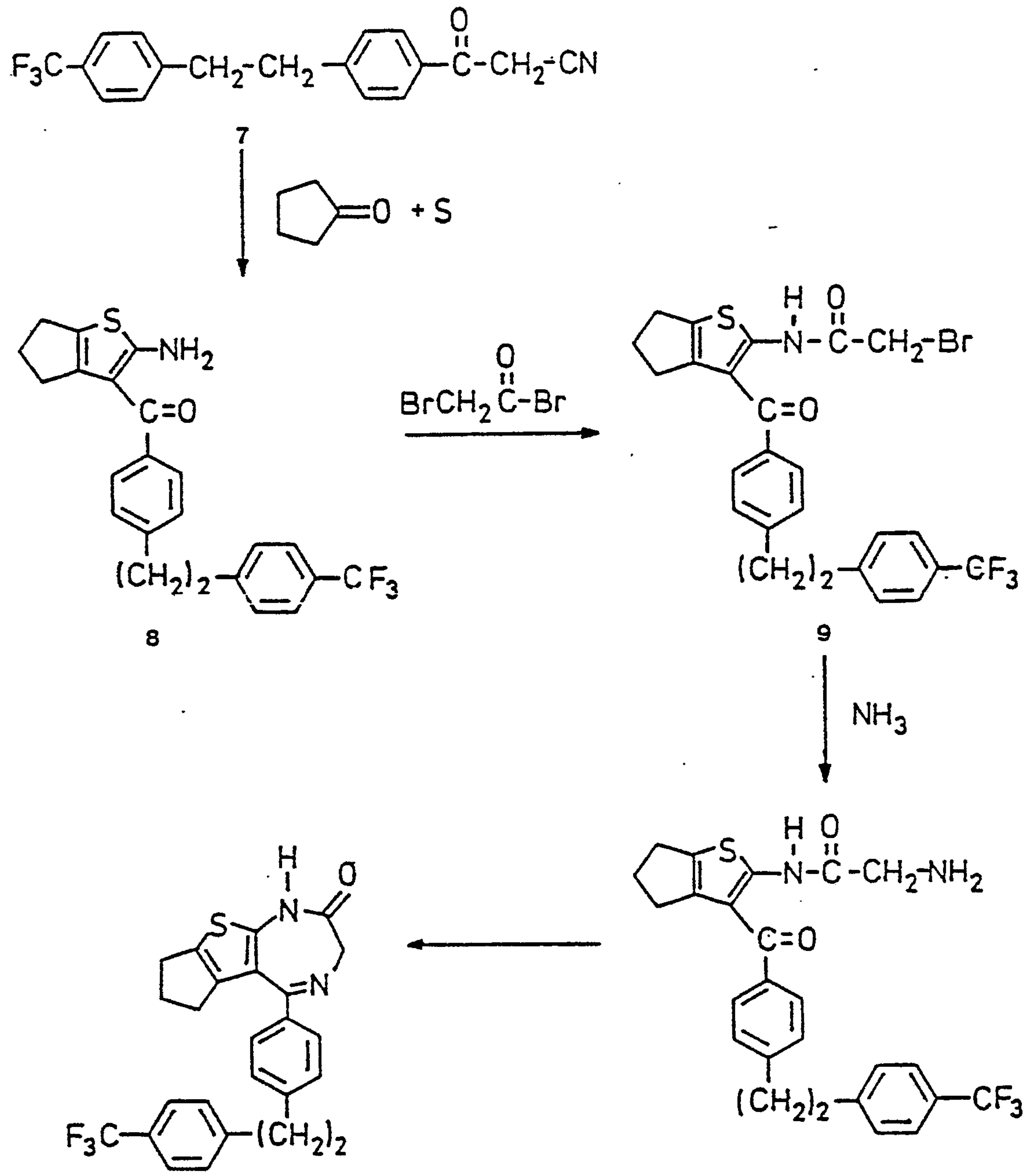


7

8

9

10

11

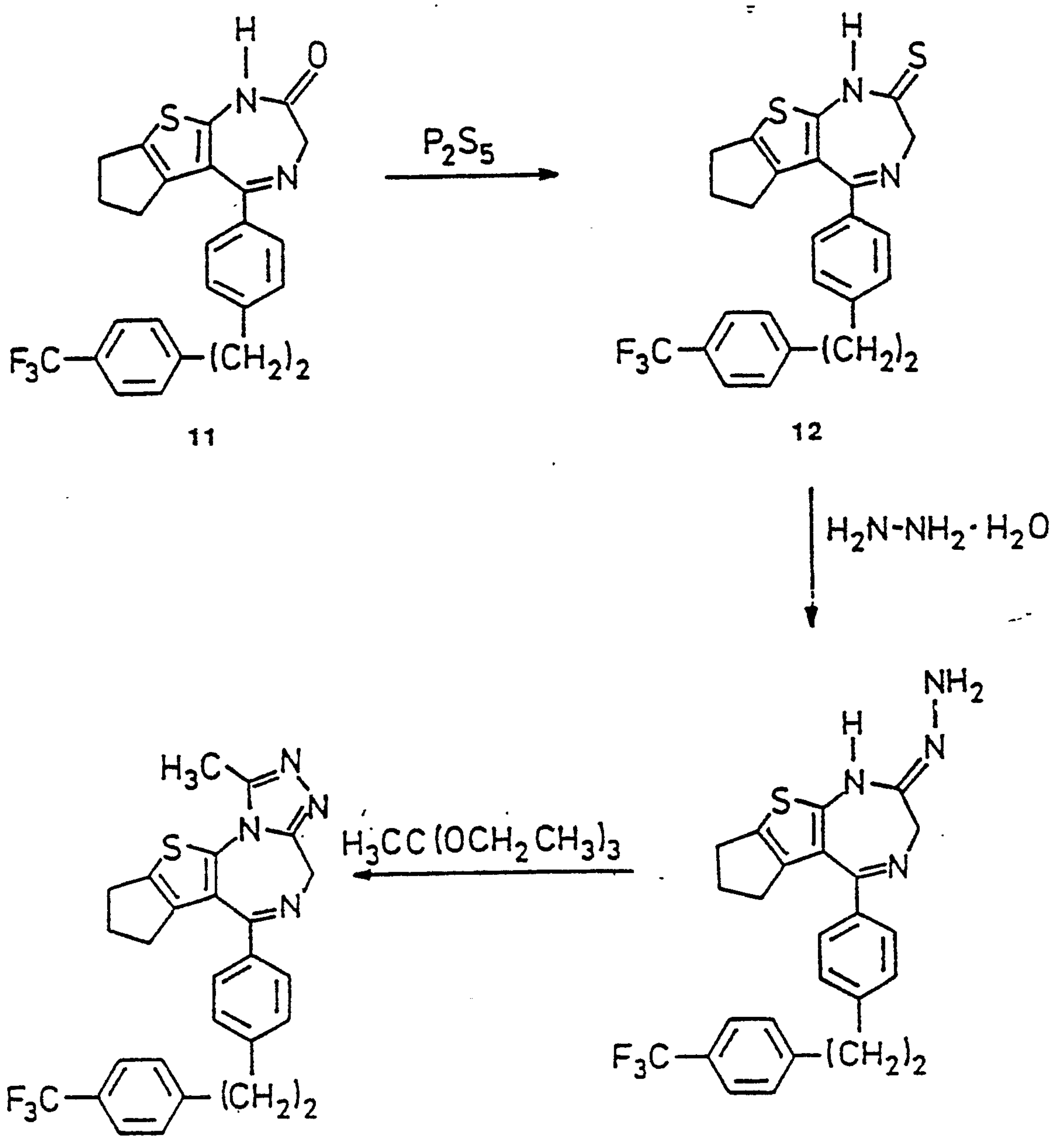


11 12 13 14

Die nachfolgenden Beispiele sollen die Erfindung erläutern ohne sie einzuschränken:

Beispiel 1

6-(4-(4-Trifluormethyl-phenethyl)-phenyl)-8,9-dihydro-1-methyl-4H,7H-cyclopenta[4,5]-thieno[3,2-f][1,2,4]-triazolo[4,3-a][1,4]-diazepin (14)

20 g (42,7 mmol) der Hydrazinoverbindung (13) werden in 200 ml Ethanol suspendiert. Nach Zugabe von 20 ml Orthoessigsäuretriethylester wird die Lösung 1 Stunde unter Rückfluß gerührt. Anschließend wird der Alkohol im Vakuum abdestilliert und der verbleibende Rückstand über eine $SiO_2$-Säule chromatographiert. Zum Eluieren benutzt man ein Methylenchlorid/Methanol-Gemisch (96:4). Die Hauptfraktion wird eingedampft und der Rückstand aus Isopropanol umkristallisiert. Man erhält 6 g (28 % d. Th.) der Titelverbindung vom Schmp.: 245-246 °C.

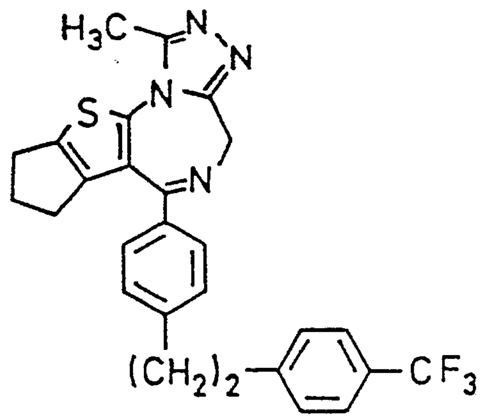

$^1$H-NMR ($CDCl_3$; 250 MHz): δ = 7.49, 7.18 (4H, m, Aryl-H); 7.39, 7.09 (4H, 2d, J = 9 Hz, Aryl-H); 5.51, 4.14 (2H, AB-System, $J_{AB}$ = 13 Hz, $CH_2$-7-Ring); 2.98 (6H, m, $CH_2$-4; $CH_2CH_2$); 2.69 (3H, s, $CH_3$-Triazol); 2.40 -2.02 (4H, m, $CH_2CH_2$-3).

Die Ausgangsverbindung 13 wird wie folgt erhalten:

In 300 ml Tetrahydrofuran werden 20 g (43 mmol) des Deazepinthions 12 vorgelegt. Nach Zufügen von 2,4 ml Hydrazinhydrat wird die entstandene Lösung 1 Stunde bei Raumtemperatur gerührt. Nach dem Abdestillieren des Lösungsmittels erhält man 18 g (90 % d. Th.) der Hydrazinoverbindung 13, die als Rohprodukt weiterverarbeitet werden kann.

Das Diazepinthion 12 gewinnt man durch folgende Umsetzung:

20 g (44 mmol) des Diazepinons 11 werden mit 7,5 g Natriumhydrogencarbonat und 10,8 g Phosphorpentasulfid in 370 ml Diethylenglykoldimethylether 3 Stunden bei 65 -70 °C gerührt. Die Reaktionslösung wird unter Rühren auf Eis gegeben und der entstandene Niederschlag nach einer Stunde abgesaugt. Diesen Niederschlag löst man in Methylenchlorid auf, trocknet mit Natriumsulfat und destilliert das Lösungsmittel nach dem Entfernen des Trockenmittels vorsichtig im Vakuum ab. Der verbleibende Rückstand wird mit Isopropylether aufgeschlemmt und die Kristalle abgesaugt. Man erhält 20 g der Verbindung 12 (97 % d. Th.) vom Schmp. 155-165 °C.

Die Verbindung 11 synthetisiert man auf folgendem Weg:

42 g (89 mmol) der Aminobenzoylverbindung 10 werden mit 250 g $SiO_2$ in 1,5 l Toluol vorgelegt und unter kräftigem Rühren 3 Stunden am Rückfluß gekocht, wobei man das entstehende Wasser in einer Spezialvorlage sammelt. Nach dem Abdenkantieren des Toluols wird der verbleibende Rückstand 3 mal mit Methanol ausgekocht. Die einzelnen Methanolfraktionen werden vereinigt und im Vakuum eingedampft. Der verbleibende Rückstand kristallisiert nach Zugabe von Isopropylether. Man erhält 26 g (64 % d. Th.) des Diazepinons 11 vom Schmp.: 223 °C.

Die oben eingesetzte Verbindung 10 wird folgendermaßen erhalten:

43 g (91 mmol) der Bromacetylverbindung 9 werden in 800 ml Essigester gelöst. Man leitet 2 Stunden einen kräftigen Ammoniakstrom in das Reaktionsgemisch und rührt bei Raumtemperatur 12 Stunden nach. Anschließend wird die Suspension 3 mal mit Wasser gewaschen, die organische Phase abgetrennt, mit Natriumsulfat getrocknet und nach Entfernen des Trockenmittels anschließend im Vakuum eingedampft. Es hinterbleiben 35 g Aminobenzoylverbindung 10 (92 % d. Th.)

Die Verbindung 9 erhält man auf folgendem Weg:

60 g (141 mmol) Aminoketon 8 werden in 450 ml Dioxan suspendiert. Man fügt 12 ml Pyridin hinzu und tropft bei Raumtemperatur 13 ml Bromacetylbromid langsam ein. Anschließend wird eine Stunde bei Raumtemperatur nachgerührt. Die Suspension wird über Krieselgur abfiltriert und das Filtrat im Vakuum eingedampft. Es verbleibt ein öliger Rückstand, den man über einer $SiO_2$-Säule chromatographiert (Methylenchlorid). Nach dem Eindampfen der Hauptfraktion erhält man 43 g der Verbindung 9 in Form gelber Kristalle (55 % d. Th.) vom Schmp.: 145-147 °C.

Die oben eingesetzte Verbindung 8 wird wie folgt hergestellt:

155 g (488 mmol) der Verbindung 7,46 g Cyclopentanon und 17,4 g Schwefel werden in 270 ml Dimethylformamid suspendiert bzw. gelöst. Man fügt anschließend 38 ml Triethylamin hinzu und rührt 3 Stunden bei 60 °C. Nach dem Abkühlen wird das Reaktionsgemisch mit Essigester verdünnt und die organische Phase 2 mal mit Wasser gewaschen. Nach Abdampfen der Lösungsmittel im Vakuum wird der Rückstand auf eine $SiO_2$-Säule gegeben und das gewünschte Produkt mit Methylenchlorid eluiert. Der Rückstand läßt sich durch Zugabe von Isopropylether/Petrolether kristallisieren. Man erhält 64 g des

gewünschten Aminoketons 8 (32 % d. Th.) vom Schmp. 140-150° C.

Das Cyanoketon 7 wird wie folgt synthetisiert:

33 g (0,1 mol) des Esters 6 werden in 100 ml Toluol gelöst. Man fügt 4,8 g einer 55 %-igen Natriumhydrid-Dispersion und anschließend 6,9 ml Acetonitril hinzu und kocht das Reaktionsgemisch 6 Stunden unter Rückfluß. Nach dem Abkühlen wird mit verdünnter Salzsäure auf pH 5 bis 6 angesäuert. Die Suspension wird 3 mal mit Methylenchlorid extrahiert, die organischen Extrakte getrocknet und im Vakuum eingedampft. Den Rückstand chromatographiert man über eine $SiO_2$-Säule wobei Methylenchlorid als Elutionsmittel benutzt wird. Die Hauptfraktion wird eingedampft, der Rückstand kristallisiert. Ausbeute: 15,5 g (46 % d. Th.), Schmp.: 105° C.

Der oben eingesetzte Ester 6 kann wie folgt hergestellt werden:

37 g (0,12 mol) des Olefins 5 wird in 600 ml Tetrahydrofuran mit Raney-Nickel als Katalysator bei 20° C und 5 bar hydriert. Nach Absaugen des Katalysators dampft man das Tetrahydrofuran ab und erhält 33,5 g der Verbindung 6 als weiße Kristalle (90 % d. Th.) vom Schmp.: 88-90° C.

Das oben benutzte Olefin 5 kann durch Wittig-Olefinierung gewonnen werden:

In einer Stickstoff-Atmosphäre werden 8,7 g 55 %-ige Natriumhydrid-Dispersion in 100 ml absolutes Dimethylsulfoxid gegeben und 45 Minuten bei 80 ° C gerührt. Nach Abkühlen auf Raumtemperatur tropft man eine Suspension von 98 g (0,2 mol) des Phosphoniumsalzes 4 ein und rührt das Reaktionsgemisch 10 Minuten nach. Darauf werden 34,8 g Trifluormethylbenzaldehyd eingetropft und 2 Stunden bei Raumtemperatur gerührt. Anschließend wird das Gemisch mit 400 ml Essigester verdünnt und 2 mal mit Wasser gewaschen. Die organische Phase wird getrocknet und der Rückstand chromatographiert ($SiO_2$-Säule, Elutionsmittel: Methylenchlorid). Die Hauptfraktion wird im Vakuum eingedampft und der Rückstand aus Isopropylether umkristallisiert. Man erhält 37,5 g (61 % d. Th.) des Stilbenderivates 5 als Kristalle vom Schmp.: 157-158° C

Das Phosphoniumsalz 4 ist wie folgt erhältlich:

13,3 g (58 mmol) der Verbindung 3 werden mit 15,1 g Triphenylphosphin in 130 ml Benzol 6 Stunden unter Rückfluß gekocht. Die bei der Reaktion ausgefallenen Kristalle werden abgesaugt und getrocknet. Man erhält 27,4 g (96 % d. Th.) des Phosphoniumbromids 4 als Kristalle vom Schmp. 258-260° C. Das für die Darstellung des bei der Wittig-Olefinierung eingesetzten Phosponiumsalzes 4 als Ausgangsmaterial eingesetzte Alkylbromid 3 läßt sich wie folgt herstellen:

11,5 g (70 mmol) 4-Hydroxymethylbenzoesäuremethylester werden in 120 ml Methylenchlorid gegeben und mit 47,3 ml Phosphortribromid versetzt. Man rührt eine Stunde bei Raumtempertur und gibt unter Eiskühlung vorsichtig halbkonzentrierte Lösung von Ammoniak in Wasser hinzu bis ein pH-Wert von 8 bis 9 erreicht ist. Die organische Phase wird abgetrennt, getrocknet und das Lösungsmittel abdestilliert. Man erhält 13,4 g (84 % d. Th.) des gewünschten Bromids 3.

Beispiel 2

6-(4-(4-Chlorphenethyl)-phenyl)-8,9-dihydro-1-methyl-4H,7H-cyclopenta[4,5]thieno[3,2-f][1,2,4]triazolo[4,3-a]-[1,4]diazepin

Die Verbindung wird analog Beispiel 1 aufgebaut. Bei der Wittig-Olefinierung benutzt man p-Chlorbenzaldehyd an Stelle von 4-Trifluormethylbenzaldehyd.

Das entsprechende Aminoketon wird in Form eines gelben Öles erhalten.

Die Titelverbindung schmilzt bei 198-199° C.

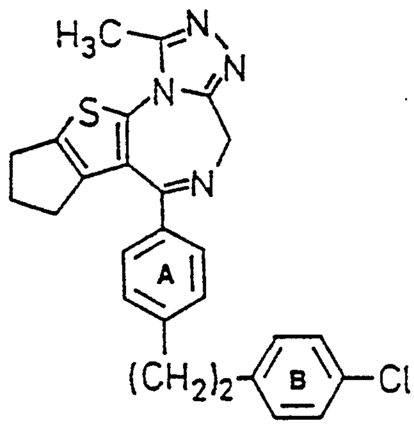

$^1$H-NMR ($CDCl_3$; 250 MHz): δ = 7.37, 7.08 (4H, 2d, J = 8 Hz, Aryl-H, Ring A); 7.18, 7.00 (4H, 2d, J = 9 Hz, Aryl-H, Ring B); 5.51, 4.13 (2H, 2d, breit, AB-System, $CH_2$-7-Ring); 2.98 (2H, m, $CH_2$-4); 2.69 (3H, s, $CH_3$); 2.40 - 2.05 (4H, m, $CH_2$-$CH_2$-3).

Beispiel 3

6-(4-(4-Methoxyphenethyl)-phenyl)-8,9-dihydro-1-methyl-4H,7H-cyclopenta[4,5]thieno[3,2-f][1,2,4[triazolo-[4,3-a][1,4]diazepin

Diese analog zu Beispiel 1 hergestellte Verbindung liegt in Form farbloser Kristalle vom Schmp.: 185-188° C vor.

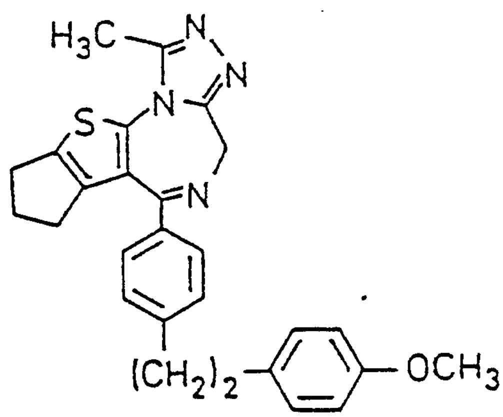

$^1$H-NMR ($CDCl_3$; 250 MHz): δ = 7.37, 7.12, 7.00, 6.77 (8H, 4d, J = 9 Hz, Aryl-H); 5.51, 4.13 (2H, AB-System, $J_{AB}$ = 12 Hz); 3.76 (3H, s, $OCH_3$); 2.96 (2H, m, $CH_2$-H); 2.88 (4H, m, $CH_2CH_2$); 2.69 (3H, s, $CH_3$-Triazol); 2.45 - 2.07 (4H, m, $CH_2CH_2$-3).

Beispiel 4

6-(4-(3,4,5-Trimethoxyphenethyl)-phenyl)-8,9-dihydro-1-methyl-4H,7H-cyclopenta[4,5]thieno[3,2-f][1,2,4]-triazolo[4,3-a][1,4]diazepin

Hergestellt auf analoge Weise über das bei 180-182° C schmelzende Aminoketon.

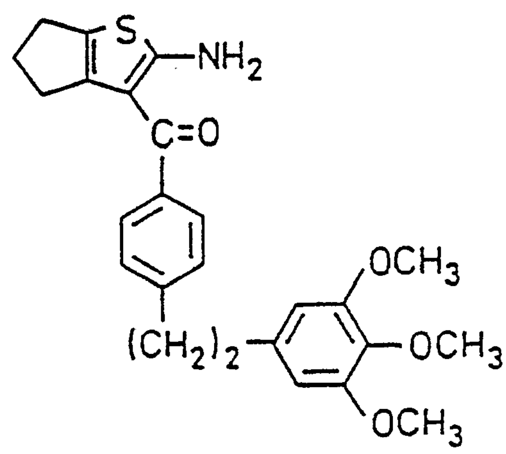

$^1$H-NMR ($CDCl_3$; 250 MHz): δ = 7.39, 7.19 (4H, 2d, J = 8 Hz, Aryl-H); 6.93 (2H, s, breit, $NH_2$); 6.38 (2H, s, Aryl-H); 3.82, 3.81 (9H, 2s, 3-$OCH_3$); 2.92 (4H, m, $CH_2CH_2$); 2.67 (2H, m, $CH_2$-4); 2.11 (4H, m, $CH_2CH_2$-3).

Die Verbindung wird in Form von Kristallen vom Schmp.: 175-177 °C gewonnen.

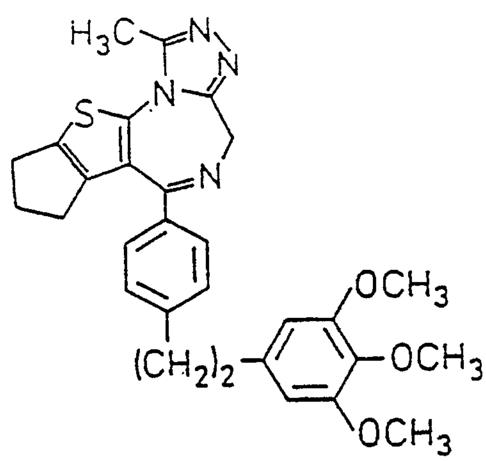

$^1$H-NMR ($CDCl_3$; 250 MHz): δ = 7.40, 7.16 (4H, 2d, J = 9 Hz, Aryl-H); 6.36 (2H, s, Aryl-H); 5.52, 4.14 (2H, AB-System, $J_{AB}$ = 13 Hz, $CH_2$-7-Ring); 3.82, 3.81 (9H, 2s, 3-$OCH_3$); 2.98 (2H, m, $CH_2$-4); 2.90 (4H, m, $CH_2CH_2$); 2.70 (3H, s, $CH_3$-Triazol); 2.43 - 2.09 (4H, m, $CH_2CH_2$-3).

Beispiel 5

6-(4-(Isopropylphenethyl)-phenyl)-8,9-dihydro-1-methyl-4H,7H-cyclopenta[4,5]thieno[3,2-f][1,2,4]triazolo[4,3-a][1,4]diazepin

Auch diese Verbindung wird analog Beispiel 1 synthetisiert. Das Aminoketon schmilzt bei 141-142 °C.

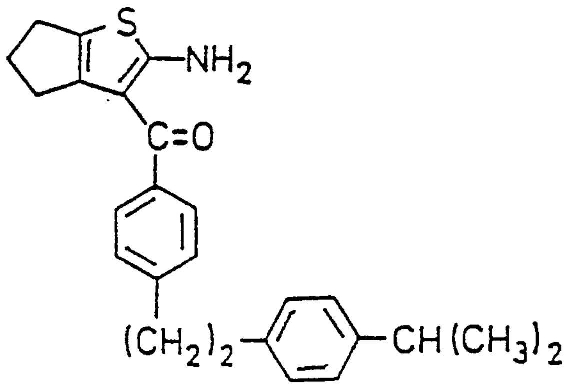

$^1$H-NMR ($CDCl_3$; 250 MHz): δ = 7.43 - 7.00 (8H, m, Aryl-H); 6.88 (2H, s, breit, $NH_2$); 2.93 (4H, m, $CH_2CH_2$); 2.88 (1H, sep, J = 6 Hz, CH ); 2.67 (2H, m, $CH_2$-4); 2.14 (4H, m, $CH_2CH_2$-3); 1.24 (6H, d, J = 6 Hz, $(CH_3)_2$-C).

Das Endprodukt schmilzt bei 198-200 °C.

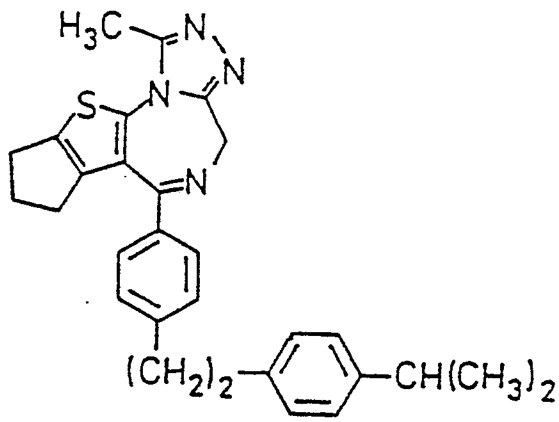

$^1$H-NMR (DMSO-d6; 250 MHz): δ = 7.37, 7.24 (4H, 2d, J = 8 Hz, Aryl-H, Ring A); 7.09 (4H, s, Aryl-H, Ring-B); 5.25, 4.14 (2H, 2d, AB-System, $J_{AB}$ = 13 Hz, $CH_2$-7-Ring); 2.90 (4H, m, $CH_2CH_2$); 2.82 (1H, p, J = 6 Hz, CH ); 2.61 (3H, s, $CH_3$-Triazol); 2.33 - 1.90 (4H, m, $CH_2CH_2$-3); 1.17 (6H, d, J = 6 Hz, $(CH_3)_2$-C).

[Zuordnung Ring B siehe Abbildung in Beispiel 2]

Beispiel 6

6-(4-Carbomethoxyphenyl)-8,9-dihydro-1-methyl-4H,7H-cyclopenta[4,5]thieno[3,2-f][1,2,4]triazolo[4,3-a][1,4]-diazepin

Dieser Ester ist ausgehend von Terephthalsäuredimethylester über das bei 178-180 °C schmelzende Aminoketon das folgendes NMR-Spektrum besitzt:

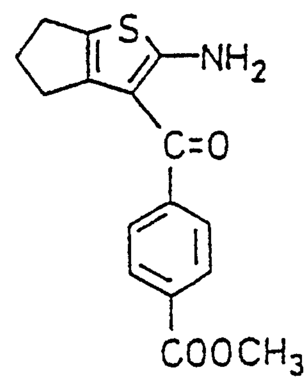

$^1H$-NMR ($CDCl_3$); 250 MHz): δ = 8.07, 7.50 (4H, 2d, J = 8 Hz, Aryl-H); 6.72 (2H, s, breit, $NH_2$); 3.94 (3H, s, $OCH_3$); 2.64 (2H, m, $CH_2$-H); 2.20 - 1.90 (4H, m, $CH_2$-$CH_2$-3).
zugänglich.

Die Titelverbindung schmilzt bei 225° C.

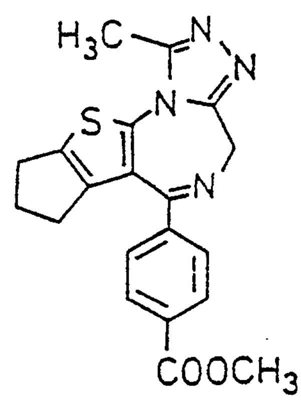

$^1H$-NMR ($CDCl_3$; 250 MHz): δ = 8.03, 7.57 (4H, 2d, J = 9 Hz, Aryl-H); 5.57, 4.18 (2H, AB-System, $J_{AB}$ = 12 Hz, $CH_2$-7-Ring); 3.93 (3H, s, $OCH_3$); 2.98 (2H, m, $CH_2H$); 2.71 (3H, s, $CH_3$-Triazol); 2.42 - 2.01 (4H, m, $CH_2$-$CH_2$-3).

Beispiel 7

6-(4-Hydroxymethylphenyl)-8,9-dihydro-1-methyl-4H,7H-cyclopenta[4,5]thieno[3,2-f][1,2,4]triazolo[4,3-a][1,4]-diazepin

1,9 g (5 mmol) des obigen Carbonsäureesters werden in 30 ml Tetrahydrofuran suspendiert, 0,19 g Lithiumaluminiumhydrid hinzugefügt und 1 Stunde bei Raumtemperatur gerührt. Man fügt unter Kühlung 10 ml gesättigte Ammoniumtartratlösung hinzu, trennt die organische Phase ab, destilliert das Lösungsmittel ab und kocht den Rückstand mit wenig Essigester auf. Die sich bildenden Kristalle werden abgesaugt, mit Essigester gewaschen und getrocknet. Man erhält 0,8 g der Titelverbindung vom Schmp.: 183-185° C.

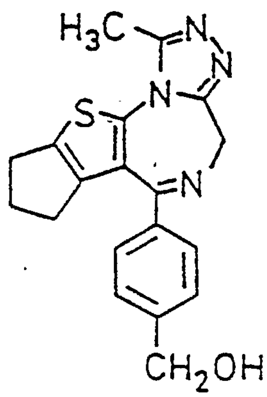

$^1$H-NMR (DMSO-d6; 250 MHz): δ = 7.44, 7.35 (4H, 2d, J = 9 Hz, Aryl-H); 5.30 (1H, t, J = 6 Hz, OH); 5.25, 4.17 (2H, AB-System, $J_{AB}$ = 13 Hz, $CH_2$-7-Ring); 4.55 (2H, d, J = 6 Hz, $OCH_2$); 2.95 (2H, m, $CH_2$-4); 2.62 (3H, s, $CH_3$-Triazol); 2.34 - 1.96 (4H, m, $CH_2CH_2$-3).

Beispiel 8

6-(4-Carboxyphenyl)-8,9-dihydro-1-methyl-4H,7H-cyclopenta[4,5]thieno[3,2-f][1,2,4]triazolo[4,3-a][1,4]-diazepin

3.0 g (7.9 mmol) des nach Beispiel 6 hergestellten Esters werden mit 30 ml einer 2N alkoholischen Kalilauge eine Stunde bei Raumtemperatur gerührt. Man dampft die Reaktionslösung im Vakuum ein, nimmt den Rückstand in 5 bis 10 ml Wasser auf und säuert mit 2N Salzsäure an (pH 6,5-7). Die sich abscheidenden Kristalle werden abgesaugt und bei 60 °C i. Vak. getrocknet. Man erhält 2,5 g der Titelverbindung, die sich ab 205 °C zersetzt.

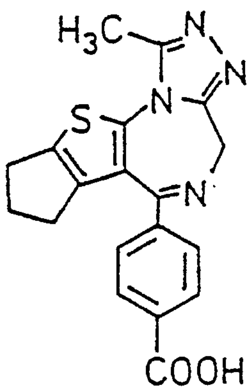

$^1$H-NMR (DMSO-d6; 250 MHz): δ = 7.99, 7.59 (4H, 2d, J = 9 Hz, Aryl-H); 5.34, 4.25 (2H, AB-System, $J_{AB}$ = 12 Hz, $CH_2$-7-Ring); 2.95 (2H, m, $CH_2$-4); 2.63 (3H, s, $CH_3$-Triazol); 2.37-1.86 (4H, m, $CH_2CH_2$-3); COO H stark verbreitert und nicht sichtbar.

Beispiel 9

6-(4-Chloranilinylcarbonyl)phenyl-8,9-dihydro-1-methyl-4H,7H-cyclopenta[4,5]thieno[3,2-f][1,2,4]triazolo[4,3-a][1,4]-diazepin

1,82 g (5 mmol) der in Beispiel 8 hergestellten Säure werden in 20-25 ml absolutem Dimethylformamid suspendiert und mit 0.64 g p-Chloranalin und 0,7 g Hydroxybenztriazol versetzt. Unter Rühren und

Eiskühlung fügt man 1 g Dicyclohexylcarbodiimid hinzu und läßt 48 Stunden bei 0-5° C rühren. Der gebildete Harnstoff wird abgesaugt und das Filtrat i.Vak. eingedampft. Der Rückstand wird in Methylenchlorid gelöst, die Lösung mit Natriumhydrogencarbonat-Lösung geschüttelt, die organische Phase wird abgetrennt, getrocknet und eingedampft. Aus Essigester erhält man Kristalle vom Schmp. 312° C in einer Ausbeute von 1.8 g.

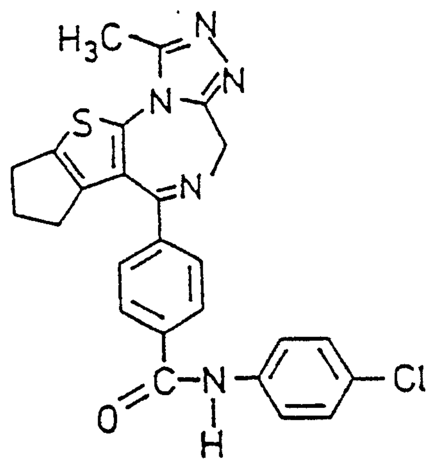

$^1$H-NMR ($CDCl_3$; 250 MHz): δ = 7.91, 7.74, 7.51, 7.30 (8H, 4d, J = 8 Hz, Aryl-H); 5.40, 4.13 (2H, 2d, AB-System, $J_{AB}$ = 13 Hz, $CH_2$-7-Ring); 2.98 (2H, m, $CH_2$-4); 2.63 (3H, s, $CH_3$-Triazol); 2.38-2.03 (4H, m, $CH_2CH_2$-3); 9.30 (1H, s, NH).

Beispiel 10

6-(4-Morpholinylcarbonylphenyl)-8,9-dihydro-1-methyl-4H,7H-cyclopenta[4,5]thieno[3,2-f][1,2,4]triazolo[4,3-a][1,4]diazepin

1.2 g (3,3 mmol) der Carbonsäure aus Beispiel 8 werden in 15 ml Methylenchlorid suspendiert und anschließend mit 0,55 g Carbonyldiimidazol versetzt und bei Raumtemperatur 30 Minuten gerührt. Dann fügt man 0.3 g Morpholin zu, rührt eine Stunde bei Raumtemperatur und schüttelt zum Entfernen unumgesetzter Carbonsäure mit Natriumhydrogencarbonat-Lösung. Die organische Phase wird abgetrennt, eingedampft und der verbleibende Rückstand über eine $SiO_2$-Säule chromatographiert (Elutionsmittel:Methylenchlorid/Methanol 93:7). Man erhält 0,6 g der Titelverbindung.

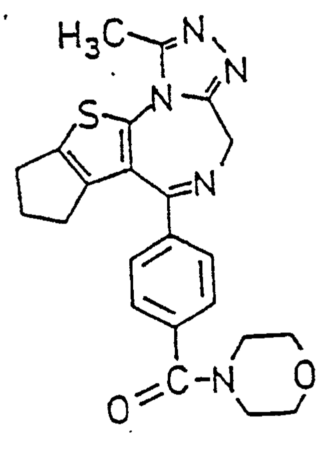

$^1$H-NMR ($CDCl_3$; 250 MHz): δ = 7.57, 7.43 (4H, 2d, J = 9 Hz, Aryl-H); 5.55, 4.18 (2H, AB-System, $J_{AB}$ = 12 Hz, $CH_2$-7-Ring); 3.92-3.20 (8H, m, Morpholin-H); 2.99 (2H, m, $CH_2$-4); 2.71 (3H, s, $CH_3$-Triazol); 2.44-2.12 (4H, m, $CH_2CH_2$-3).

Beispiel 11

2-Ethyl-4-(4-(3,4,5-trimethoxyphenethyl)-phenyl)-9-methyl-6H-thieno[3,2-f][1,2,4]triazolo[4,3-a][1,4]diazepin

Ausgehend von Trimethoxybenzaldehyd und dem im Beispiel 1 eingesetzten Phosphoniumsalz erhält man das 4-(3,4,5-Trimethoxyphenethyl)cyanoacetophenon, welches mit Butyraldehyd und Schwefel nach der Methode von Gewald zu dem entsprechenden Aminothiophen umgesetzt wird (Schmp.: 128-138 °C). Die nachfolgende Umsetzung mit Bromacetylbromid und anschließende Aminierung mit Ammoniak sowie Ringschluß analog Beispiel 1 führt zum entsprechenden Diazepinon (Schmp.: 150-152 °C). Die entsprechende Behandlung des Diazepinons mit $P_2S_5$ und nachfolgend mit Hydrazin und Orthoessigsäuretriethylester liefert die Titelverbindung mit einem Schmp.: 145-147 °C.

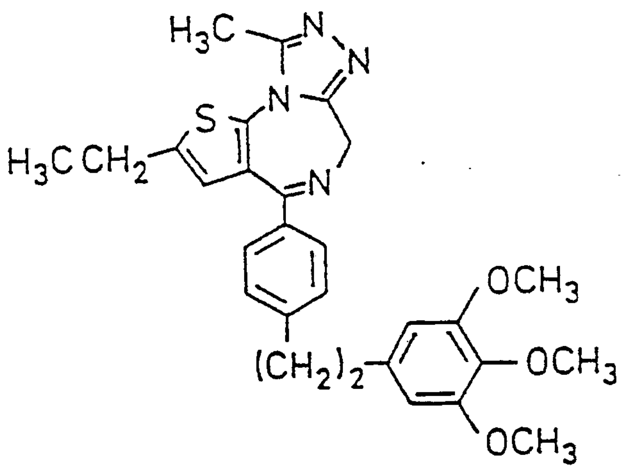

$^1$H-NMR ($CDCl_3$; 250 MHz): δ = 7.52, 7.20 (4H, 2d, J = 8 Hz, Aryl-H); 6.66 (1H, s, Thiophen-H); 6.36 (2H, s, Aryl-H); 4.80 (2H, s, breit, $CH_2$-7-Ring); 3.82, 3.81 (9H, 2s, 3-$OCH_3$); 2.92 (4H, m, $CH_2CH_2$); 2.88 (2H, qu, J = 6 Hz, $\underline{CH}_2$-$CH_3$); 2.70 (3H, s, $CH_3$-Triazol); 1.36 (3H, t, J = 6 Hz, $CH_2$-$\underline{CH}_3$).

Beispiel 12

4-(4-(3,4,5-Trimethoxyphenethyl)-phenyl)-9-methyl-6H-thieno[3,2-f][1,2,4]triazolo[4,3-a][1,4]diazepin

Aus dem 4-(3,4,5-Trimethoxyphenethyl)cyanoacetophenon und Mercaptoacetaldehyd erhält man das entsprechende Aminoketon vom Schmp.: 110-112 °C. Analog Beispiel 11 wird hieraus die Titelverbindung aufgebaut, welche in Form von Kristallen vom Schmp.: 260-261 °C erhalten wird.

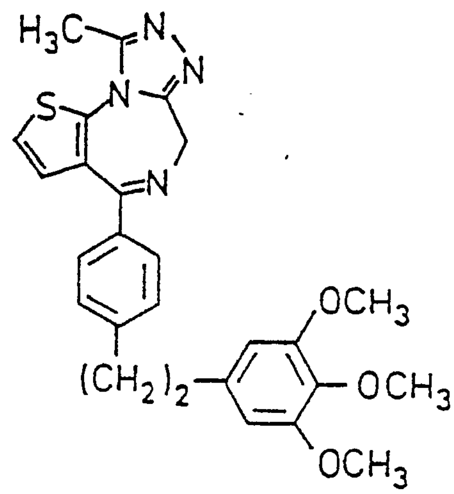

$^1$H-NMR ( $CDCl_3$; 250 MHz): δ = 7.51, 7.19 (4H, 2d, J = 8 Hz, Aryl-H); 7.26, 7.00 (2H, 2d, J = 6 Hz, Thiophen-H); 6.36 (2H, s, Aryl-H); 4.88 (2H, s, breit, $CH_2$-7-Ring); 3.82, 3.81 (9H, 2s, 3-$OCH_3$); 2.90 (4H, m, $CH_2CH_2$); 2.73 (3H, s, $CH_3$-Triazol).

Beispiel 13

6-(4-(3,4,5-Trimethoxyphenethyl)-phenyl)-8,9-dihydro-1-methyl-8-(4-morpholinylcarbonyl]-4H,7H-cyclopenta-[4,5]thieno[3,2-f][1,2,4ltriazolo[4,3-a][1,4]diazepin

Aus 4-(3,4,5-Trimethoxyphenethyl)cyanoacetophenon und Cyclopentanon-3-carbonsäuremethylester erhält man in Gegenwart von Schwefel und Triethylamin das Aminoketon vom Schmp.: 165-166 ° C.

Dieses Aminothiophenderivat wird analog den beschriebenen Synthesewegen weiter umgesetzt und liefert schließlich den Thienotriazolodiazepincarbonsäureester.

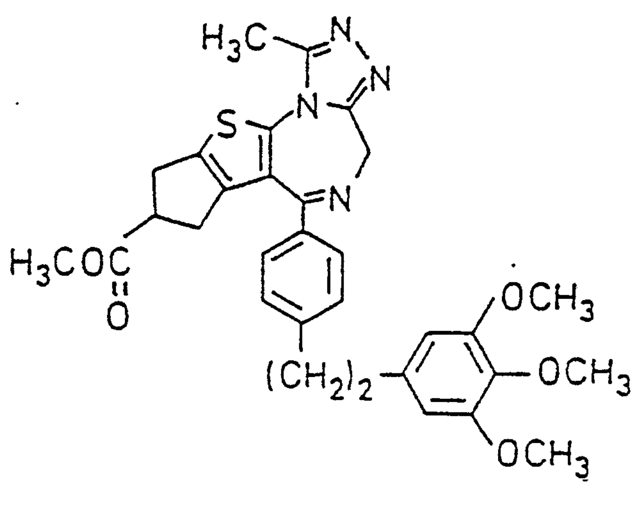

$^1$H-NMR ($CDCl_3$; 250 MHz): δ = 7.41, 7.18 (4H, 2d, J = 8 Hz, Aryl-H); 6.36 (2H, s, Aryl-H); 5.52, 4.15 (2H, AB-System, $J_{AB}$ = 13 Hz, $CH_2$-7-Ring); 3.81, 3.80 (9H, 2s, 3-$OCH_3$); 3.69 (3H, s, $OCH_3$); 3.58 (1H, m, H-3); 3.31 (2H, m, $CH_2$-4); 2.90 (4H, m, $CH_2CH_2$); 2.69 (3H, s, $CH_3$-Triazol); 2.57 (2H, m, $CH_2$-2).

Der Ester wird verseift, woraus die entsprechende Carbonsäure resultiert. Durch weitere Umsetzung dieser Carbonsäure mit Morpholin erhält man die Titelverbindung.

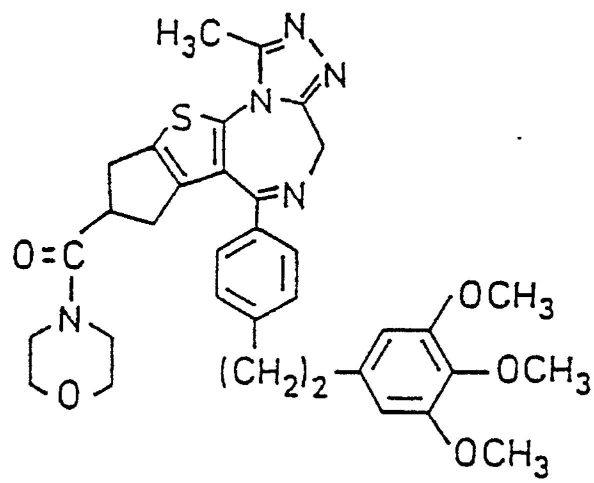

$^1$H-NMR ($CDCl_3$; 250 MHz): δ = 7.41, 7.20 (4H, 2d, J = 8 Hz, Aryl-H); 6.37 (2H, s, Aryl-H); 5.51, 4.13 (2H, AB-System, $J_{AB}$ = 13 Hz, $CH_2$-7-Ring); 3.82, 3.81 (9H, 2s, $3OCH_3$); 3.78-3.31 (8H, m, Morpholin-$CH_2$); 3.24 (2H, m, $CH_2$-4); 3.75 (1H, m, H-3); 2.90 (4H, m, $CH_2CH_2$); 2.69 (3H, s, $CH_3$-Triazol); 2.56 (2H, m, $CH_2$-2).

Beispiel 14

6-(4-(4-Chlorphenoxymethyl)phenyl)-8,9-dihydro-1-methyl-4H,7H-cyclopenta[4,5]thieno[3,2-f][1,2,4]triazolo-[4,3-a][1,4]diazepin

58 g (0.253 mol) p-Brommethylbenzoesäuremethylester werden in 300 ml absolutem Tetrahydrofuran gelöst. Zu dieser Lösung tropft man das aus 32.5 g p-Chlorphenol und 11 g 55 %ige Natriumhydrid-Dispersion in 300 ml Tetrahydrofuran hergestellte Phenolat und kocht 5 Stunden unter Rückfluß. Man filtriert die festen Reaktionsbestandteile ab (Nutsche), dampft das Filtrat ein, verteilt den Rückstand zwischen Wasser und Methylenchlorid und isoliert aus der organischen Phase 60 g Kristalle vom Schmp.: 82-85 °C. Aus dieser Zwischenverbindung kann in Analogie zu den zuvor beschriebenen Verfahren mit Acetonitril und Natriumhydrid das entsprechende Cyanoketon vom Schmp.: 150-152 °C hergestellt werden, welches in das entsprechende Aminobenzoylthiophenderivat überführt werden kann (hellgelbe Kristalle vom Schmp.: 157-158 °C).

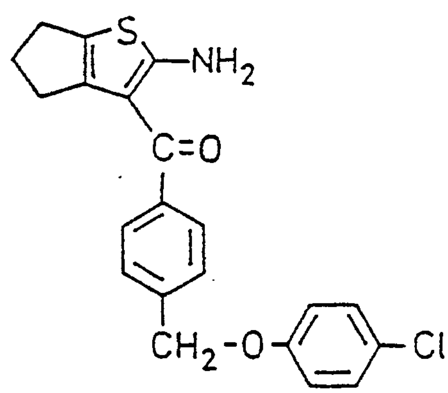

$^1$H-NMR ($CDCl_3$; 250 MHz): δ = 7.46 (4H, m, Aryl-H (Ring A)); 7.27, 6.90 (4H, 2d, J = 9 Hz, Aryl-H (Ring B); 6.95 (2H, s, $NH_2$); 5.09 (2H, s, $OCH_2$); 2.67 (2H, m, $CH_2$-4); 2.09 (4H, m, $CH_2CH_2$-3).

Aus diesem Aminoketon ist das entsprechende Diazepinon vom Schmp.: 210 °C darstellbar, das anhand des folgenden NMR-Spektrums identifiziert werden kann:
$^1$H-NMR ($CDCl_3$; 250 MHz): δ = 7.53, 7.40, 7.22, 6.88 (8H, 4d, J = 9 Hz, Aryl-H); 5.06 (2H, s, $OCH_2$); 4.40 (2H, s, $CH_2$-7-Ring); 2.84 (2H, m, $CH_2$-4); 2.17 (4H, m, $CH_2CH_2$-3).

Aus diesem Diazepinon ist schließlich die Titelverbindung zugänglich.

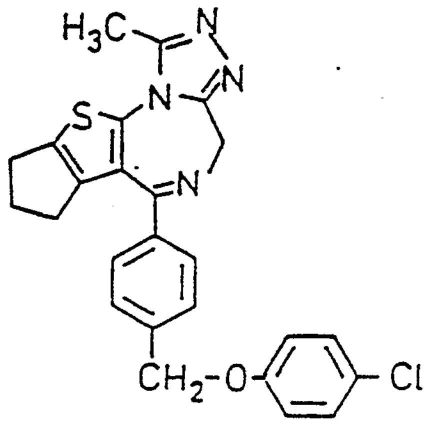

$^1$H-NMR ($CDCl_3$; 250 MHz): δ = 7.51, 7.41, 7.20, 6.88 (8H, 4d, J = 9 Hz, Aryl-H); 5.53, 4.15 (2H, AB-System, $J_{AB}$ = 13 Hz, $CH_2$-7-Ring); 5.07 (2H, s, $OCH_2$); 2.98 (2H, m, $CH_2$-4); 2.69 (2H, s, $CH_3$-Triazol); 2.24 (4H, m, $CH_2CH_2$-3).

Beispiel 15

6-(4-(4-phenyl)phenyl)-8,9-diyhdro-1-methyl-4H,7H-cyclopenta[4,5]thieno[3,2-f][1,2,4]triazolo[4,3-a][1,4]-diazepin

Ausgehend von Diphenylcarbonsäureethylester erhält man das entsprechende Aminoketon vom Schmp.: 188-189 °C.

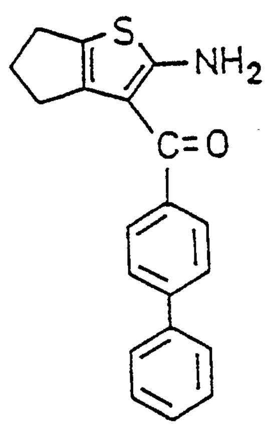

$^1$H-NMR ($CDCl_3$, 250 MHz): δ = 7.17-7.24 (9H, m, Aryl-H); 6.97 (2H, s, $NH_2$); 2.67 (2H, m, $CH_2$-4); 2.15 (4H, m, $CH_2CH_2$-3).

Das hieraus auf üblichem Weg synthetisierte Diazepinon schmilzt bei 285 °C. Aus dem Diazepinon läßt sich die Titelverbindung herstellen, welche in Form von Kristallen vom Schmp.: 253-255 °C isoliert wird.

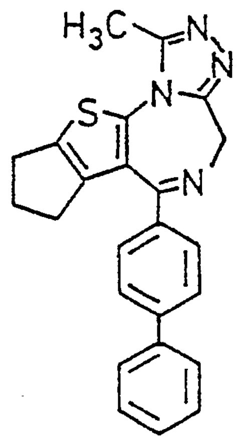

$^1$H-NMR ($CDCl_3$; 250 MHz): δ = 7.69-7.28 (9H, m, Aryl-H); 5.55, 4.18 (2H, AB-System, $J_{AB}$ = 13 Hz, $CH_2$-7-Ring); 2.99 (2H, m, $CH_2$-4); 2.71 (3H, s, $CH_3$-Triazol); 2.29 (4H, m, $CH_2CH_2$-3).

Beispiel 16

6-(4-Phenbutyl)phenyl-8,9-dihydro-1-methyl-4H,7H-cyclopenta[4,5]thieno[3,2-f][1,2,4]triazolo[4,3-a][1,4]-diazepin

Aus dem in Beispiel 1 beschriebenen Phosphoniumsalz und 3-Phenylpropionaldehyd erhält man das entsprechende Olefin und hieraus durch Hydrierung das entsprechende Dihydro-Derivat, welches auf üblichem Wege in das Cyanoketon überführt wird aus dem man das entsprechende Aminoketon vom Schmp.: 135-138 °C darstellt.

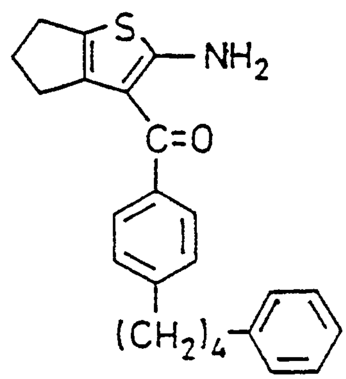

$^1$H-NMR ($CDCl_3$; 250 MHz): δ = 7.44-7.06 (9H, m, Aryl-H); 6.84 (2H,s, $NH_2$); 2.67 (6H, m, $CH_2$-4, 2$C_6H_5$-$CH_2$); 2.12, 1.67 (8H, m, $C_6H_5$-$CH_2CH_2CH_2$ und $CH_2CH_2$-3).

Dieses Aminoketon läßt sich analog der vorgenannten Beschreibung in die Titelverbindung überführen:

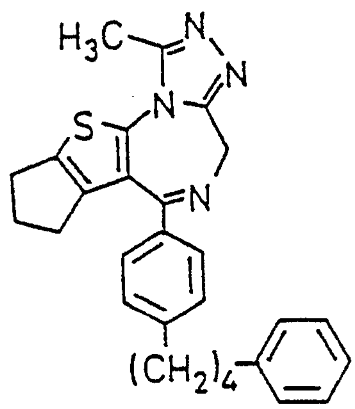

$^1$H-NMR ($CDCl_3$; 250 MHz): δ = 7.46-7.02 (9H, m, Aryl-H); 5.50, 4.12 (2H, AB-System, $J_{AB}$ = 13 Hz, $CH_2$-7-Ring); 2.72 (6H, m, $CH_2$-4, $C_6H_5$-$\underline{CH}_2$); 2.70 (3H, s, $CH_3$-Triazol); 2.32 (8H, m, $C_6H_5$-$CH_2$-$\underline{CH}_2$-$\underline{CH}_2$ und $CH_2CH_2$-3).

Beispiel 17

6-(4-(3,4,5-Trimethoxyphenethyl)-phenyl)-8,9-dihydro-1-hydroxymethyl-4H,7H-cyclopenta[4,5]thieno[3,2-f]-[1,2,4]triazolo[4,3-a][1,4]diazepin

Aus dem in Beispiel 4 charakterisierten Aminoketon vom Schmp.: 180-182° C wird das entsprechende Diazepinon hergestellt (Kristalle - aus Isopropanol - vom Schmp.: 198-199° C).

5 g (10 mmol) dieser Verbindung werden mit $P_2S_5$ in Diglyme umgesetzt und liefern 5.1 g des entsprechenden Thions vom Schmp.: 178-180° C.

5,3 g (10 mmol) des so erhaltenen Thions werden in 60 ml Dioxan suspendiert und mit 1 g Glykolsäurehydrazid versetzt. Man rührt 3 Stunden bei Raumtemperatur nach, entfernt das Lösungsmittel i. Vak., nimmt den Rückstand in Methylenchlorid auf, wäscht die organische Phase mit Wasser, trocknet und erhält nach dem Entfernen des Lösungsmittels Kristalle vom Schmp.: 168-171° C.

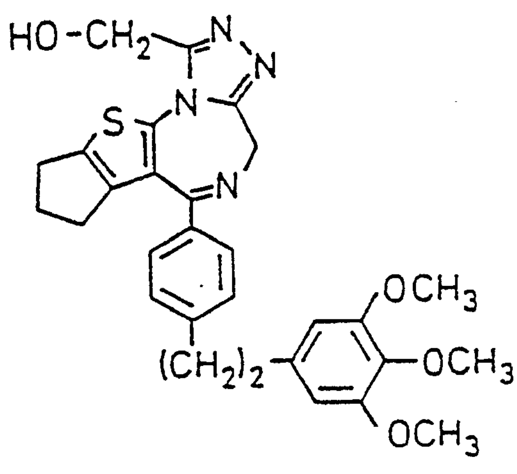

$^1$H-NMR ($CDCl_3$; 250 MHz): δ = 7.39, 7.15 (4H, 2d, J = 9 Hz, Aryl-H); 6.36 (2H, s, Aryl-H); 5.43, 4.13 (2H, AB-System, $J_{AB}$ = 13 Hz, $CH_2$-7-Ring); 5.37 (1H, s, breit, OH); 4.84 (2H, AB-System, $J_{AB}$ = 15 Hz, $CH_2$-O); 3.80, 3.81 (9H, 2s, 3 $OCH_3$); 2.95 (2H, m, $CH_2$-4); 2.88 (4H, m, $CH_2CH_2$); 2.20 (4H, m, $CH_2CH_2$-3).

Beispiel 18

6-(4-(3,4,5-Trimethoxyphenethyl)-phenyl)-8,9-dihydro-1-trifluormethyl-4H,7H-cyclopenta[4,5]thieno[3,2-f]-[1,2,4]triazolo[4,3-a][1,4]diazepin

Das in Beispiel 17 beschriebene Thion vom Schmp.: 178-180 °C ergibt nach der Umsetzung mit Hydrazinhydrat in Tetrahydrofuran die entsprechende Hydrazinoverbindung, welche einen Schmp. von 180-182 °C aufweist.

4.9 g der Hydrazinoverbindung werden mit 2.3 g Trifluoracetanhydrid und 1.5 ml Triethylamin in 80 ml Methylenchlorid 2 Stunden lang bei Raumtemperatur gerührt. Die rote Lösung wird mit Wasser gewaschen und eingedampft. Der Rückstand wird mit 150 ml Toluol und 35 g $SiO_2$ versetzt und 3 Stunden unter Rückfluß gekocht, wobei das anfallende Wasser in einer speziellen Vorlage aufgefangen wird. Das Toluol wird anschließend abdekandiert und die rohe Titelverbindung mit Methanol extrahiert. Nach chromatographischer Reinigung erhält man die gewünschte Trifluormethylverbindung, die anhand des folgenden $^1$H-NMR-Spektrums identifiziert werden kann:

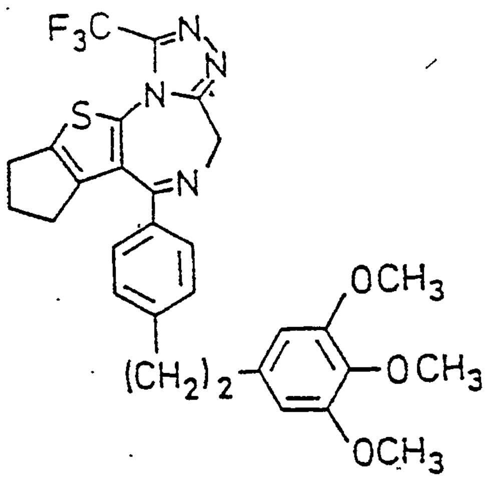

$^1$H-NMR ($CDCl_3$; 250 MHz): $\delta$ = 7.40, 7.16 (4H, 2d, J = 9 Hz, Aryl-H); 6.36 (2H, s, Aryl-H); 5.52, 4.14 (2H, AB-System, $J_{AB}$ = 13 Hz, $CH_2$-7-Ring); 3.82, 3.81 (9H, 2s, 3 $OCH_3$); 2.98 (2H, m, $CH_2$-4); 2.90 (4H, m, $CH_2CH_2$); 2.43-2.09 (4H, m, $CH_2CH_2$-3).

Beispiel 19

cis-6-[4-(2-(4-Trifluormethylphenyl)vinyl)phenyl]-8,9-dihydro-1-methyl-4H,7H-cyclopenta[4,5]thieno[3,2-f]-[1,2,4]triazolo[4,3-a][1,4]diazepin

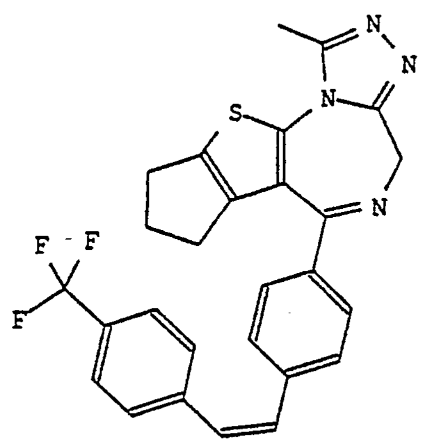

Das auf dem in Beispiel 1 beschriebenen Reaktionsweg erhaltene Stilben-Derivat 5 (s. Reaktionsschema) wird durch Kristallisation aus Diisopropylether in die beiden cis/trans-Isomeren getrennt. Man erhält das entsprechende trans-Stilben-Derivat vom Typ 5 in Form farbloser Kristalle vom Schmelzpunkt 155-157 °C (Fraktion A). Das entsprechende cis-Olefin wird durch Eindampfen der Mutterlauge erhalten (Fraktion B) und analog zu dem in Beispiel 1 beschriebenen Weg umgesetzt. Im folgenden werden daher lediglich die Ansatzgrößen und die dazugehörigen Ausbeuten wiedergegeben:

a) Herstellung des Cyanoketons (entsprechend Schritt 6 nach 7 im Reaktionsschema)

30,5 g (0,1 mol) des cis-Olefins (Franktion B) werden in 100 ml Toluol gelöst und mit 4,5 g Natriumhydrid-Dispersion (55 %-ig) und 12,8 ml Acetonitril versetzt. Nach der Reaktionsdurchführung analog Beispiel 1 erhält man 17,0 g (55 % d. Th.) des entsprechenden Cyanoketons vom Schmp.: 95-100 °C.

b) Herstellung des Aminoketons (entsprechend Schritt 7 nach 8 im Reaktionsschema)

17,0 g (50 mmol) des nach Verfahrensschritt a) hergestellten ungesättigten Cyanoketons vom Typ 7 werden mit 4,2 g Cyclopentanon und 2,0 g Schwefel in Dimethylformamid vermischt sowie mit 4,2 ml Triethylamin in 54 ml Ethanol versetzt und 7 Stunden bei 60 °C gerührt. Das Reaktionsgemisch wird anschließend - in Abwandlung von Beispiel 1 - mit 1,2-Dichlorethan verdünnt und zweimal mit Wasser gewaschen. Die organische Phase wird i. Vak. eingeengt und an einer mit $SiO_2$ gefüllten Säule mit Dichlormethan als Elutionsmittel chromatographiert. Man erhält 13,9 g (62 % d. Th.) des ungesättigten Aminoketons (vom Typ 8) als gelbe Kristalle vom Schmp.: 130-132 °C

c) Herstellung des Bromacetylamids (entsprechend Schritt 8 nach 9)

13,0 g (30 mmol) des nach Verfahrensschritt b) hergestellten Aminoketons werden in 150 ml Dichlormethan gelöst und mit 2,6 ml Pyridin versetzt. Danach werden 2,9 ml Bromacetylbromid zugetropft. Nach der weiteren Reaktionsdurchführung - analog Beispiel 1 - erhält man 15,0 g (89 % d. Th.) des entsprechenden Stilben-Derivats (vom Typ 9) in Form gelber Kristalle (aus Petrolether) vom Schmp.: 95-100 °C.

d) Herstellung des Amino-Derivats (entsprechend Schritt 9 nach 10)

15,0 g (28 mmol) der nach Verfahrensschritt c) hergestellten Bromacetylverbindung werden in 300 ml Essigsäureethylester gelöst und analog Beispiel 1 mit gasförmigem Ammoniak behandelt. Nach der weiteren Reaktionsdurchführung gemäß Beispiel 1 erhält man 13,5 g der entsprechenden Aminoverbindung vom Typ 10 als Rohprodukt, welche im anschließenden Reaktionsschritt sofort cyclisiert wird.

e) Herstellung des Diazepinons (entsprechend Schritt 10 nach 11 im Reaktionsschema)

13,5 g der nach Verfahrensschritt d) hergestellten Aminoverbindung werden in 200 ml Toluol in Gegenwart von 75 g Kieselgel cyclisiert. Die Reaktionsführung gemäß Beispiel 1 führt zu 12,0 g (94 % d. Th.) des entsprechenden Diazepinons vom Typ 11.

f) Herstellung des ungesättigten Diazepinthion-Derivats (entsprechend Schritt 11 nach 12 im Reaktionsschema)

6,0 g (13,2 mmol) des nach Verfahrensschritt e) hergestellten Diazepinon-Derivates werden in 52 ml Pyridin gelöst und mit 4,4 g Phosphorpentasulfid versetzt. Die Reaktionsmischung wird über einen Zeitraum von zwei Stunden auf 60 °C erhitzt. Anschließend wird das Gemisch mit Wasser verdünnt und mit Dichlormethan extrahiert. Nach dem Entfernen des Extraktionsmittels i. Vak. erhält man 3,5 g (56 % d. Th.) des entsprechenden Diazepinthion-Derivats vom Schmp.: 178-180 °C.

g) Herstellung der Hydrazono-Verbindung vom Typ 13 (entsprechend Schritt 12 nach 13 im Reaktionsschema)

3,5 g (7,4 mmol) des nach Verfahrensschritt f) hergestellten Diazepinthions werden in 30 ml Tetrahydrofuran vorgelegt und mit 0,4 ml Hydrazin-Hydrat versetzt. Nach Durchführung der Umsetzung gemäß Beispiel 1 erhält man 2,5 g (72 % d. Th.) des entsprechenden Hydrazons vom Typ 13 als Kristalle vom Schmp.: 170 °C (Zersetzung).

h) Herstellung der Titelverbindung (entsprechend Schritt 13 nach 14 im Reaktionsschema)

2,5 g (5,3 mmol) des nach Verfahrensschritt g) hergestellten Hydrazons werden in 30 ml Ethanol suspendiert und mit 2 ml Orthoessigsäuretriethylester zur Titelverbindung umgesetzt. Man erhält nach der Aufarbeitung gemäß Beispiel 1 1,5 g (57 % d. Th.) der Titelverbindung in Form von Kristallen vom Schmp.: 192-195 °C.

Beispiel 20

trans-6-[4-(2-(4-Trifluormethylphenyl)-vinyl)phenyl]-8,9-dihydro-1-methyl-4H,7H-cyclopenta[4,5]thieno[3,2-f]-[1,2,4]triazolo[4,3-a][1,4]diazepin

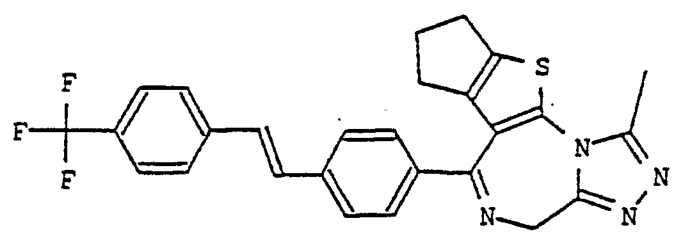

Die aus der Isomerentrennung gewonnene Fraktion A des 1-Methoxycarbonyl-4-[2-(4-trifluormethylphenyl)vinyl]benzols (5) aus Beispiel 19 kommt die trans-Konfiguration bezüglich der Substitution des Olefins zu. Die folgenden Umsetzungen mit dem Stilben-Derivat, 5 werden in Analogie zu dem in Beispiel 19 beschriebenen Verfahren durchgeführt.

a) Herstellung des Cyanoketons (entsprechend Schritt 6 nach 7 im Reaktionsschema)

22,5 g (73 mmol) des trans-Olefins (Fraktion A) werden in 75 ml Toluol gelöst und mit 3,3 g Natriumhydrid-Dispersion (55 %-ig) und 9,5 ml Acetonitril versetzt. Nach der Reaktionsdurchführung analog Beispiel 19 erhält man 19,0 g (83 % d. Th.) des entsprechenden Cyanoketons vom Schmp.: 146 °C.

b) Herstellung des Aminoketons (entsprechend Schritt 7 nach 8 im Reaktionsschema)

12,4 g (39 mmol) des nach Verfahrensschritt a) hergestellten Cyanoketons vom Typ 7 werden mit 3,0 g Cyclopentanon und 1,4 g Schwefel in Dimethylformamid vermischt sowie mit 3 ml Triethylamin in 39 ml Ethanol versetzt und 7 Stunden bei 60 °C gerührt. Das Reaktionsgemisch wird anschließend - in Abwandlung von Beispiel 1 - mit 1,2-Dichlorethan verdünnt und zweimal mit Wasser gewaschen. Die organische Phase wird i. Vak. eingeengt und an einer mit $SiO_2$ gefüllten Säule mit Dichlormethan als Elutionsmittel chromatographiert. Man erhält 9,5 g (58 % d. Th.) des ungesättigten Aminoketons (vom Typ 8) als gelbe Kristalle vom Schmp.: 197-199 °C.

c) Herstellung des Bromacetylamids (entsprechend Schritt 8 nach 9)

9,0 G (22 mmol) des nach Verfahrensschritt b) hergestellten Aminoketons werden in 100 ml Dichlormethan gelöst und mit 1,8 ml Pyridin versetzt. Danach werden 2,0 ml Bromacetylbromid zugetropft. Nach der weiteren Reaktionsdurchführung - analog Beispiel 19 - erhält man 11,0 g (95 % d. Th.) des entsprechenden Stilben-Derivats (vom Typ 9) in Form gelber Kristalle vom Schmp.: 200 °C.

d) Herstellung des Amino-Derivats (entsprechend Schritt 9 nach 10)

11,0 g (20 mmol) der nach Verfahrensschritt c) hergestellten Bromacetylverbindung werden in 200 ml Essigsäureethylester gelöst und analog Beispiel 1 mit gasförmigem Ammoniak behandelt. Nach der weiteren Reaktionsdurchführung gemäß Beispiel 1 erhält man 9,0 g (93 % d. Th.) der entsprechenden ungesättigten Aminoverbindung vom Typ 10 als Kristalle vom Schmp.: 187-190 °C.

e) Herstellung des Diazepinons (entsprechend Schritt 10 nach 11 im Reaktionsschema)

9,0 g der nach Verfahrensschritt d) hergestellten Aminoverbindung werden in 300 ml Toluol in Gegenwart von 50 g Kieselgel cyclisiert. Die Reaktionsführung gemäß Beispiel 1 führt zu 8,0 g (92 % d. Th.) des entsprechenden Diazepinons vom Typ 11.

f) Herstellung des Diazepinthion-Derivates (entsprechend Schritt 11 nach 12 im Reaktionsschema)

6,0 g (13,2 mmol) des nach Verfahrensschritt e) hergestellten Diazepinon-Derivates werden in 52 ml Pyridin gelöst und mit 4,4 g Phosphorpentasulfid versetzt. Die Reaktionsmischung wird über einen Zeitraum von zwei Stunden auf 60 °C erhitzt. Anschließend wird das Gemisch mit Wasser verdünnt und mit Dichlormethan extrahiert. Nach dem Entfernen des Extraktionsmittels i. Vak. erhält man 1,6 g (26 % d. Th.) des entsprechenden Diazepinthion-Derivates vom Schmp.: 265 °C.

g) Herstellung der Hydrazono-Verbindung vom Typ 13 (entsprechend Schritt 12 nach 13 im Reaktionsschema)

1,6 g (3,4 mmol) des nach Verfahrensschritt f) hergestellten Diazepinthions werden in 15 ml Tetrahydrofuran vorgelegt und mit 0,2 ml Hydrazin-Hydrat versetzt. Nach Durchführung der Umsetzung gemäß Beispiel 1 erhält man 1,3 g (82 % d. Th.) des entsprechenden Hydrazons vom Typ 13 als Kristalle vom Schmp.: 255 °C.

h) Herstellung der Titelverbindung (entsprechend Schritt 13 nach 14 im Reaktionsschema)

1,3 g (2,8 mmol) des nach Verfahrensschritt g) hergestellten Hydrazons werden in 10 ml Ethanol suspendiert und mit 1 ml Orthoessigsäuretriethylester in die Titelverbindung überführt. Nach der Aufarbeitung gemäß Beispiel 1 erhält man 1,1 g (80 % d. Th.) in Form von Kristallen vom Schmp.: 299-301 °C.

Beispiel 21

Die Synthese des Stilben-Derivats 5 erfolgt analog Beispiel 1 und die Auftrennung in die cis/trans-Isomeren analog Beispiel 19. Für die weitere Synthese im vorliegenden Beispiel wird - wie in Beispiel 20 - das entsprechende trans-Stilben-Derivat 5 eingesetzt.

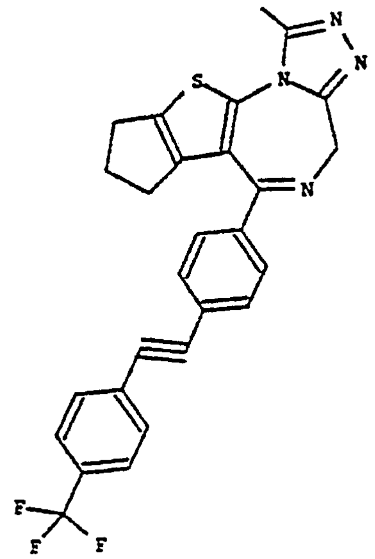

a) Herstellung des Dibromethanderivates vom Typ 6.

22,0 g (72 mmol) des trans-Stilben-Derivats 5 werden in 200 ml Chloroform vorgelegt. Danach werden 2,55 ml Brom zugetropft. Die Reaktionsmischung wird 2 Stunden bei Raumtemperatur nachgerührt und danach mit Diisopropylether versetzt. Das dabei kristallin anfallende Bromierungsprodukt wird abgesaugt und getrocknet. Man isoliert 20,2 g (87 % d. Th.) der entsprechenden Dibromverbindung vom Typ 6 vom Schmp.: 205 °C.

b) Herstellung des 1-Methoxycarbonyl-4-[2-(4-trifluormethylphenyl)ethinyl]benzols

20,2 g (43 mmol) der nach Verfahrensschritt a) hergestellten Dibromverbindung werden in 170 ml n-Butanol gelöst und mit 52 g Kaliumhydroxid und 5 ml Wasser versetzt und über einen Zeitraum von 3 Stunden unter Rühren auf Rückflußtemperatur erhitzt. Anschließend wird das Reaktionsgemisch mit 300 ml Wasser verdünnt. Danach wird die organische Phase abgetrennt und i. Vak. eingeengt. Der verbleibende Rückstand wird in Wasser aufgenommen, die Lösung wird mit Salzsäure angesäuert, wobei sich das Reaktionsprodukt in kristalliner Form abscheidet. Man isoliert 12 g (96 % d. Th.) des entsprechenden Alkins in Form farbloser Kristalle vom Schmp.: 278-285 °C.

c) Herstellung des 1-Ethoxycarbonyl-4-[2-(4-trifluorethylphenyl)ethinyl]benzols durch Umesterung

31,0 g (106 mmol) des nach Verfahrensschritt b) hergestellten Methoxycarbonylbenzol-Derivats werden 8 Stunden lang in einer Mischung aus 150 ml Ethanol und 1,2 ml konzentrierter Schwefelsäure unter Rühren auf Rückflußtemperatur erhitzt. Die bei dieser Umsetzung anfallende - in dem Reaktionsgemisch unlösliche -Carbonsäure wird isoliert und erneut zur Reaktion gebracht. Anschließend wird das Lösungsmittel i. Vak. abdestilliert, der verbleibende Rückstand in Dichlormethan aufgenommen, zweimal mit Wasser gewaschen und mit Natriumsulfat getrocknet. Danach wird das Lösungsmittel i. Vak. entfernt. Man isoliert 26,0 g (76 % d. Th.) der Titelverbindung als Kristalle vom Schmp.: 65-68 °C.

d) Herstellung des Cyanoketons (entsprechend Schritt 6 nach 7 im Reaktionsschema)

26,0 (81 mmol) des nach Verfahrensschritt c) hergestellten Esters in 90 ml Toluol gelöst und mit 3,8 g Natriumhydrid-Dispersion (55 %-ig) und 11 ml Acetonitril versetzt. Nach der Reaktionsdurchführung analog Beispiel 1 erhält man 14,0 g (55 % d. Th.) des entsprechenden Cyanoketons vom Schmp.: 110-120 °C.

e) Herstellung des Aminoketons (entsprechend Schritt 7 nach 8 im Reaktionsschema)

14,0 g (45 mmol) des nach Verfahrensschritt d) hergestellten Cyanoketons werden 4,0 g Cyclopentanon und 1,85 g Schwefel in Dimethylformamid vermischt sowie mit 4 ml Triethylamin in 51 ml Ethanol versetzt und über einen Zeitraum von 7 Stunden bei 60° C gerührt. Das Reaktionsgemisch wird in Analogie zu Beispiel 1 umgesetzt. Man erhält 7,3 (40 % d. Th.) des Aminoketons (vom Typ 8) als gelbe Kristalle vom Schmp.: 195-200° C.

f) Herstellung des Bromacetylamids (entsprechend Schritt 8 nach 9)

7,0 g (17 mmol) des nach Verfahrensschritt e) hergestellten Aminoketons werden in 80 ml Dichlormethan gelöst und mit 1,4 ml Pyridin versetzt. Danach werden 1,6 ml Bromacetylbromid zugetropft. Nach der weiteren Reaktionsdurchführung - analog Beispiel 1 - erhält man 8,0 g (88 % d. Th.) des entsprechenden Bromacetylamids in Form gelber Kristalle vom Schmp.: 205-207° C.

g) Herstellung des Amino-Derivats (entsprechend Schritt 9 nach 10)

8,0 g (15 mmol) des nach Verfahrensschritt f) hergestellten Bromacetylamids werden in 150 ml Essigsäureethylester gelöst und analog Beispiel 1 mit gasförmigem Ammoniak behandelt. Nach der weiteren Reaktionsdurchführung gemäß Beispiel 1 erhält man 6,5 g (92 % d. Th.) der entsprechenden ungesättigten Aminoverbindung als Kristalle vom Schmp.: 215-220° C.

h) Herstellung des Diazepinons (entsprechend Schritt 10 nach 11 im Reaktionsschema)

6,5 g (13,8 mmol) der nach Verfahrensschritt g) hergestellten Aminoverbindung werden in 200 ml Toluol in Gegenwart von 35 g Kieselgel cyclisiert. Die Reaktionsdurchführung gemäß Beispiel 1 führt zu 3,0 g (48 % d. Th.) des entsprechenden Diazepinons als Kristalle vom Schmp.: 275° C.

i) Herstellung des Diazepinthion-Derivats (entsprechend Schritt 11 nach 12 im Reaktionsschema)

3,0 g (6,6 mmol) des nach h) hergestellten Diazepinon-Derivats werden in 30 ml Pyridin gelöst und mit 2,2 g Phosphorpentasulfid versetzt. Die Reaktionsmischung wird über einen Zeitraum von zwei Stunden auf 60° C erhitzt. Anschließend wird das Gemisch mit Wasser verdünnt und mit Dichlormethan extrahiert. Nach dem Entfernen des Extraktionsmittels i. Vak. erhält man 1,0 g (32 % d. Th.) des entsprechenden Diazepinthion-Derivats vom Schmp.: 255° C (Zersetzung).

j) Herstellung der Hydrazono-Verbindung (entsprechend Schritt 12 nach 13 im Reaktionsschema)

1,0 g (2,0 mmol) des nach Verfahrensschritt i) hergestellten Diazepinthions werden in 10 ml Tetrahydrofuran vorgelegt und mit 0,1 g Hydryzinhydrat versetzt. Nach Durchführung der Umsetzung gemäß Beispiel 1 erhält man 1,0 g (100 % d. Th.) des entsprechenden Hydrazons vom Typ 13 als Kristalle vom Schmp.: 255° C.

k) Herstellung der Titelverbindung (entsprechend Schritt 13 nach 14 im Reaktionsschema)

1,0 g (2,0 mmol) des nach Verfahrensschritt j) hergestellten Hydrazons werden in 10 ml Ethanol suspendiert und mit 1 ml Orthoessigsäuretriethylester zur Titelverbindung umgesetzt. Man erhält nach der Aufarbeitung gemäß Beispiel 1 0,8 g (76 % d. Th.) des 6-[4-(2-(4-Trifluormethylphenyl)ethinyl)phenyl]-8,9-dihydro-1-methyl-4H,7H-cyclopenta[4,5]thienotriazolo[3,2-f]-[1,2,4]diazepin in Form von Kristallen vom Schmp.: 308-310° C.

Beispiel 22

6-(4-(4-Chlor-N-methylanilino)carbonylphenyl)-8,9-dihydro-1-methyl-4H,7H-cyclopenta[4,5]thieno[3,2-f]-[1,2,4]triazolo[4,3-a][1,4]diazepin

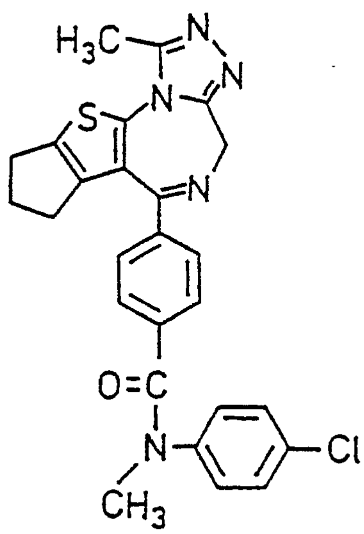

0,9 g (2,5 mmol) der nach Beispiel 8 hergestellten Carbonsäure werden mit 350 mg 4-Chlor-N-methylanilin, 350 mg 1-Hydroxy-1H-benzotriazol-Hydrat und 500 mg N,N'-Dicyclohexylcarbodiimid 2 Tage lang bei Raumtemperatur in 10 ml Dimethylformamid gerührt. Danach wird die resultierende Suspension an Kieselgel adsorptiv filtriert und das Filtrat i. Vak. eingeengt. Der verbliebene Rückstand wird an einer mit Siliciumdioxyd gefüllten Säule mit einer Mischung aus Dichlormethan und 4 % Methanol als Elutionsmittel chromatographiert. Man erhält 0,4 g (33 % d. Th.) der Titelverbindung als Kristalle vom Schmp.: 288 °C.

Beispiel 23

6-(4-(4-Chlorphenylthiomethyl)phenyl)-8,9-dihydro-1-methyl-4H,7H-cyclopenta[4,5]thieno[3,2-f][1,2,4]triazolo-[4,3-a][1,4]diazepin

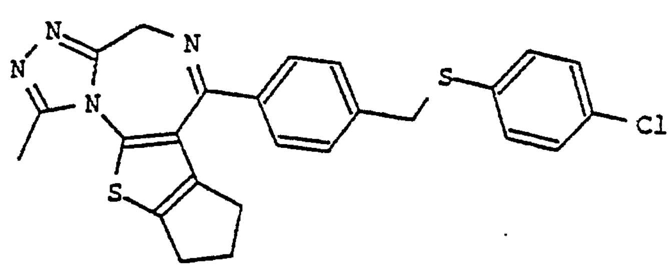

a) 1,75 g (5 mmol) des nach Beispiel 7 hergestellten Alkohols werden in der ersten Reaktionsstufe in 350 ml Dichlormethan gelöst und mit 1,5 ml Thionylchlorid versetzt und 1 Stunde lang bei Raumtemperatur gerührt. Anschließend wird das Reaktionsgemisch mit wässeriger Ammoniaklösung neutralisiert. Nach dem Separieren und Trocknen der organischen Phase, wird das Lösungsmittel im Vakuum entfernt. Man erhält 1,80 g (98 % d. Th.) der entsprechenden Chlorverbindung in Form von Kristallen mit einem Schmp.: >300 °C.

b) 100 mg einer 50 %-igen Natriumhydriddispersion und 310 mg p-Chlorthiophenol werden in 20 ml Tetrahydrofuran vorgelegt und 10 Minuten bei Raumtemperatur gerührt. Nach Zugabe von 800 mg (2,2

mmol) des nach Reaktionsschritt a) hergestellten Chlorids wird die Reaktionsmischung für einen Zeitraum von 5 Stunden unter Rühren auf Rückflußtemperatur erhitzt. Danach wird das Lösungsmittel i. Vak. abdestilliert. Der verbliebene Rückstand wird an Kieselgel mit einer Mischung aus Dichlormethan und 4 % Methanol als Elutionsmittel chromatographiert. Man erhält 450 mg (43 % d. Th.) der Titelverbindung als Kristalle vom Schmp.: 153-155° C.

Beispiel 24

6-(4-(4-Chloranilino)phenyl)-8,9-dihydro-1-methyl-4H,7H-cyclopenta[4,5]thieno[3,2-f][1,2,4]triazolo[4,3-a][1,4]-diazepin

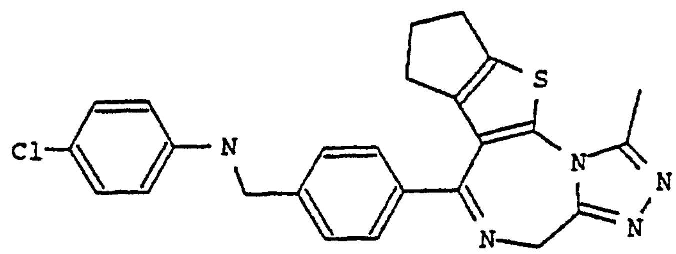

a) Der nach Beispiel 7 hergestellte Alkohol wird gemäß Beispiel 23, Verfahrensschritt a) in das entsprechende Chlorid überführt.
b) 960 mg (2,6 mmol) des Chlorids wird in 20 ml Dioxan (1,4-Dioxacyclohexan) gelöst, mit 650 mg p-Chloranilin versetzt und unter Rühren eine Stunde lang auf Rückflußtemperatur erhitzt.
Nach dem Zufügen von 10 ml Dimethylformamid wird das Reaktionsgemisch weitere 4 Stunden auf Rückflußtemperatur erhitzt. Anschließend wird das Lösungsmittel i. Vak. entfernt. Der Rückstand wird zwischen Essigsäureethylester und Wasser verteilt. Die organische Phase wird extrahiert und nach dem Trocknen mit Magnesiumsulfat und Abfiltrieren des Trockenmittels i. Vak. eingeengt. Der verbliebene Rückstand wird an einer mit Siliciumdioxid gefüllten Säule mit einer Mischung aus Dichlormethan und 4 % Methanol adsorptiv filtriert. Man erhält 300 mg (25 % d. Th.) der Titelverbindung in Form von Kristallen vom Schmp.: 195-200° C.

Beispiel 25

6-(4-(4-Chlorphenylmethylenoxy)phenyl)-8,9-dihydro-1-methyl-4H,7H-cyclopenta[4,5]thieno[3,2-f][1,2,4]-triazolo[4,3a][1,4]diazepin

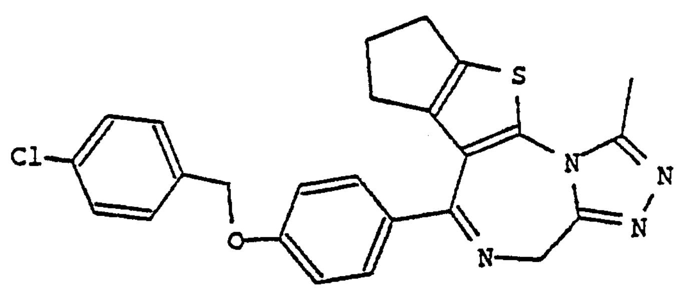

a) Herstellung des p-Chlorbenzoesäureethylesters

83 g (0,6 mol) 4-Hydroxybenzoesäure werden in 400 ml Ethanol auf Rückflußtemperatur erhitzt. In der Siedehitze wird über einen Zeitraum von 2,5 Stunden Chlorwasserstoff in die Reaktionslösung eingeleitet.

Nach dem Abkühlen der Reaktionsmischung wird der Alkohol i. Vak. entfernt und der Rückstand

anschließend in Diethylether gelöst. Die Lösung wird zweimal mit Wasser gewaschen und mit Magnesiumsulfat getrocknet. Nach dem Entfernen des Trockenmittels und Abziehen des Lösungsmittels i. Vak. erhält man 100 g (100 % d. Th.) des p-Chlorbenzoesäureethylesters in Form von Kristallen vom Schmp.: 103-105 ° C.

b) Herstellung des 4-[(p-Chlorphenyl)methylenoxy]benzoesäureethylester

52,1 g (0,3 mol) des nach Reaktionsschritt a) hergestellten Esters werden in 300 ml Dimethylformamid vorgelegt und unter Eiskühlung mit 13,8 g einer 55 %-igen Natriumhydrid-Dispersion versetzt. Man läßt 10 Minuten nachrühren und fügt dann eine Lösung von 50,2 g p-Chlorbenzylchlorid in 120 ml Dimethylformamid der Reaktionsmischung zu und erhitzt das Reaktionsgemisch unter Rühren über einen Zeitraum von 4 Stunden auf 150 ° C. Anschließend läßt man die Reaktionsmischung auf Raumtemperatur abkühlen, verdünnt mit 600 ml Essigsäureethylester. Die organische Phase wird abgetrennt und zweimal mit Wasser gewaschen, getrocknet und nach dem Abfiltrieren des Trockenmittels i. Vak. eingeengt. Der verbliebene Rückstand wird mit Dichlormethan an Kieselgel chromatographiert. Man erhält 62 g (68 % d. Th.) des 4-[(p-Chlorphenyl)methylenoxy]benzoesäureethylesters in Form farbloser Kristalle vom Schmp.: 65-68 ° C.

c) Herstellung des Cyanoketons (entsprechend Schritt 6 nach 7 im Reaktionsschema)

62 g (213 mmol) des nach Verfahrensschritt b) hergestellten Esters werden in 200 ml Toluol gelöst und mit 9,7 g Natriumhydrid-Dispersion (55 %-ig) sowie 13,9 ml Acetonitril versetzt. Nach der Reaktionsdurchführung analog Beispiel 1 erhält man 30,0 g (49 % d. Th.) des entsprechenden Cyanoketons vom Schmp.: 129-136 ° C.

d) Herstellung des Aminoketons (entsprechend Schritt 7 nach 8 im Reaktionsschema)

30,0 g (105 mmol) des nach Verfahrensschritt c) hergestellten Cyanoketons werden mit 8,0 g Cyclopentanon und 3,8 Schwefel in Dimethylformamid vermischt sowie mit 8,0 ml Triethylamin in 105 ml Ethanol versetzt und anschließend - gemäß Beispiel 1 - 7 Stunden lang bei 60 ° C gerührt, wonach das Reaktionsgemisch mit 500 ml Dichlormethan verdünnt wird. Man erhält nach dem Umkristallisieren aus einer Mischung aus Diethylether und Diisopropylether 16,0 g (40 % d. Th.) des Aminoketons als gelbe Kristalle vom Schmp.: 147-149 ° C.

e) Herstellung des Bromacetylamids (entsprechend Schritt 8 nach 9)

12,3 g (32 mmol) des nach Verfahrensschritt d) hergestellten Aminoketons werden in 100 ml Dichlormethan gelöst und mit 2,5 ml Pyridin versetzt. Danach werden 2,7 ml Bromacetylbromid zugetropft. Nach der weiteren Reaktionsdurchführung und Aufarbeitung - analog Beispiel 1 - erhält man 13,0 g der entsprechenden Bromacetylverbindung in Form gelber Kristalle vom Schmp.: 183-185 ° C.

f) Herstellung des Diazepinon-Derivates

9,0 g (18 mmol) der nach Verfahrensschritt e) hergestellten Bromacetylverbindung werden in einer Mischung aus 100 ml Dichlormethan und 100 ml Methanol gelöst und - analog Beispiel 1 - 1 Stunde lang mit gasförmigem Ammoniak behandelt. Man läßt das Reaktionsgemisch 5 Tage bei Raumtemperatur unter Rühren ausreagieren. Danach wird die Reaktionsmischung i. Vak. eingeengt. Nach mehrmaliger Chromatographie des verbliebenen Rückstandes mit einer Mischung aus Dichlormethan und 2 % Methanol an Kieselgel erhält man 300 mg (4 % d. Th.) des entsprechenden Diazepinons.

g) Herstellung des Diazepinthions (entsprechend Schritt 11 nach 12 im Reaktionsschema)

300 mg (0,7 mmol) des nach Verfahrensschritt f) hergestellten Diazepinons werden in 7 ml Diethylenglykoldimethylether gelöst und mit 130 mg Natriumhydrogencarbonat und 180 mg Phosphorpentasulfid analog Beispiel 1 zum entsprechenden Diazepinthion-Derivat umgesetzt. Man erhält 0,3 g (98% d. Th.) des Reaktionsproduktes in kristalliner Form.

h) Herstellung der Hydrazono-Verbindung (entsprechend Schritt 12 nach 13 im Reaktionsschema)

300 mg (0,7 mmol) des nach Verfahrensschritt g) hergestellten Diazepinthions werden in 10 ml Tetrahydrofuran mit 0,1 ml Hydrazin-Hydrat versetzt. Nach der Durchführung der Reaktion in Analogie zu Beispiel 1 erhält man 250 mg (84 % d. Th.) des entsprechenden Hydrazons in Form von Kristallen vom Schmp.: 172° C.

i) Herstellung der Titelverbindung (entsprechend Schritt 13 nach 14 im Reaktionsschema)

250 mg (0,57 mmol) des nach Verfahrensschritt h) hergestellten Hydrazons werden in 10 ml Ethanol suspendiert und mit 0,5 ml Orthoessigsäuretriethylester zur Titelverbindung umgesetzt. Nach der Aufarbeitung gemäß Beispiel 1 erhält man 100 mg (40 % d. Th.) der Titelverbindung als Kristalle vom Schmp.: 184-186° C.

Beispiel 26

6-(4-(4-Methylphenethyl)phenyl)-8,9-dihydro-1-methyl-4H,7H-cyclopenta[4,5]-thieno[3,2-f][1,2,4]triazolo[4,3-a][1,4]diazepin

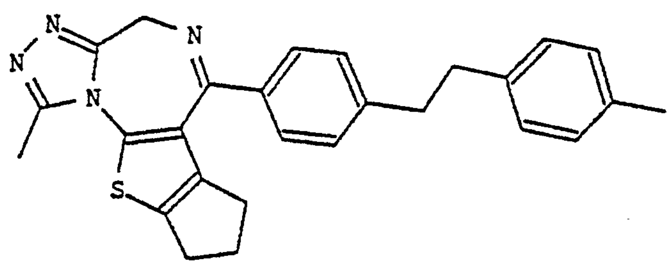

Die Synthesedurchführung zur Herstellung der Titelverbindung erfolgt in Analogie zu den in Beispiel 1 bzw. Beispiel 19 beschriebenen Verfahren mit der Ausnahme, daß bei der Wittig-Olefinierung (im Reaktionsschema Schritt 4 nach 5) anstelle von p-Trifluormethylbenzaldehyd p-Tolylaldehyd eingesetzt wird.

a) Wittig-Olefinierung (entsprechend Schritt 4 nach 5 im Reaktionsschema)

12,5 g einer Natriumhydrid-Dispersion (55 %-ig) werden unter Inertgas in 150 ml absolutem Dimethylsulfoxid vorgelegt und gemäß Beispiel 1 mit 140,8 g (0,29 mol) des entsprechenden Phosphoniumsalzes - suspendiert in 290 ml Dimethylsulfoxid - und darauf mit 34,8 g (0,29 mol) p-Tolyladehyd versetzt. Die Aufarbeitung gemäß Beispiel 1 liefert 62 g (85 % d. Th.) des entsprechenden Olefins.

b) Reduktion des Olefins (entsprechend Schritt 5 nach 6 im Reaktionsschema)

Analog Beispiel 1 werden 62 g (0,24 mol) des nach Verfahrensschritt a) hergestellten Olefins in 1 l Tetrahydrofuran in Gegenwart von Raney-Nickel als Katalysator bei 20° C und 5 bar Wasserstoffdruck über einen Zeitraum von 4 Stunden hydriert. Man erhält 48,5 g (78 % d. Th.) des 1,2-Diphenylethanderivats als Kristalle vom Schmp.: 75 ° C.

c) Herstellung des Cyanoketons (entsprechend Schritt 6 nach 7 im Reaktionsschema)

48,5 g (0,19 mol) des nach Verfahrensschritt b) hergestellten Diphenylethanderivats werden in 180 ml Toluol gelöst und mit 8,7 g Natriumhydrid-Dispersion (55 %-ig) und 25 ml Acetonitril versetzt. Nach der Reaktionsdurchführung analog Beispiel 1 erhält man 48,0 g (96 % d. Th.) des entsprechenden Cyanoketons als Kristalle vom Schmp.: 85-90 °C.

d) Herstellung des Aminoketons (entsprechend Schritt 7 nach 8 im Reaktionsschema)

48,0 g (182 mmol) des nach Verfahrensschritt c) hergestellten Cyanoketons vom Typ 7 werden mit 14,0 g Cyclopentanon und 6,6 g Schwefel in Dimethylformamid vermischt sowie mit 14 ml Triethylamin in 175 ml Ethanol versetzt und über einen Zeitraum von 7 Stunden bei 60 °C gerührt. Das Reaktionsgemisch wird anschließend - in Analogie zu Beispiel 19 - mit 1,2-Dichlorethan verdünnt und zweimal mit Wasser gewaschen. Die organische Phase wird i. Vak. eingeengt und an einer mit Siliciumdioxyd gefüllten Säule mit Dichlormethan als Elutionsmittel chromatographiert. Man erhält nach dem Umkristallisieren aus Diisopropylether 17,0 g (26 % d. Th.) des Aminoketons (vom Typ 8) als gelbe Kristalle vom Schmp.: 135-140 °C

e) Herstellung des Bromacetylamids (entsprechend Schritt 8 nach 9)

18,0 g (50 mmol) des nach d) hergestellten Aminoketons werden in 200 ml Dichlormethan gelöst und mit 4,0 ml Pyridin versetzt. Danach werden 4,4 ml Bromacetylbromid zugetropft. Nach der weiteren Reaktionsdurchführung - analog Beispiel 1 - und Umkristallisieren aus Diisopropylether erhält man 17,9 g (75 % d. Th.) des entsprechenden Bromacetylamids in Form gelber Kristalle vom Schmp.: 142-145 °C.

f) Herstellung des Amino-Derivats (entsprechend Schritt 9 nach 10)

17,9 g (37 mmol) der nach Verfahrensschritt e) hergestellten Bromacetylverbindung werden in 300 ml Essigsäureethylester gelöst und analog Beispiel 1 mit gasförmigem Ammoniak behandelt. Nach der weiteren Reaktionsdurchführung gemäß Beispiel 1 erhält man 12,0 g (77 % d. Th.) der entsprechenden Aminoverbindung vom Typ 10 in Form von Kristallen vom Schmp.: 160-162 °C.

g) Herstellung des Diazepinon-Derivats (entsprechend Schritt 10 nach 11 im Reaktionsschema)

12,0 g (28,6 mmol) der nach Verfahrensschritt f) hergestellten Aminoverbindung werden in 250 ml Toluol in Gegenwart von 60 g Kieselgel cyclisiert. Die weitere Reaktionsführung und Aufarbeitung gemäß Beispiel 1 führt zu 6,5 g (57 % d. Th.) des entsprechenden Diazepinons vom Typ 11 in Form von Kristallen vom Schmp.: 232 °C.

h) Herstellung des Diazepinthion-Derivats (entsprechend Schritt 11 nach 21 im Reaktionsschema)

6,5 g (16 mmol) des nach Verfahrensschritt g) hergestellten Diazepinon-Derivats werden in 65 ml Pyridin gelöst und mit 5,5 g Phosphorpentasulfid versetzt. Die Reaktionsmischung wird über einen Zeitraum von zwei Stunden auf 60 °C erhitzt. Anschließend wird das Gemisch mit Wasser verdünnt und mit Dichlormethan extrahiert. Nach dem Entfernen des Extraktionsmittels i. Vak. erhält man 4,0 g (59 % d. Th.) des entsprechenden Diazepinthion-Derivates vom Schmp.: 212-215 °C.

i) Herstellung der Hydrazono-Verbindung vom Typ 13 (entsprechend Schritt 12 nach 13 im Reaktionsschema)

4,0 g (9,6 mmol) des nach h) hergestellten Diazepinthions werden in 40 ml Tetrahydrofuran vorgelegt und mit 0,5 ml Hydrazin-Hydrat versetzt. Nach Durchführung der Umsetzung gemäß Beispiel 1 erhält man 2,6 g (65 % d. Th.) des entsprechenden Hydrazons vom Typ 13 als Kristalle vom Schmp.: 218 °C.

j) Herstellung der Titelverbindung (entsprechend Schritt 13 nach 14 im Reaktionsschema)

2,6 g (6,3 mmol) des nach Verfahrensschritt i) hergestellten Hydrazons werden in 30 ml Ethanol suspendiert und mit 2 ml Orthoessigsäuretriethylester zur Titelverbindung umgesetzt. Man erhält 2,2 g (80 % d. Th.) in Form von Kristallen vom Schmp.: 203 °C.

Beispiel 27

6-[4-(4-Isobutylphenethyl)phenyl]-8,9-dihydro-1-methyl-4H,7H-cyclopenta[4,5]thieno[3,2-f][1,2,4]triazolo[4,3-a][1,4]diazepin

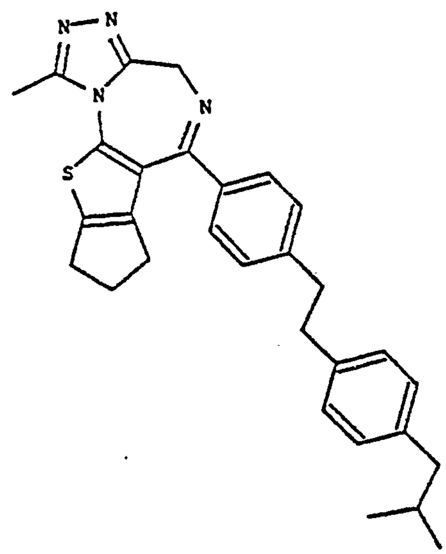

Die Synthesedurchführung zur Herstellung der Titelverbindung erfolgt im wesentlichen in Analogie zu den in Beispiel 1 bzw. Beispiel 19 beschriebenen Verfahren.

a) Herstellung des p-Isobutylbenzaldehyds

Eine Mischung aus 117,4 g (0,87 mol) Isobutylbenzol und 180 ml Benzol wird im Autoklaven vorgelegt. Anschließend wird die Lösung unter einem Chlorwasserstoffdruck von ca. 2 bis 3 bar mit Chlorwasserstoff gesättigt. Danach wird Kohlenmonoxyd bis zu einem Druck von 35 bar aufgepreßt. Nach einem anfänglichen Druckabfall wird im Autoklaven für einen Zeitraum von 4 Stunden erneut ein Kohlenmonoxid-Druck im Bereich von 35 bis 39 bar eingestellt. Nach dem Entspannen wird die Reaktionslösung in eine Mischung aus 1,1 kg Eis und 10 ml konzentrierter Salzsäure getropft. Anschließend wird die organische Phase separiert und mit Wasser und danach mit einer gesättigten Natriumhydrogencarbonat-Lösung gewaschen. Nach dem Trocknen und Einengen der Lösung destilliert man den verbliebenen Rückstand im Wasserstrahlvakuum und erhält 55 g (39 % d. Th.) des p-Isobutylbenzaldehyds $Sdp._{40\ mbar}$: 132-140 °C.

b) Wittig-Olefinierung (entsprechend Schritt 4 nach 5 im Reaktionsschema)

12,5 g einer Natriumhydrid-Dispersion (55 %-ig) werden unter Inertgas in 150 ml absolutem Dimethylsulfoxid vorgelegt und gemäß Beispiel 1 mit 140,8 g (0,29 mol) des entsprechenden Phosphoniumsalzes - suspendiert in 290 ml Dimethylsulfoxid - und darauf mit 47,0 g (0,29 mol) 4-Isobutylbenzaldehyd versetzt. Die Aufarbeitung gemäß Beispiel 1 liefert 68 g (80 % d. Th.) des entsprechenden Olefins.

c) Reduktion des Olefins (entsprechend Schritt 5 nach 6 im Reaktionsschema)

Analog Beispiel 1 werden 68 g (0,23 mol) des nach Verfahrensschritt b) hergestellten Olefins in 1 l Tetrahydrofuran in Gegenwart von Raney-Nickel als Katalysator 5 Stunden lang bei 20 °C und einem Wasserstoffdruck von 5 bar hydriert. Man erhält 58,0 g (85 % d. Th.) des entsprechenden 1,2-Diphenylethanderivats als Öl.

d) Herstellung des Cyanoketons (entsprechend Schritt 6 nach 7 im Reaktionsschema)

58,0 g (196 mmol) des nach Verfahrensschritt c) hergestellten 1,2-Diphenylethanderivats werden in 185 ml Toluol gelöst und mit 8,9 g einer 55 %-igen Natriumhydrid-Dispersion und 25,4 ml Acetonitril versetzt. Nach der Reaktionsdurchführung analog Beispiel 1 erhält man 83,0 g (83 % d. Th.) des entsprechenden Cyanoketons als Öl.

e) Herstellung des Aminoketons (entsprechend Schritt 7 nach 8 im Reaktionsschema)

48,0 g (162 mmol) des nach Verfahrensschritt d) hergestellten Cyanoketons vom Typ 7 werden mit 12,4 g Cyclopentanon und 5,8 g Schwefel in Dimethylformamid vermischt sowie mit 12,4 ml Triethylamin in 160 ml Ethanol versetzt und 7 Stunden bei 60 °C gerührt. Das Reaktionsgemisch wird anschließend - in Analogie zu Beispiel 19 - mit 1,2-Dichlorethan verdünnt und zweimal mit Wasser gewaschen. Die organische Phase wird i. Vak. eingeengt und an einer mit Siliciumdioxid gefüllten Säule mit Dichlormethan als Elutionsmittel chromatographiert. Man erhält nach dem Umkristallisieren aus Diisopropylether 17,5 g (27 % d. Th.) des Aminoketons (vom Typ 8) als gelbe Kristalle vom Schmp.: 108-110 °C.

f) Herstellung des Bromacetylamids (entsprechend Schritt 8 nach 9)

17,0 g (42 mmol) des nach Verfahrensschritt e) hergestellten Aminoketons werden in 150 ml Dichlormethan gelöst und mit 3,4 ml Pyridin versetzt. Danach werden 3,3 ml Bromacetylbromid zugetropft. Nach der weiteren Reaktionsdurchführung - analog Beispiel 19 - erhält man 18,5 g (84 % d. Th.) des entsprechenden Bromacetylamids als Öl.

g) Herstellung des Amino-Derivats (entsprechend Schritt 9 nach 10)

18,5 g (35 mmol) der nach Verfahrensschritt f) hergestellten Bromacetylverbindung werden in 150 ml Essigsäureethylester gelöst und analog Beispiel 1 mit gasförmigem Ammoniak behandelt. Nach der weiteren Reaktionsdurchführung gemäß Beispiel 1 erhält man 16,0 g (98 % d. Th.) der entsprechenden Aminoverbindung vom Typ 10 in Form von Kristallen vom Schmp.: 133-135 °C.

h) Herstellung des Diazepinon-Derivats (entsprechend Schritt 10 nach 11 im Reaktionsschema)

16,0 g (35 mmol) nach Verfahrensschritt g) hergestellten Aminoverbindung werden in 200 ml Toluol in Gegenwart von 80 g Kieselgel cyclisiert. Die weitere Reaktionsdurchführung und Aufarbeitung gemäß Beispiel 1 führt zu 10,0 g (65 % d. Th.) des entsprechenden Diazepinons vom Typ 11 in Form von Kristallen vom Schmp.: 218 °C.

i) Herstellung des Diazepinthion-Derivats (entsprechend Schritt 11 nach 12 im Reaktionsschema

9,0 g (20 mmol) des nach Verfahrensschritt h) hergestellten Diazepinon-Derivates werden in 165 ml Diethylenglycoldimethylether gelöst und mit 3,5 g Natriumhydrogencarbonat sowie mit 5,5 g Phosphorpentasulfid versetzt. Die Reaktionsmischung wird - wie in Beispiel 1 beschrieben - behandelt. Nach dem Aufarbeiten und Umkristallisieren aus Diethylether erhält man 5,2 g (56 % d. Th.) des entsprechenden Diazepinthion-Derivates als Kristalle vom Schmp.: 218 °C.

j) Herstellung der Hydrazono-Verbindung vom Typ 13 (entsprechend Schritt 12 nach 13 im Reaktionsschema)

5,2 g (11,0 mmol) des nach Verfahrensschritt i) hergestellten Diazepinthions werden in 40 ml Tetrahydrofuran vorgelegt und mit 0,7 ml Hydrazin-Hydrat versetzt. Nach Durchführung der Umsetzung gemäß Beispiel 1 und Umkristallisieren aus Diethylether erhält man 4,8 g (93 % d. Th.) des entsprechenden Hydrazons vom Typ 13 als Kristalle vom Schmp.: 198-200 °C (Zersetzung)

k) Herstellung der Titelverbindung (entsprechend Schritt 13 nach 14 im Reaktionsschema)

4,8 g (10,0 mmol) des nach Verfahrensschritt j) hergestellten Hydrazons werden in 30 ml Ethanol suspendiert und mit 3 ml Orthoessigsäuretriethylester zur Titelverbindung umgesetzt. Man erhält nach dem Umkristallisieren aus Essigsäureethylester 4,4 g (87 % d. Th.) der Titelverbindung in Form von Kristallen vom Schmp.: 210 °C.

Beispiel 28

6-(4-Phenethylphenyl)-8,9-dihydro-1-methyl-4H,7H-cyclopenta[4,5]thieno[3,2-f][1,2,4]triazolo[4,3-a][1,4]-diazepin

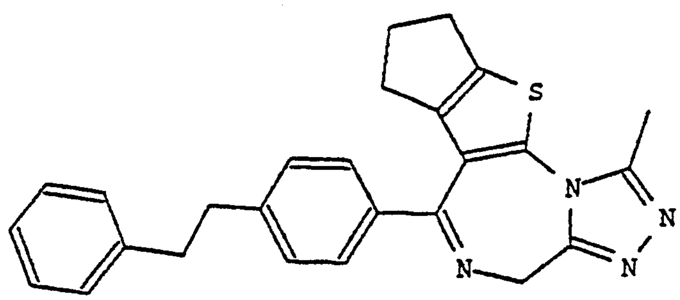

Die Synthesedurchfühung zur Herstellung der Titelverbindung erfolgt in Analogie zu den in Beispiel 1 bzw. Beispiel 19 beschriebenen Verfahren mit der Ausnahme, daß bei der Wittig-Olefinierung (im Reaktionsschema Schritt 4 nach 5) Benzaldehyd anstelle von p-Trifluormethylbenzaldehyd eingesetzt wird.

a) Wittig-Olefinierung (entsprechend Schritt 4 nach 5 im Reaktionsschema)

8,7 g einer Natriumhydrid-Dispersion (55 %-ig) werden unter Inertgas in 100 ml absolutem Dimethylsulfoxid vorgelegt und gemäß Beispiel 1 mit 98 g (0,2 mol) des entsprechenden Phosphoniumsalzes - suspendiert in 200 ml Dimethylsulfoxid - und darauf mit 21,5 g Benzaldehyd versetzt. Die Aufarbeitung gemäß Beispiel 1 liefert 30,2 g (48 % d. Th.) des entsprechenden Olefins als Öl.

b) Reduktion des Olefins (entsprechend Schritt 5 nach 6 im Reaktionsschema)

Analog Beispiel 1 werden 30,2 g (0,13 mol) des nach Verfahrensschritt a) hergestellten Olefins in 700 ml Tetrahydrofuran in Gegenwart von Raney-Nickel als Katalysator über einen Zeitraum von 3,5 Stunden bei 20 °C und 5 bar Wasserstoffdruck hydriert. Man erhält 30,5 g (100 % d. Th.) des 1,2-Diphenylethanderivats als Öl.

c) Herstellung des Cyanoketons (entsprechend Schritt 6 nach 7 im Reaktionsschema)

30,5 g (125 mmol) des nach Verfahrensschritt b) hergestellten Diphenylethanderivats werden in 120 ml Toluol gelöst und mit 5,7 g Natriumhydrid-Dispersion (55 %-ig) und 16,4 ml Acetonitril versetzt. Nach der Reaktionsdurchführung - analog Beispiel 1 - erhält man 19,0 g (61 % d. Th.) des entsprechenden Cyanoketons als Kristalle vom Schmp.: 80-83 °C.

d) Herstellung des Aminoketons (entsprechend Schritt 7 nach 8 im Reaktionsschema)

19,0 g (76 mmol) des nach Verfahrensschritt c) hergestellten Cyanoketons vom Typ 7 werden mit 5,8 g Cyclopentanon und 2,8 g Schwefel in Dimethylformamid vermischt sowie mit 5,8 ml Triethylamin in 76 ml Ethanol versetzt und 7 Stunden bei 60 °C gerührt. Das Reaktionsgemisch wird anschließend - in Analogie zu Beispiel 19 - mit 1,2-Dichlorethan verdünnt und zweimal mit Wasser gewaschen. Die organische Phase wird i. Vak. eingeengt und an einer mit Siliciumdioxyd gefüllten Säule mit Dichlormethan als Elutionsmittel chromatographiert. Man erhält nach dem Umkristallisieren aus Diisopropylether 9,2 g (35 % d. Th.) des Aminoketons (vom Typ 8) als gelbe Kristalle vom Schmp.: 118-121 °C.

e) Herstellung des Bromacetylamids (entsprechend Schritt 8 nach 9)

9,5 g (27 mmol) des nach Verfahrensschritt d) hergestellten Aminoketons werden in 85 ml Dichlormethan gelöst und mit 2,2 ml Pyridin versetzt. Danach werden 2,3 ml Bromacetylbromid zugetropft. Nach der weiteren Reaktionsdurchführung - analog Beispiel 19 - und Umkristallisieren aus Diethylether erhält man 12,0 g (94 % d. Th.) des entsprechenden Bromacetylamids in Form gelber Kristalle vom Schmp.: 108 °C.

f) Herstellung des Amino-Derivats (entsprechend Schritt 9 nach 10)

12,0 g (26 mmol) der nach e) hergestellten Bromacetylverbindung werden in 150 ml Essigsäureethylester gelöst und analog Beispiel 1 mit gasförmigem Ammoniak behandelt. Nach der weiteren Reaktionsdurchführung gemäß Beispiel 1 erhält man 9,5 g (92 % d. Th.) der entsprechenden Aminoverbindung vom Typ 10, welche ohne weitere Reinigungsmaßnahmen im anschließenden Reaktionsschritt cyclisiert wird.

g) Herstellung des Diazepinon-Derivates (entsprechend Schritt 10 nach 11 im Reaktionsschema)

9,5 g (24 mmol) der nach Verfahrensschritt f) hergestellten Aminoverbindung werden in 200 ml Toluol in Gegenwart von 50 g Kieselgel cyclisiert. Die weitere Reaktionsdurchführung und Aufarbeitung gemäß Beispiel 1 führt nach dem Umkristallisieren aus Diethylether zu 5,2 g (57 % d. Th.) des entsprechenden Diazepinons vom Typ 11 in Form von Kristallen vom Schmp.: 192-195 °C.

h) Herstellung des Diazepinthion-Derivates (entsprechend Schritt 11 nach 12) im Reaktionsschema)

3,9 g (10 mmol) des nach Verfahrensschritt g) hergestellten Diazepinon-Derivates werden in 80 ml Diethylenglycoldimethylether gelöst und mit 1,7 g Natriumhydrocarbonat sowie mit 2,5 g Phosphorpentasulfid versetzt. Die Reaktionsmischung wird - wie in Beispiel 1 beschrieben - behandelt. Nach dem Aufarbeiten erhält man 3,7 g (91 % d. Th.) des entsprechenden Diazepinthion-Derivates als Kristalle vom Schmp.: 225 °C.

i) Herstellung der Hydrazono-Verbindung vom Typ 13 (entsprechend Schritt 12 nach 13 im Reaktionsschema)

3,7 g (92 mmol) des nach Verfahrensschritt h) hergestellten Diazepinthions werden in 60 ml Tetrahydrofuran vorgelegt und mit 0,5 ml Hydrazin-Hydrat versetzt. Nach Durchführung der Umsetzung gemäß Beispiel 1 erhält man nach dem Versetzen der Reaktionslösung mit 800 ml Diethylether 2,4 g (65 % d. Th.) des entsprechenden Hydrazons vom Typ 13 als Kristalle vom Schmp.: 175 °C.

j) Herstellung der Titelverbindung (entsprechend Schritt 13 nach 14 im Reaktionsschema)

2,4 g (6,0 mmol) des nach Verfahrensschritt i) hergestellten Hydrazons werden in 30 ml Ethanol suspendiert und mit 2 ml Orthoessigsäuretriethylester zur Titelverbindung umgesetzt. Man erhält nach dem Umkristallisieren aus Diethylether 1,2 g (47 % d. Th.) in Form von Kristallen. Schmp. 198° C

Beispiel 29

6-(4-Benzylphenyl)-8,9-dihydro-1-methyl-4H,7H-cyclopenta[4,5]thieno[3,2-f][1,2,4]triazolo[4,3-a][1,4]diazepin

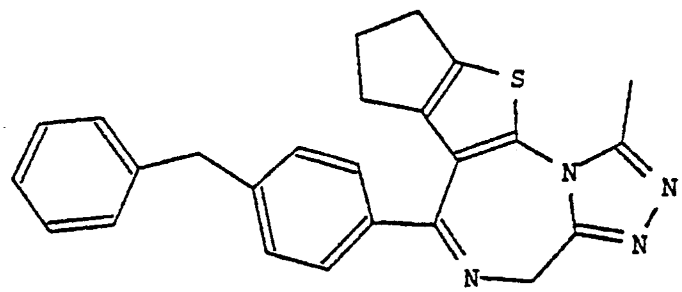

a) Herstellung von 4-Benzoylbenzoesäure

50,0 g (260 mmol) 4-Methylbenzophenon werden in 260 ml Eisessig vorgelegt und auf 100° C erhitzt. Danach wird eine Lösung von 70 g Chrom-VI-oxid in einer Mischung aus 160 ml Wasser, 260 ml Eisessig und 50 ml konzentrierter Schwefelsäure langsam zugetropft. Anschließend wird das Reaktionsgemisch unter Rühren über einen Zeitraum von einer Stunde auf Rückflußtemperatur erhitzt. Danach wird das Reaktionsgemisch langsam in 4 l Eiswasser gegossen, wobei die Säure in Form von Kristallen ausfällt. Man erhält 40 g (69 % d. Th.) der 4-Benzoylbezoesäure als farblose Kristalle vom Schmp. 188° C.

b) Herstellung der 4-Benzylbenzoesäure

34,0 g (150 mmol) der 4-Benzoylbenzoesäure werden in 400 ml Tetrahydrofuran gelöst und in Gegenwart von 7,0 g Palladium auf Kohle (10 % Pd-Gehalt) bei 20° C und unter einem Wasserstoffdruck von 5 bar über einen Zeitraum von 3 Stunden hydriert. Nach dem Entspannen wird der Katalysator abfiltriert und das Lösungsmittel i. Vak. entfernt. Man erhält 30,0 g (94 % d. Th.) der 4-Benzylbenzoesäure als farblose Kristalle.

c) Herstellung des 4-Benzylbenzoesäureethylesters

35,0 g (165 mmol) der 4-Benzylbenzoesäure werden in 100 ml Ethanol gelöst und mit 2 ml konzentrierter Schwefelsäure versetzt und unter Rühren über einen Zeitraum von 5 Stunden auf Rückflußtemperatur erhitzt. Nach dem Abdestillieren des Ethanols i. Vak. wird der verbliebene Rückstand in Dichlormethan aufgenommen und nacheinander mit Wasser und gesättigter Natriumhydrogencarbonat-Lösung gewaschen. Die organische Phase wird separiert, getrocknet und eingeengt. Der verbliebene Rückstand wird an einer mit Siliciumdioxyd gefüllten Säule mit Dichlormethan als Elutionsmittel chromatographiert. Diejenigen Eluate, die einen einheitlichen Hauptfleck im Dünnschichtchromatogramm aufweisen, werden vereinigt und vom Elutionsmittel befreit. Man erhält auf diese Weise 35,7 g (90 % d. Th.) des Esters als Öl.

Die nachfolgenden Verfahrensschritte werden in Analogie zu den in Beispiel 1 bzw. Beispiel 19 beschriebenen Verfahren durchgeführt.

d) Herstellung des Cyanoketons (entsprechend Schritt 6 nach 7 im Reaktionsschema)

35,7 g (148 mmol) des nach Verfahrensschritt c) hergestellten Benzoesäureesters werden in 140 ml Toluol gelöst und mit 6,7 g Natriumhydrid-Dispersion (55 %-ig) und 19,2 ml Acetonitril versetzt. Nach der Reaktionsdurchführung - analog Beispiel 1 - erhält man 25,0 g (72 % d. Th.) des entsprechenden Cyanoketons als Kristalle vom Schmp.: 102-104 °C.

e) Herstellung des Aminoketons (entsprechend Schritt 7 nach 8 im Reaktionsschema)

23,5 g (100 mmol) des nach Verfahrensschritt d) hergestellten Cyanoketons werden mit 7,7 g Cyclopentanon und 3,6 g Schwefel in Dimethylformamid vermischt sowie mit 7,7 ml Triethylamin in 100 ml Ethanol versetzt und 7 Stunden bei 60 °C gerührt. Das Reaktionsgemisch wird anschließend - in Analogie zu Beispiel 19 - mit 1,2-Dichlorethan verdünnt und 2 mal mit Wasser gewaschen. Die organische Phase wird i. Vak. eingeengt und an einer mit Siliciumdioxyd gefüllten Säule mit Dichlormethan als Elutionsmittel chromatographiert. Man erhält 12,5 g (38 % d. Th.) des Aminoketons als Kristalle vom Schmp.: 115-120 °C.

f) Herstellung des Bromacetylamids (entsprechend Schritt 8 nach 9)

10,0 g (30 mmol) des nach Verfahrensschritt e) hergestellten Aminoketons werden in 100 ml Dichlormethan gelöst und mit 2,4 ml Pyridin versetzt. Danach werden 2,6 ml Bromacetylbromid zugetropft. Nach der weiteren Reaktionsdurchführung und Aufarbeitung - analog Beispiel 1 - erhält man 10,5 g (75 % d. Th.) des entsprechenden Bromacetylamids in Form von Kristallen vom Schmp.: 113-115 °C.

g) Herstellung des Amino-Derivats (entsprechend Schritt 9 nach 10)

10,5 g (23 mmol) der nach Verfahrensschritt f) hergestellten Bromacetylverbindung werden in 200 ml Essigsäureethylverbindung gelöst und analog Beispiel 19 mit gasförmigem Ammoniak behandelt. Nach der weiteren Reaktionsdurchführung und Aufarbeitung gemäß Beispiel 1 erhält man 7,0 g (78 % d. Th.) der entsprechenden Aminoverbidung in Form von Kristallen vom Schmp.: 118-121 °C.

h) Herstellung des Diazepinon-Derivats (entsprechend Schritt 10 nach 11 im Reaktionsschema)

7,0 g (18 mmol) der nach Verfahrensschritt g) hergestellten Aminoverbindung werden in 150 ml Toluol in Gegenwart von 35 g Kieselgel cyclisiert. Die weitere Reaktionsführung auf Aufarbeitung gemäß Beispiel 1 führt zu 5,5 g (82 % d. Th.) des entsprechenden Diazepinons vom Typ 11 in Form von Kristallen vom Schmp.: 246 °C.

i) Herstellung des Diazepinthion-Derivats (entsprechend Schritt 11 nach 12 im Reaktionsschema)

5,5 g (4,7 mmol) des nach Verfahrensschritt h) hergestellten Diazepinon-Derivats werden in 120 ml Diethylenglycoldimethylether gelöst und mit 3,6 g Phosphorpentasulfid sowie mit 2,5 g Natriumhydrogencarbonat versetzt. Die Reaktionsmischung wird - wie in Beispiel 1 beschrieben - behandelt. Nach der Aufarbeitung erhält man 5,5 g (96 % d. Th.) des entsprechenden Diazepinthion-Derivates als Kristalle

j) Herstellung der Hydrazono-Verbindung vom Typ 13 (entsprechend Schritt 12 nach 13 im Reaktionsschema)

5,5 g (14,0 mmol) des nach i) hergestellten Diazepinthions werden in 90 ml Tetrahydrofuran vorgelegt und mit 0,8 ml Hydrazinhydrat versetzt. Nach Durchführung der Umsetzung gemäß Beispiel 1 erhält man 4,5 g (82 % d. Th.) des entsprechenden Hydrazons vom Typ 13 als Kristalle vom Schmp.: 205 °C.

k) Herstellung der Titelverbindung (entsprechend Schritt 13 nach 14 im Reaktionsschema)

4,5 g (12,0 mmol) des nach Verfahrensschritt j) hergestellten Hydrazons werden in 50 ml Ethanol suspendiert und mit 3 ml Orthoessigsäuretriethylester zur Titelverbindung umgesetzt. Man erhält 3,3 g (69 % d. Th.) der Titelverbindung in kristalliner Form. Schmp.: 204-206 °C

Beispiel 30

6-(3-Benzylphenyl)-8,9-dihydro-1-methyl-4H,7H-cyclopenta[4,5]thieno[3,2-f][1,2,4]triazolo[4,3-a][1,4]diazepin

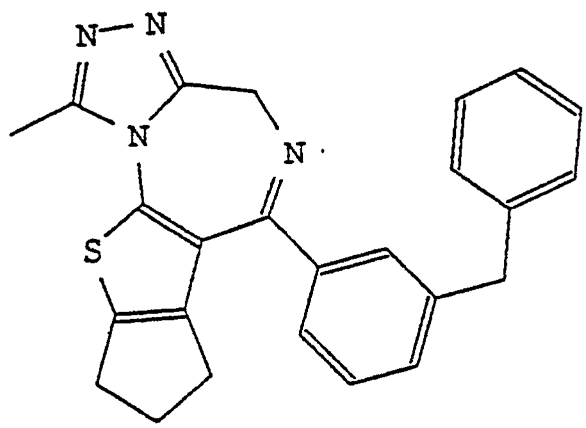

Das Herstellungsverfahren der Titelverbindung wird in Analogie zu Beispiel 29 durchgeführt. Anstelle von 4-Benzoylbenzoesäure wird jedoch 3-Benzoylbenzoesäure eingesetzt.

à) Herstellung der 3-Benzylbenzoesäure

40,0 g (176 mmol) des 3-Carboxybenzophenon werden in 400 ml Tetrahydrofuran gelöst und in Gegenwart von 8,0 g Palladium auf Kohle (10 % Pd-Gehalt) bei 20 °C über einen Zeitraum von 3 Stunden bei 20 °C unter einem Wasserstoffdruck von 5 bar hydriert. Nach dem Entspannen wird der Katalysator abfiltriert und das Lösungsmittel i. Vak. entfernt. Man erhält 37,0 g (98 % d. Th.) der 3-Benzylbenzoesäure als farblose Kristalle vom Schmp.: 107-108 °C.

b) Herstellung des 3-Benzylbenzoesäureethylesters

37,0 g (174 mmol) der 3-Benzylbenzoesäure werden in 100 ml Ethanol gelöst und mit 2 ml konzentrierter Schwefelsäure versetzt und unter Rühren über einen Zeitraum von 5 Stunden auf Rückflußtemperatur erhitzt. Nach dem Abdestillieren des Ethanols i. Vak. wird der verbliebene Rückstand in Dichlormethan aufgenommen und nacheinander mit Wasser und gesättigter Natriumhydrogencarbonat-Lösung gewaschen. Die organische Phase wird separiert, getrocknet und eingeengt. Der verbliebene Rückstand wird an einer mit Siliciumdioxyd gefüllten Säule mit Dichlormethan als Elutionsmittel chromatographiert. Diejenigen Eluate, die einen einheitlichen Hauptfleck im Dünnschichtchromatogramm aufweisen, werden vereinigt und vom Elutionsmittel befreit. Man erhält 23,7 g (57 % d. Th.) des Esters als gelbes Öl.

Die nachfolgenden Verfahrensschritte werden wiederum in Analogie zu Beispiel 1 bzw. Beispiel 19 durchgeführt.

c) Herstellung des Cyanoketons (entsprechend Schritt 6 nach 7 im Reaktionsschema)

23,5 g (0,1 mol) des nach Verfahrensschritt b) hergestellten Benzoesäureesters werden in 90 ml Toluol

gelöst und mit 4,5 g Natriumhydrid-Dispersion (55 %-ig) und 13 ml Acetonitril versetzt. Nach der Reaktionsdurchführung analog Beispiel 1 erhält man 14,0 g (61 % d. Th.) des entsprechenden Cyanoketons als Kristalle vom Schmp.: 102-104° C.

d) Herstellung des Aminoketons (entsprechend Schritt 7 nach 8 im Reaktionsschema)

14,0 g (60 mmol) des nach Verfahrensschritt c) hergestellten Cyanoketons werden mit 4,6 g Cyclopentanon und 2,2 g Schwefel in Dimethylformamid vermischt sowie mit 4,6 ml Triethylamin in 60 ml Ethanol versetzt und 7 Stunden bei 60° C gerührt. Das Reaktionsgemisch wird anschließend - in Analogie zu Beispiel 19 - mit 1,2-Dichlorethan verdünnt und zweimal mit Wasser gewaschen. Die organische Phase wird i. Vak. eingeengt und an einer mit Siliciumdioxid gefüllten Säule mit Dichlormethan als Elutionsmittel chromatographiert. Man erhält 9,3 g (47 % d. Th.) des Aminoketons als Kristalle vom Schmp.: 140-142° C.

e) Herstellung des Bromacetylamids (entsprechend Schritt 8 nach 9)

9,0 g (27 mmol) des nach Verfahrensschritt d) hergestellten Aminoketons werden in 100 ml Dichlormethan gelöst und mit 2,2 ml Pyridin versetzt. Danach werden 2,1 ml Bromacetylbromid zugetropft. Nach der weiteren Reaktionsdurchführung - analog Beispiel 1 - und Umkristallisieren aus Diisopropylether erhält man 10,0 g (82 % d. Th.) des entsprechenden Bromacetylamids als Öl.

f) Herstellung des Amino-Derivats (entsprechend Schritt 9 nach 10)

10,0 g (22 mmol) der nach Verfahrensschritt e) hergestellten Bromacetylverbindung werden in 150 ml Essigsäureethylester gelöst und analog Beispiel 1 mit gasförmigem Ammoniak behandelt. Nach der weiteren Reaktionsdurchführung gemäß Beispiel 1 erhält man 7,0 g (81 % d. Th.) der entsprechenden Aminoverbindung vom Typ 10 als Öl, welches im anschließenden Reaktionsschritt cyclisiert wird.

g) Herstellung des Diazepinon-Derivats (entsprechend Schritt 10 nach 11 im Reaktionsschema)

7,0 g (18 mmol) der nach Verfahrensschritt f) hergestellten Aminoverbindung werden in 150 ml Toluol in Gegenwart von 40 g Kieselgel cyclisiert. Die weitere Reaktionsführung und Aufarbeitung gemäß Beispiel 1 führt nach dem Umkristallisieren aus Ether zu 6,3 g (94 % d. Th.) des entsprechenden Diazepinons vom Typ 11 in Form von Kristallen vom Schmp.: 210-212° C.

h) Herstellung des Diazepinthion-Derivats (entsprechend Schritt 11 nach 12 im Reaktionsschema)

6,3 g (17 mmol) des nach Verfahrensschritt g) hergestellten Diazepinon-Derivates werden in 138 ml Diethylenglycoldimethylether gelöst und mit 4,1 g Phosphorpentasulfid sowie mit 2,9 g Natriumhydrogencarbonat versetzt. Die Reaktionsmischung wird - wie in Beispiel 1 beschrieben - behandelt und aufgearbeitet. Man erhält 6,3 g (96 % d. Th.) des entsprechenden Diazepinthion-Derivats als Kristalle.

i) Herstellung der Hydrazono-Verbindung vom Typ 13 (entsprechend Schritt 12 nach 13 im Reaktionsschema)

6,3 g (16,2 mmol) des nach h) hergestellten Diazepinthions werden in 60 ml Tetrahydrofuran vorgelegt und mit 0,9 ml Hydrazin-Hydrat versetzt. Nach Durchführung der Umsetzung gemäß Beispiel 1 erhält man 4,6 g (73 % d. Th.) der entsprechenden Hydrazonoverbindung vom Typ 13 in Form von Kristallen vom Schmp.: 190-192° C.

j) Herstellung der Titelverbindung (entsprechend Schritt 13 nach 14 im Reaktionsschema)

4,6 g (12,0 mmol) des nach Verfahrensschritt i) hergestellten Hydrazons werden in 30 ml Ethanol suspendiert und mit 3 ml Orthoessigsäuretriethylester zur Titelverbindung umgesetzt. Man erhält 4,4 g (90 % d. Th.) als Kristalle vom Schmp.: 213-215 °C.

Beispiel 31

6-(4-Isobutylphenyl)-8,9-dihydro-1-methyl-4H,7H-cyclopenta[4,5]thieno[3,2-f][1,2,4]triazolo[4,3-a][1,4]diazepin

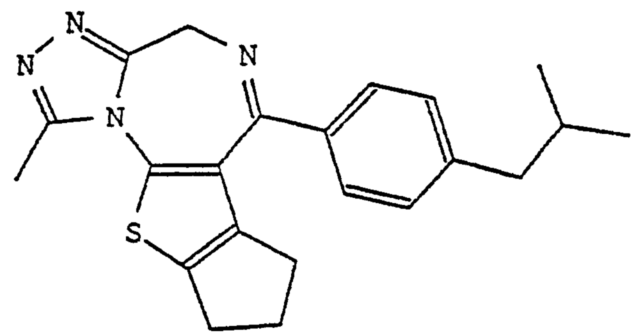

Die Darstellung von 4-Isobutylbenzaldehyd erfolgt in Analogie zu Beispiel 27:

a) Herstellung der 4-Isobutylbenzoesäure

70,0 g (430 mmol) 4-Isobutylbenzaldehyd werden in 1 l 2N Natronlauge vorgelegt und nach der Zugabe von 45,3 g Kaliumpermanganat 2 Tage bei Raumtemperatur gerührt. Die resultierende dunkle Suspension wird über Kieselgel/Kohle abgesaugt. Nach Ansäuern des Filtrats mit Salzsäure erhält man 34 g (44 % d. Th.) der 4-Isobutylbenzoesäure als farblose Kristalle vom Schmp.: 133-140 °C.

b) Herstellung des 4-Isobutylbenzoesäureethylesters

34,0 g (190 mmol) der 4-Isobutylbenzoe säure werden in 100 ml Ethanol gelöst und mit 2 ml konzentrierter Schwefelsäure versetzt und unter Rühren über einen Zeitraum von 5 Stunden auf Rückflußtemperatur erhitzt. Nach dem Abdestillieren des Ethanols i. Vak. wird der verbliebene Rückstand in Dichlormethan aufgenommen und nacheinander mit Wasser und gesättigter Natriumhydrogencarbonat-Lösung gewaschen. Die organische Phase wird separiert, getrocknet und eingeengt. Der verbliebene Rückstand wird an einer mit Siliciumdioxyd gefüllten Säule mit Dichlormethan als Elutionsmittel chromatographiert. Diejenigen Eluate, die einen einheitlichen Hauptfleck im Dünnschichtchromatogramm aufweisen, werden vereinigt und vom Elutionsmittel befreit. Man erhält 27,0 g (69 % d. Th.) des Esters als Öl.

Die nachfolgenden Verfahrensschritte werden in Analogie zu Beispiel 1 bzw. Beispiel 19 durchgeführt.

c) Herstellung des Cyanoketons (entsprechend Schritt 6 nach 7 im Reaktionsschema)

27,0 g (131 mmol) des nach Verfahrensschritt b) hergestellten Benzoesäureesters werden in 125 ml Toluol gelöst und mit 6,0 g Natriumhydrid-Dispersion (55 %-ig) und 17 ml Acetonitril versetzt. Nach der Reaktionsdurchführung analog Beispiel 1 erhält man 21,0 g (80 % d. Th.) des entsprechenden Cyanoketons.

d) Herstellung des Aminoketons (entsprechend Schritt 7 nach 8 im Reaktionsschema

21,0 g (104 mmol) des nach Verfahrensschritt c) hergestellten Cyanoketons werden mit 8,0 g

Cyclopentanon und 3,7 g Schwefel in Dimethylformamid vermischt sowie mit 8 ml Triethylamin in 100 ml Ethanol versetzt und 7 Stunden bei 60 ° C gerührt. Das Reaktionsgemisch wird anschließend - in Analogie zu Beispiel 19 - mit 1,2 Dichlorethan verdünnt und zweimal mit Wasser gewaschen. Die organische Phase wird i. Vak. eingeengt und an einer mit $SiO_2$ gefüllten Säule mit Dichlormethan als Elutionsmittel chromatographiert. Man erhält nach dem Umkristallisieren aus Diisopropylether 6,3 g (20 % d. Th.) des Aminoketons (vom Typ 8) als Kristalle vom Schmp.: 126 ° C.

e) Herstellung des Bromacetylamids (entsprechend Schritt 8 nach 9)

6,0 g (20 mmol) des nach d) hergestellten Aminoketons werden in 70 ml Dichlormethan gelöst und mit 1,6 ml Pyridin versetzt. Danach werden 1,6 ml Bromacetylbromid zugetropft. Nach der weiteren Reaktionsdurchführung - analog Beispiel 1 - und Umkristallisieren aus Diisopropylether erhält man 7,8 g (93 % d. Th.) des entsprechenden Bromacetylamids als Kristalle.

f) Herstellung des Amino-Derivats (entsprechend Schritt 9 nach 10)

7,8 g (37 mmol) der nach Verfahrensschritt e) hergestellten Bromacetylverbindung werden in 100 ml Essigsäureethylester gelöst und analog Beispiel 1 mit gasförmigem Ammoniak behandelt. Nach der weiteren Reaktionsdurchführung gemäß Beispiel 1 erhält man 6,5 g (98 % d. Th.) der entsprechenden Aminoverbindung als schaumiges Öl (Schaum).

g) Herstellung des Diazepinon-Derivates (entsprechend Schritt 10 nach 11 im Reaktionsschema)

6,5 g (18 mmol) der nach Verfahrensschritt f) hergestellten Aminoverbindung werden in 200 ml Toluol in Gegenwart von 40 g Kieselgel cyclisiert. Die weitere Reaktionsführung und Aufarbeitung gemäß Beispiel 1 führt zu 5,5 g (92 % d. Th.) des entsprechenden Diazepinons vom Typ 11 in Form eines schaumigen Öls.

h) Herstellung des Diazepinthion-Derivates (entsprechend Schritt 11 nach 12 im Reaktionsschema)

5,5 g (16 mmol) des nach Verfahrensschritt g) hergestellten Diazepinon-Derivates werden in 65 ml Pyridin gelöst und mit 5,5 g Phosphorpentasulfid versetzt. Die Reaktionsmischung wird über einen Zeitraum von zwei Stunden auf 60 ° C erhitzt. Anschließend wird das Gemisch mit Wasser verdünnt und mit Dichlormethan extrahiert. Nach dem Entfernen des Extraktionsmittels i. Vak. erhält man 2,1 g (36 % d. Th.) des entsprechenden Diazepinthion-Derivates als Kristalle vom Schmp.: 195-200 ° C

i) Herstellung der Hydrazono-Verbindung vom Typ 13 (entsprechend Schritt 12 nach 13 im Reaktionsschema)

2,1 g (6,0 mmol) des nach Verfahrensschritt h) hergestellten Diazepinthions werden in 20 ml Tetrahydrofuran vorgelegt und mit 0,4 ml Hydrazin-Hydrat versetzt. Nach Durchführung der Umsetzung gemäß Beispiel 1 erhält man nach dem Umkristallisieren aus Diethylether 1,3 g des entsprechenden Hydrazons vom Typ 13 als Kristalle.

j) Herstellung der Titelverbindung (entsprechend Schritt 13 nach 14 im Reaktionsschema)

1,3 g (3,8 mmol) des nach Verfahrensschritt i) hergestellten Hydrazons werden in 20 ml wasserfreiem Ethanol suspendiert und mit 1 ml Orthoessigsäuretriethylester zur Titelverbindung umgesetzt. Man erhält 1,0 g (70 % d. Th.) in Form von farblosen Kristallen vom Schmp.: 190-192 ° C.

Beispiel 32

6-[4-((4-Phenethyl)phenethyl)phenethyl]-8,9-dihydro-1-methyl-4H,7H-cyclopenta[4,5]thieno[3,2-f][1,2,4]-triazolo[4,3-a][1,4]diazepin

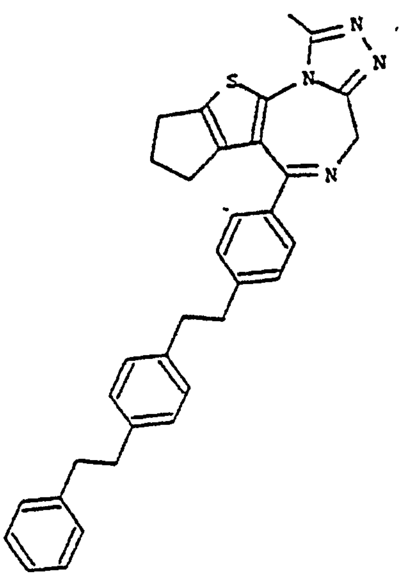

a) Herstellung des 4-[2-(4-Diethoxyphenyl)ethenyl] benzoesäuremethylesters (1. Wittig-Olefinierung) und Überführung in den freien Aldehyd.

12,5 g einer 55 %-igen Natriumhydrid-Dispersion werden unter Inertgas in 150 ml absolutem Dimethylsulfoxid vorgelegt und mit 140,8 g (287 mmol) des in Beispiel 1 beschriebenen Phosphoniumsalzes -suspendierend in 300 ml Dimethylsulfoxid - und anschließend mit 57,7 (290 mmol) Diethoxymethanbenzaldehyd versetzt. Die Durchführung der Reaktion und die Aufarbeitung erfolgt in Analogie zu Beispiel 1. Das resultierende Diethylacetal wird anschließend in Methanol gelöst und mittels verdünnter Salzsäure in den freien Aldehyd überführt. Zur Aufarbeitung wird das Reaktionsmedium i. Vak. entfernt; der verbliebene Rückstand wird in Dichlormethan aufgenommen und mit Wasser gewaschen. Nach dem Trocknen der organischen Phase, dem Abfiltrieren des Trockenmittels und dem Abdestillieren des Lösungsmittels i. Vak. wird das Gemisch der beiden Stilben-Isomeren durch Kristallisation aus Diisopropylether in die Isomeren getrennt. Man erhält zunächst 35 g des entsprechenden cis-Isomeren in Form farbloser Kristalle. Nach dem Einengen der Mutterlauge erhält man 24 g des trans-Isomeren als Kristalle vom Schmp.: 145-148 °C.

b) Herstellung des 1-(2-(4-Methoxycarbonylphenyl)-ethyl)-4-phenylethenylbenzols (2. Wittig-Olefinierung)

Der nach Verfahrensschritt a) erhaltene Aldehyd (trans-Isomeres) wird analog Beispiel 1 einer weiteren Wittig-Olefinierung unterworfen: 5,7 g einer 55 %-igen Natriumhydrid-Dispersion werden unter Schutzgasatmosphäre in 75 ml Dimethylsulfoxid vorgelegt, mit 61,9 g (143 mmol) des in Beispiel 1 beschriebenen Phosphoniumsalzes - suspendiert in 150 ml Dimethylsulfoxid - sowie mit 38,0 g (143 mmol) des nach Verfahrensschritt a) hergestellten Aldehyds versetzt. Nach Reaktionsdurchführung und Aufarbeitung gemäß Beispiel 1 erhält man 19 g (39 % d. Th.) des im Titel bezeichneten Benzolderivats als gelbe Kristalle vom Schmp.: 110 °C , welches in Analogie zu Beispiel 19 weiter umgesetzt wird.

c) Reduktion des Diolefins

19 g (55 mmol) des nach Verfahrensschritt b) hergestellten Diolefins werden in 250 ml Tetrahydrofuran in Gegenwart von Raney-Nickel als Katalysator bei 20 °C und 5 bar Wasserstoffdruck 2 Stunden lang hydriert. Man erhält 18,7 g (78 % d. Th.) des 1,4-Diphenethylbenzol-Derivats als farblose Kristalle vom Schmp.: 97-100 °C

d) Herstellung des Cyanoketons (entsprechend Schritt 6 nach 7 im Reaktionsschema)

18,7 g (54 mmol) des nach Verfahrensschritt c) hergestellten Diphenylethanderivats werden in 100 ml Toluol gelöst und mit 2,4 g Natriumhydrid-Dispersion (55 %-ig) und 7 ml Acetonitril versetzt. Nach der Reaktionsdurchführung analog Beispiel 1 erhält man 9,0 g (47 % d. Th.) des entsprechenden Cyanoketons als Kristalle vom Schmp.: 128-130 °C.

e) Herstellung des Aminoketons (entsprechend Schritt 7 nach 8 im Reaktionsschema)

9,0 g (25 mmol) des nach Verfahrensschritt d) hergestellten Cyanoketons vom Typ 7 werden mit 2,0 g Cyclopentanon und 0,9 g Schwefel in Dimethylformamid vermischt sowie mit 2 ml Triethylamin in 50 ml Ethanol versetzt und 7 Stunden bei 60 °C gerührt. Das Reaktionsgemisch wird anschließend - in Analogie zu Beispiel 19 - mit 1,2-Dichlorethan verdünnt und zweimal mit Wasser gewaschen. Die organische Phase wird i. Vak. eingeengt und an einer mit Siliciumdioxyd gefüllten Säule mit Dichlormethan als Elutionsmittel chromatographiert. Man erhält nach dem Umkristallisieren aus Diisopropylether 3,5 g (30 % d. Th.) des Aminoketons (vom Typ 8) als Kristalle vom Schmp.: 115-120 °C.

f) Herstellung des Bromacetylamids (entsprechend Schritt 8 nach 9)

4,6 g (10 mmol) des nach Verfahrensschritt e) hergestellten Aminoketons werden in 50 ml Dichlormethan gelöst und mit 0,80 ml Pyridin versetzt. Danach werden 0,87 ml Bromacetylbromid zugetropft. Nach der weiteren Reaktionsdurchführung -analog Beispiel 19 - und Umkristallisieren aus Diisopropylether erhält man 5,0 g (86 % d. Th.) des entsprechenden Bromacetylamids in Form gelber Kristalle vom Schmp.: 112-116 °C.

g) Herstellung des Amino-Derivats (entsprechend Schritt 9 nach 10)

5,0 g (96 mmol) der nach Verfahrensschritt f) hergestellten Bromacetylverbindung werden in 100 ml Essigsäureethylester gelöst und analog Beispiel 1 mit gasförmigem Ammoniak behandelt. Nach der weiteren Reaktionsdurchführung gemäß Beispiel 1 erhält man 4,4 g (99 % d. Th.) der entsprechenden Aminoverbindung vom Typ 10, welche im anschließenden Reaktionsschritt cyclisiert wird.

h) Herstellung des Diazepinon-Derivates (entsprechend Schritt 10 nach 11 im Reaktionsschema)

4,4 g (8 mmol) der nach Verfahrensschritt g) hergestellten Aminoverbindung werden in 150 ml Toluol in Gegenwart von 25 g Kieselgel cyclisiert. Die weitere Reaktionsführung und Aufarbeitung gemäß Beispiel 1 führt zu 3,4 g (80 % d. Th.) des entsprechenden Diazepinons vom Typ 11 in Form von Kristallen vom Schmp.: 210-215 °C.

i) Herstellung des Diazepinthion-Derivates (entsprechend Schritt 11 nach 12 im Reaktionsschema)

3,4 g (7 mmol) des nach Verfahrensschritt h) hergestellten Diazepinon-Derivates werden in 27 ml Pyridin gelöst und mit 2,3 g Phosphorpentasulfid versetzt. Die Reaktionsmischung wird über einen Zeitraum von zwei Stunden auf 60 °C erhitzt. Anschließend wird das Gemisch mit Wasser verdünnt und mit Dichlormethan extrahiert. Nach dem Entfernen des Extraktionsmittels i. Vak. erhält man 2,6 g (74 % d. Th.) des entsprechenden Diazepinthion-Derivates als Kristalle vom Schmp.: 190 °C.

j) Herstellung der Hydrazono-Verbindung vom Typ 13 (entsprechend Schritt 12 nach 13 im Reaktionsschema)

2,6 g (5 mmol) des nach Verfahrensschritt i) hergestellten Diazepinthions werden in 30 ml Tetrahydrofuran vorgelegt und mit 0,3 ml Hydrazin-Hydrat versetzt. Nach Durchführung der Umsetzung gemäß Beispiel

1 erhält man 2,5 g (97 % d. Th.) des entsprechenden Hydrazons vom Typ 13.

k) Herstellung der Titelverbindung (entsprechend Schritt 13 nach 14 im Reaktionsschema)

2,5 g (5 mmol) des nach Verfahrensschritt j) hergestellten Hydrazons werden in 20 ml Ethanol suspendiert und mit 2 ml Orthoessigsäureethylester zur Titelverbindung umgesetzt. Man erhält 0,4 g (15 % d. Th.) in Form von Kristallen vom Schmp.: 228° C.

Beispiel 33

4-Methyl-6-(3,4,5-trimethoxyphenethyl)-phenyl-8,9-dihydro-1-methyl-4H,7H-cyclopenta[4,5]thieno[3,2-f]-[1,2,4]triazolo[4,3-a][1,4]diazepin

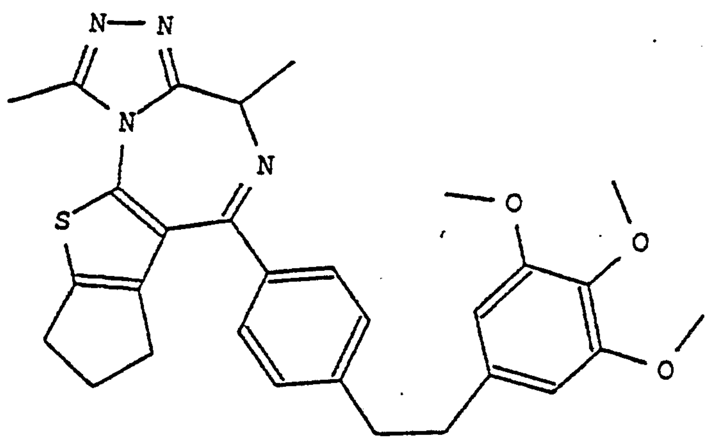

Ausgangsmaterial für die Herstellung der Titelverbindung ist das in Beispiel 4 als Zwischenprodukt synthetisierte Aminoketon.

a) Acylierung des Aminoketons (analog Schritt 8 nach 9 im Reaktionsschema)

16,1 g (37 mmol) des nach Beispiel 4 hergestellten Aminoketons werden mit 3,3 g Pyridin in 200 ml Dioxan (1,4-Dioxacyclohexan) vorgelegt und tropfenweise mit 3,9 ml 2-Chlorpropionsäurechlorid versetzt. Man rührt 2 Stunden bei Raumtemperatur nach und filtriert die Reaktionslösung anschließend über Kieselgur. Nach dem Einengen des Filtrats chromatographiert man den Rückstand an einer mit Kieselgel gefüllten Säule (Flash-Chromatographie) mit einer Mischung aus Dichlormethan und 2 % Methanol als Elutionsmittel. Nach dem Einengen des Eluats und Umkristallisieren aus Diisopropylether erhält man 18,4 (95 % d. Th.) des entsprechenden Priopionsäureamids als gelbe Kristalle vom Schmelzpunkt 138-140° C.

b) Halogenaustausch (Finkelstein-Reaktion)

18,4 g (35 mmol) des nach Verfahrensschritt a) hergestellten Säureamids werden in 290 ml Aceton gelöst und mit 10,5 g Natriumiodid versetzt und über einen Zeitraum von 2 Stunden auf Rückflußtemperatur erwärmt. Nach dem Abdestillieren des Lösungsmittels wird der Rückstand zwischen Dichlormethan und Wasser verteilt, die organische Phase wird separiert und getrocknet und nach dem Abfiltrieren des Trockenmittels i. Vak. eingeengt. Nach dem Verreiben des Rückstandes mit Diethylether erhält man 14,2 g (66 % d. Th.) des entsprechenden Jod-Derivats als Kristalle vom Schmelzpunkt 165-167° C.

c) Herstellung der Aminoverbindung (entsprechend Schritt 9 nach 10)

3,9 g (37 mmol) der nach Verfahrensschritt b) hergestellten Jodverbindung werden in 50 ml Essigsäureethylester gelöst und über einen Zeitraum von 2 Stunden mit gasformigem Ammoniak behandelt und anschließend 8 Tage lang bei Raumtemperatur gerührt. Nach dem Abdestillieren des Lösungsmittels i. Vak. erhält man 4,0 g der entsprechenden Aminoverbindung als Rohprodukt, welche ohne weitere Reinigungsschritte im anschließenden Reaktionsschritt cyclisiert wird.

d) Herstellung des Methyldiazepinon-Derivats (Cyclisierung entsprechend Schritt 10 nach 11 im Reaktionsschema)

4,0 g (ca. 6 mmol) des aus Verfahrensschritt c) resultierenden Rohproduktes werden in 100 ml Toluol in Gegenwart von 20 g Kieselgel über einen Zeitraum von 2 Stunden am Wasserabscheider erhitzt. Nach dem Abdekantieren des Toluols wird das Reaktionsprodukt durch dreimaliges Auskochen des Kieselgels mit Methanol extrahiert. Nach dem Abdestillieren des Extraktionsmittels i. Vak. wird der feste Rückstand mit Essigsäureethylester verrieben, wonach man 1,8 g (62 % d. Th.) des entsprechenden Diazepinon-Derivats in Form gelb-brauner Kristalle vom Schmp.: 265 °C erhält.

Die weiteren Umsetzungen zur Herstellung der Titelverbindung werden in Analogie zu Beispiel 19 durchgeführt.

e) Herstellung des Diazepinthion-Derivats (entsprechend Schritt 11 nach 12 im Reaktionsschema)

1,0 g (2 mmol) des nach Verfahrensschritt d) hergestellten Methyldiazepinon-Derivats werden in 15 ml Pyridin gelöst und mit 0,5 g Phosphorpentasulfid versetzt. Die Reaktionsmischung wird über einen Zeitraum von zwei Stunden auf 60 °C erhitzt. Anschließend wird das Gemisch mit Wasser verdünnt und mit Dichlormethan extrahiert. Nach dem Entfernen des Extraktionsmittels i. Vak. erhält man 0,5 g (48 % d. Th.) des entsprechenden Diazepinthion-Derivates als rotbraunes Öl.

f) Herstellung der Titelverbindung (entsprechend Schritt 12 nach 13 im Reaktionsschema)

0,5 g (1 mmol) des nach Verfahrensschritt e) hergestellten Diazepinthions werden in 5 ml Tetrahydrofuran vorgelegt und mit 0,1 ml Hydrazin-Hydrat versetzt und über einen Zeitraum von 1 Stunde bei Raumtemperatur gerührt. Nach dem Einengen der Reaktionslösung i. Vak. wird der Rückstand in 10 ml Ethanol aufgenommen und mit 0,4 g Orthoessigsäuretriethylester versetzt und für einen Zeitraum von 3 Stunden auf Rückflußtemperatur erhitzt. Nach dem Entfernen des Lösungsmittels i. Vak. erhält man 0,1 g (19 % d. Th.) der Titelverbindung als Schaum.

Beispiel 34

6-(4-Trifluormethylphenethyl)-phenyl-1,11-dimethyl-2,3,4,5-tetrahydro-8-H-pyrido[4′,3′:4,5]thieno[3,2-f]-triazolo[4,3-a][1,4]diazepin

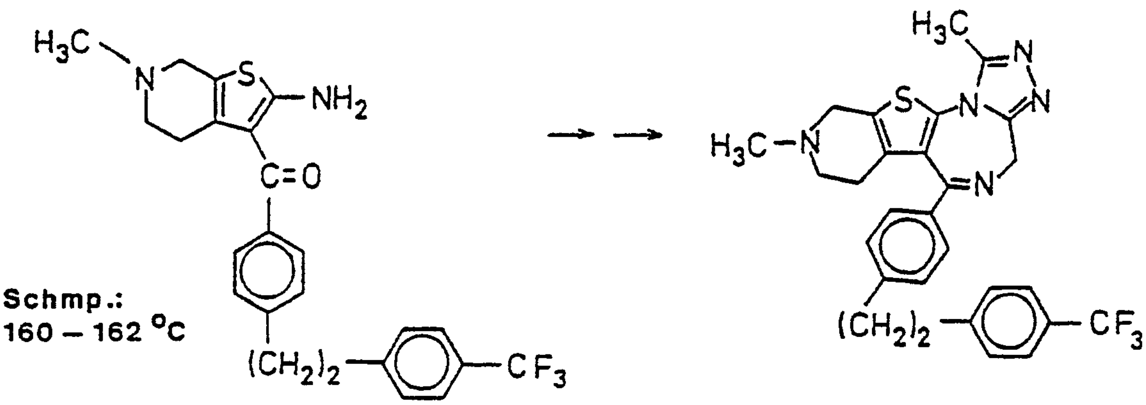

**Schmp.: 160 – 162 °C**

a) Herstellung des Aminoketons (vom Typ 8) 5,0 g (44 mmol) N-Methylpiderin-4-on, 13,9 g (44 mmol) 1-(p-Cyanomethylcarbonylphenyl)-2-(p-trifluormethylphenyl)ethan, 1,4 g (44 mmol) Schwefel und 8,9 g (88 mmol) Triethylamin werden in 200 ml Ethanol vorgelegt und über einen Zeitraum von 5 Stunden bei einer Temperatur von 60° C gerührt. Anschließend wird die Reaktionslösung i. Vak. eingeengt und der Rückstand mit Dichlormethan aufgenommen, zweimal mit Wasser gewaschen, mit Natriumsulfat getrocknet und über Kieselgel/Aktivkohle abgesaugt. Das Filtrat wird eingeengt und der verbliebene Rückstand an Kieselgel mit einer Mischung aus Dichlormethan und ca. 2 bis 4 % Methanol adsorptiv filtriert. Das Eluat wird eingeengt und der Rückstand mit Diethylether verrieben. Man erhält 8,4 g (43 % d. Th.) des Aminoketons in Form gelb-beiger Kristalle.

b) Herstellung der Bromacetylverbindung (entsprechend Schritt 8 nach 9 im Reaktionsschema)

2,0 g (4 mmol) des nach Verfahrensschritt a) erhaltenen Aminoketons werden in 45 ml Toluol gelöst und mit 4,0 g (20 mmol) Bromacetylbromid versetzt und über einen Zeitraum von 2 Stunden auf Rückflußtemperatur erhitzt. Anschließend wird die Reaktionslösung abgekühlt. Die dabei ausfallenden Kristalle werden mit Toluol gewaschen und trocken gesaugt (Schmp.: 175-177° C). Das Reaktionsprodukt ohne weitere Reinigungsschritte in der nächsten Reaktionsstufe eingesetzt.

c) Herstellung der Aminoverbindung (entsprechend Schritt 9 nach 10 im Reaktionsschema)

Die gesamte in Verfahrensschritt b) erhaltene Menge des Bromacetylamids wird in 200 ml Essigsäureethylester vorgelegt. In diese Lösung wird über einen Zeitraum von 4 Stunden gasförmiger Ammoniak eingeleitet. Das Reaktionsgemisch wird weitere drei Stunden bei Raumtemperatur nachgerührt. Anschließend wird die Reaktionslösung zweimal mit Wasser gewaschen, die organische Phase getrocknet und nach dem Entfernen des Trockenmittels i. Vak. eingeengt. Man erhält 1,6 g der Aminoverbindung als braunen schaumigen Rückstand.

d) Herstellung des Diazepinon-Derivats (entsprechend Schritt 10 nach 11 im Reaktionsschema)

1,6 g der nach Verfahrensschritt c) hergestellten Aminoverbindung werden in 100 ml Toluol in Gegenwart von 18 g Kieselgel über einen Zeitraum von 6 Stunden am Wasserabscheider auf Rückflußtemperatur erhitzt. Anschließend wird das Toluol abdekantiert, das Kieselgel wird dreimal mit Methanol extrahiert. Die vereinigten Extrakte werden über Kieselgel/Aktivkohle abgesaugt und i. Vak. eingeengt.

Der verbliebene Rückstand wird an Kieselgel mit einer Mischung aus Dichlormethan und ca. 2-6 % Methanol adsorptiv filtriert. Man erhält 0,9 g des Diazepinon-Derivates in Form eines braunen Schaumes.

e) Herstellung des Diazepinthions (entsprechend Schritt 11 nach 12 im Reaktionsschema)

2,4 g (5 mmol) des nach Verfahensschritt d) hergestellten Diazepinons werden in 20 ml Pyridin gelöst und mit 1,7 g Phosphorpentasulfid versetzt. Analoge Reaktionsführung und Aufarbeitung - wie in Beispiel 19

beschrieben - liefert 0,8 (32 % d. Th.) des Diazepinthion-Derivats als schaumiges Öl.

f) Herstellung der Titelverbindung via Hydrazon (entsprechend Schritt 12 via 13 nach 14 im Reaktionsschema)

0,8 g (1,6 mmol) des nach Verfahrensschritt e) hergestellten Diazepinthions werden in 10 ml Tetrahydrofuran gelöst und mit 0,1 ml Hydrazin-Hydrat versetzt. Reaktionsführung und Aufarbeitung gemäß Beispiel 1 führt zu 0,8 g des entsprechenden Hydrazons, die anschließend in 10 ml Ethanol gelöst wird und mit 1,0 g Orthoessigsäuretriethylester zur Titelverbindung umgesetzt werden. Zur Aufarbeitung der Reaktionsmischung wird die Lösung eingedampft und der verbliebene Rückstand in einer Mischung aus 20 ml Wasser und 3 ml 2N Salzsäure aufgenommen. Die Mischung wird mit Dichlormethan gewaschen.

Anschließend wird die wässerige Phase leicht alkalisch gestellt. Die bei einem pH-Wert von ca. 7 bis 8 ausfallende Titelverbindung, wird mit Dichlormethan extrahiert. Die vereinigten organischen Extrakte werden getrocknet, über Kieselgel/Aktivkohle abgesaugt und i.Vak. eingeengt. Man erhält auf diese Weise 0,5 g (60 % d. Th.) der Titelverbindung als Schaum.

Beispiel 35

6-(4-Thienylethylphenyl)-8,9-dihydro-1-methyl-4H,7H-cyclopenta[4,5]thieno[3,2-f][1,2,4]triazolo[4,3-a][1,4]-diazepin

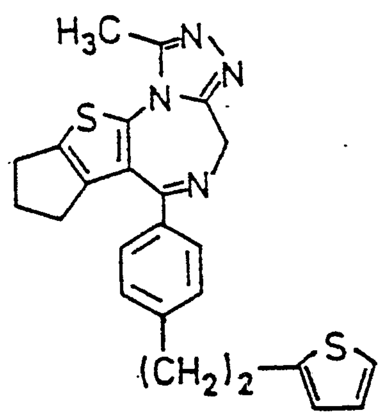

Die Synthesedurchführung zur Herstellung der Titelverbindung erfolgt in Analogie zu dem in Beispiel 1 bzw. Beispiel 19 beschriebenen Verfahren mit der Ausnahme, daß bei der Wittig-Olefinierung (im Reaktionsschema Schritt 4 nach 5) anstelle von p-Trifluormethylbenzaldehyd Thiophen-2-aldehyd eingesetzt wird.

a) Wittig-Olefinierung (entsprechend Schritt 4 nach 5 im Reaktionsschema)

12,5 g einer Natriumhydrid-Dispersion (55 %-ig) werden unter Inertgas in 150 ml absolutem Dimethylsulfoxid vorgelegt und gemäß Beispiel 1 mit 140,8 g (287 mmol) des entsprechenden Phosphoniumsalzes - suspendiert in 300 ml Dimethylsulfoxid - und darauf mit 27,3 ml (300 mmol) Thiophen-2-aldehyd versetzt. Die Aufarbeitung gemäß Beispiel 1 liefert 16,7 g (23 % d. Th.) des entsprechenden Olefins in der trans-Konfiguration (Isomerentrennung s. Beispiel 19)

b) Reduktion des Olefins (entsprechend Schritt 5 nach 6 im Reaktionsschema)

Analog Beispiel 1 werden 13 g (53 mmol) des nach Verfahrensschritt a) hergestellten trans-Olefins in 200 ml Tetrahydrofuran in Gegenwart von 2,6 g Palladium auf Kohle (10 % Pd-Gehalt) als Katalysator bei 20 °C und 5 bar Wasserstoffdruck über einen Zeitraum von 2,5 Stunden lang hydriert. Man erhält 12,0 g (92

% d. Th.) des hydrierten Produktes als Öl.

c) Herstellung des Cyanoketons (entsprechend Schritt 6 nach 7 im Reaktionsschema)

13,9 g (56,4 mmol) des nach Verfahrensschritt b) hergestellten Diphenylethanderivats werden in 56 ml Toluol gelöst und mit 2,5 g Natriumhydrid-Dispersion (55 %-ig) und 7,3 ml Acetonitril versetzt. Nach der Reaktionsdurchführung analog Beispiel 1 erhält man 8,3 g (58 % d. Th.) des entsprechenden Cyanoketons vom Schmp.: 97-105 °C.

d) Herstellung des Aminoketons (entsprechend Schritt 7 nach 8 im Reaktionsschema)

18,4 g (72 mmol) des nach Verfahrensschritt c) hergestellten Cyanoketons werden mit 5,7 g Cyclopentanon und 2,6 g Schwefel in Dimethylformamid vermischt sowie mit 5,7 ml Triethylamin in 140 ml Ethanol versetzt und 7 Stunden bei 60 °C gerührt. Das Reaktionsgemisch wird anschließend - in Analogie zu Beispiel 19 - mit 1,2-Dichlorethan verdünnt und zweimal mit Wasser gewaschen. Die organische Phase wird i. Vak. eingeengt und an einer mit Siliciumdioxyd gefüllten Säule mit Dichlormethan als Elutionsmittel chromatographiert. Man erhält nach dem Umkristallisieren aus Diisopropylether 6,5 g (26 % d. Th.) des Aminoketons (vom Typ 8), als Kristalle vom Schmp.: 110-113 °C.

e) Herstellung des Bromacetylamids (entsprechend Schritt 8 nach 9 im Reaktionsschema)

6,5 g (18 mmol) des nach Verfahrensschritt d) hergestellten Aminoketons werden in 90 ml Dichlormethan gelöst und mit 1,5 ml Pyridin versetzt. Danach werden 1,6 ml Bromacetylbromid zugetropft. Nach der weiteren Reaktionsdurchführung - analog Beispiel 19 - und Umkristallisieren aus Diisopropylether erhält man 7,0 g (80 % d. Th.) des entsprechenden Bromacetylamids als Öl.

f) Herstellung des Amino-Derivats (entsprechend Schritt 9 nach 10 im Reaktionsschema)

7,0 g (15 mmol) der nach Verfahrensschritt e) hergestellten Bromacetylverbindung werden in 140 ml Essigsäureethylester gelöst und analog Beispiel 1 mit gasförmigem Ammoniak behandelt. Nach der weiteren Reaktionsdurchführung gemäß Beispiel 1 erhält man 6,0 g (99 % d. Th.) der entsprechenden Aminoverbindung vom Typ 10, welche ohne weitere Reinigungsschritte im anschließenden Reaktionsschritt cyclsiert wird.

g) Herstellung des Diazepinon-Derivats (entsprechend Schritt 10 nach 11 im Reaktionsschema)

6,0 g (15 mmol) der nach Verfahrensschritt f) hergestellten Aminoverbindung werden in 100 ml Toluol in Gegenwart von 30 g Kieselgel cyclisiert. Die weitere Reaktionsdurchführung und Aufarbeitung gemäß Beispiel 1 führt zu 3,7 g (63. % d. Th.) des entsprechenden Diazepinons vom Typ 11 in Form von Kristallen vom Schmp.: 200-202 °C.

h) Herstellung des Diazepinthion-Derivates (entsprechend Schritt 11 nach 12 im Reaktionsschema)

3,7 g (9,4 mmol) des nach Verfahrensschritt g) hergestellten Diazepinon-Derivats werden in 80 ml Diethylenglycoldimethylether und mit 5,5 g Phosphorpentasulfid sowie mit 1,7 g Natriumhydrogencarbonat versetzt. Die Reaktionsmischung wird - wie in Beispiel 1 beschrieben - behandelt und aufgearbeitet. Nach dem Entfernen des Extraktionsmittels i. Vak. erhält man 3,8 g (99 % d. Th.) des entsprechenden Diazpinthion-Derivates als Kristalle vom Schmp.: 217 °C (Zersetzung).

i) Herstellung der Hydrazono-Verbindung vom Typ 13 (entsprechend Schritt 12 nach 13 im Reaktionsschema)

3,8 g (9 mmol) des nach Verfahrensschritt h) hergestellten Diazepinthions werden in 60 ml Tetrahydrofuran vorgelegt und mit 0,6 ml Hydrazin-Hydrat versetzt. Nach Durchführung der Umsetzung gemäß Beispiel 1 und Umkristallisieren aus Diethylether erhält man 2,6 g (69 % d. Th.) des entsprechenden Hydrazons vom Typ 13 als Kristalle vom Schmp.: 200° C (Zersetzung).

j) Herstellung der Titelverbindung (entsprechend Schritt 13 nach 14 im Reaktionsschema)

2,6 g (6,3 mmol) des nach Verfahrensschritt i) hergestellten Hydrazons werden in 30 ml Ethanol suspendiert und mit 2 ml Orthoessigsäuretriethylester zur Titelverbindung umgesetzt. Man erhält nach dem Umkristallisieren aus Essigsäureethylester 1,5 g (54 % d. Th.) der Titelverbindung in Form von Kristallen vom Schmp.: 200° C.

Beispiel 36

6-(4-(4-Chlor-N-methylamino)methylphenyl)-8,9-dihydro-1-methyl-4H,7H-cyclopenta[4,5]thieno[3,2-f][1,2,4]-triazolo[4,3-a][1,4]diazepin

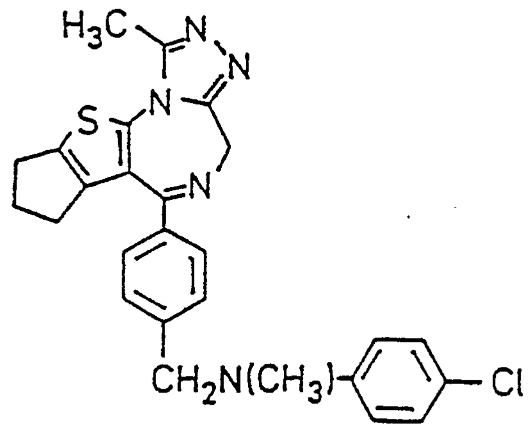

a) Der nach Beispiel 7 hergestellte Alkohol wird - wie in Beispiel 23 - beschrieben in das entsprechende Chlorid überführt.

b) 0,96 g (2,6 mmol) des nach Verfahrensschritt a) erhaltenen Chlorids werden in 30 ml Dioxan (1,4-Dioxacyclohexan) gelöst und mit 0,8 g 4-Chlor-N-methylanilin versetzt und über einen Zeitraum von 5 Stunden auf Rückflußtemperatur erhitzt. Nach dem Abdestillieren des Reaktionsmediums i. Vak. wird der Rückstand in einer Mischung aus Wasser und Essigsäureethylester aufgenommen, die organische Phase wird separiert, getrocknet und nach dem Abfiltrieren des Trockenmittels i. Vak. eingeengt. Der verbliebene Rückstand wird mittels "Flash-Chromatographie" an einer mit Kieselgel gefüllten Säule gereinigt, wobei als Elutionsmittel eine Mischung aus Dichlormethan und 4 % Methanol dient. Man erhält auf diese Weise 280 mg (23 % d. Th.) der Titelverbindung als Schaum.

Beispiel 37

6-(4-(2-Cyclohexylethyl)phenyl)-8,9-dihydro-1-methyl-4H,7H-cyclopenta[4,5]thieno[3,2-f][1,2,4]triazolo[4,3-a]-[1,4]diazepin

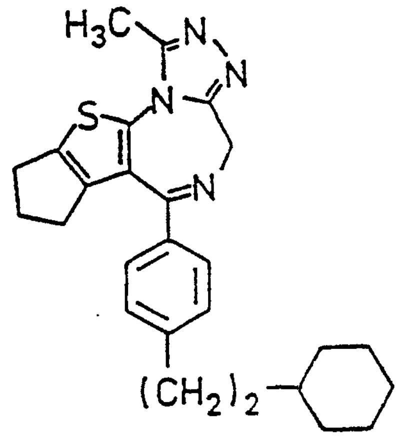

Die Titelverbindung wird in Analogie zu Beispiel 1 hergestellt, mit dem Unterschied, daß anstelle von p-Trifluormethylbenzaldehyd Cyclohexanaldehyd bei der sog. "Wittig-Olefinierung" eingesetzt wird. Die Titelverbindung fällt in Form von Kristallen vom Schmp.: 198-200°C an.

In Analogie zu den beschriebenen Verfahren lassen sich folgende Verbindungen herstellen:

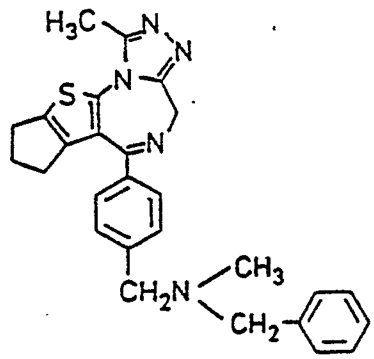

6-4(N-Benzyl-N-methyl-aminomethyl)-phenyl-8,9-dihydro-1-methyl-4H,7H-cyclopenta[4,5]thieno[3,2-f][1,2,4]-triazolo[4,3-a]-[1,4]diazepin
Schmp.: 175 - 178°C

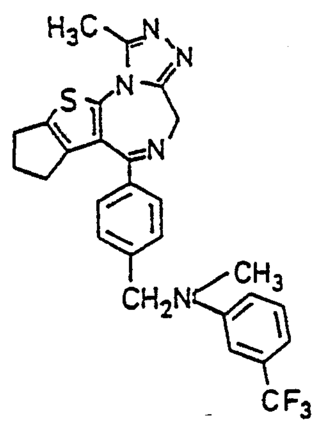

6-(4-(3-Trifluormethylphenyl)-N-methyl-aminomethylen)-phenyl)-8,9-dihydro-1-methyl-4H,7H-cyclopenta[4,5]-thieno[3,2-f]-[1,2,4]triazolo[4,3-a][1,4]diazepin.

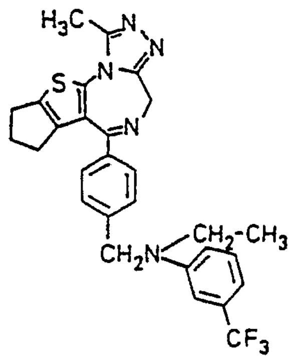

6-(4-(3-Trifluormethylphenyl-N-ethyl-aminomethylen)-phenyl-8,9-dihydro-1-methyl-4H,7H-cyclopenta[4,5]-thieno[3,2-f][1,2,4]triazolo[4,3-a][1,4]diazepin
Schmp.: 114-116 °C

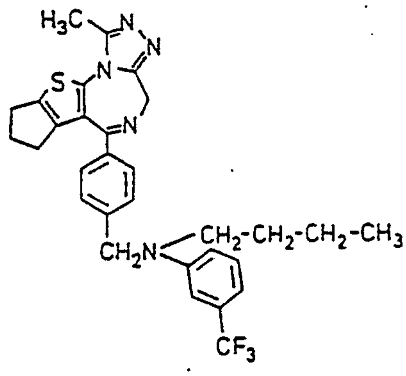

6-(4-[(3-Trifluormethylphenyl)-N-butyl-aminomethylen]-phenyl- 8,9-dihydro-1-methyl-4H,7H-cyclopenta[4,5]-thieno[3,2-f]- [1,2,4]-triazolo[4,3-a][1,4]diazepin,
Schmp.: 147 - 148 °C

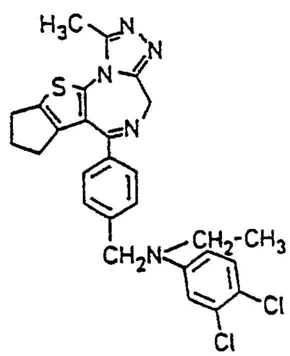

6-(4-[3,4-Dichlorphenyl-N-ethyl-aminomethylen)-phenyl-8,9-dihydro-1-methyl-4H,7H-cyclopenta[4,5]thieno-[3,2-f][1,2,4]-triazolo[4,3-a][1,4]diazepin
Schmp.: 107 - 110 °C
6-(4-(N-Benzyl-N-ethylamino)-phenyl)8,9-dihydro-1-methyl-4H,7H-cyclopenta[4,5]thieno[3,2-f][1,2,4]triazolo-[4,3-a][1,4]-diazepin,
6-(4-[4-Cyclopropylphenyl)-N-butyl-aminomethylen)phenyl-8,9-dihydro-1-methyl-4H,7H-cyclopenta[4,5]-thieno[3,2-f][1,2,4]-triazolo[4,3-a][1,4]diazepin

Die neuen Verbindungen der allgemeinen Formel Ia können warmblütigen Tieren topisch, oral, parenteral transdermal oder durch Inhalation verabreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffes bestehen, wie z.B.

Tabletten, Dragees, Kapseln, Oblaten, Pulver, Lösungen, Suspensionen, Inhalationsaerosole, Salben, Emulsionen, Sirupe, Suppositorien usw.. Eine wirksame Dosis der erfindungsgemäßen Verbindungen liegt bei oraler Anwendung zwischen 1 und 50, vorzugsweise zwischen 3 und 20 mg/Dosis, bei intravenöser oder intramuskulärer Anwendung zwischen 0,01 und 50, vorzugsweise zwischen 0,1 und 10 mg/Dosis. Für die Inhalation sollen Lösungen, die 0,01 bis 1,0, vorzugsweise 0,1 bis 0,5 % Wirkstoff enthalten, eingesetzt werden.

Die folgenden Beispiele dienen zur näheren Erläuterung der Erfindung:

Beispiel 1

Tabletten, enthaltend 10 mg Substanz der Formel I

| Zusammensetzung: | |
|---|---|
| Substanz der Formel I | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45 °C getrocknet. Das trockne Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.
Tablettengewicht: 120 mg

Beispiel 2

Dragées, enthaltend 5 mg Substanz der Formel I

| Zusammensetzung: | |
|---|---|
| Substanz B der Formel I | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45 °C

und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.
Dragéegewicht: 130 mg

Beispiel 3

Tabletten, enthaltend 50 mg Substanz der Formel I

| Zusammensetzung: | |
|---|---|
| Substanz B der Formel I | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Die Substanz, Calciumphosphat, Milchzucker und Maisstärke werden mit einer wässerigen Polyvinylpyrrolidonlösung gleichmäßig befeuchtet. Die Masse wird durch ein Sieb mit 2 mm Maschenweite gegeben, im Umlufttrockenschrank bei 50° C getrocknet und erneut gesiebt. Nach Zumischen des Schmiermittels wird das Granulat auf einer Tablettiermaschine gepreßt.

Beispiel 4

Kapseln, enthaltend 50 mg Substanz der Formel I

| Zusammensetzung: | |
|---|---|
| Substanz der Formel I | 50,0 mg |
| Maisstärke | 268,5 mg |
| Magnesiumstearat | 1,5 mg |
| | 320,0 mg |

Herstellung:

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

Beispiel 5

Suppositorien, enthaltend 50 mg Substanz der Formel I

| Zusammensetzung: | |
|---|---|
| Substanz der Formel I | 50 mg |
| Adeps solidus | 1.650 mg |
| | 1.700 mg |

Herstellung:

Das Hartfett wird geschmolzen. Bei 40° C wird die gemahlene Wirksubstanz homogen dispergiert. Es wird auf 38° C abgekühlt und in schwach vorgekühlte Suppositorienformen ausgegossen.

Beispiel 6

Orale Suspension, enthaltend 50 mg Substanz der Formel I pro 5 ml

| Zusammensetzung: | |
|---|---|
| Substanz der Formel I | 50 mg |
| Hydroxyethylcellulose | 50 mg |
| Sorbinsäue | 5 mg |
| Sorbit 70%ig | 600 mg |
| Glycerin | 200 mg |
| Aroma | 15 mg |
| Wasser ad | 5 ml |

Herstellung:

Destilliertes Wasser wird auf 70° C erhitzt. Hierin wird unter Rühren Hydroxyethylcellulose gelöst. Nach Zugabe von Sorbitlösung und Glycerin wird auf Raumtemperatur abgekühlt. Bei Raumtemperatur werden Sorbinsäure, Aroma und Substanz zugegeben. Zur Entlüftung der Suspension wird unter Rühren evakuiert.

Beispiel 7

Ampullen, enthaltend 1 mg Substanz der Formel I pro 5 ml

| Zusammensetzung: | |
|---|---|
| Substanz der Formel I | 1 mg |
| Natriumchlorid | 45 mg |
| Wasser für Injektionszwecke ad | 5 ml |

Herstellung:

Die Substanz wird bei Eigen-pH in Wasser gelöst und Natriumchlorid als Isotenz zugegeben. Die erhaltene Lösung wird pyrogenfrei filtriert und das Filtrat unter aseptischen Bedingungen in Ampullen abgefüllt, die anschließend sterilisiert und zugeschmolzen werden.

Beispiel 8

Ampullen, enthaltend 5 mg Substanz der Formel I pro 5 ml

| Zusammensetzung: | |
| --- | --- |
| Substanz der Formel I | 5 mg |
| Natriumchlorid | 45 mg |
| Wasser für Injektionszwecke ad | 5 ml |

Herstellung analog Beispiel 6.

Beispiel 9

Ampullen, enthaltend 10 mg Substanz der Formel I pro 5 ml

| Zusammensetzung: | |
| --- | --- |
| Substanz der Formel I | 10 mg |
| Methylglucamin | 35 mg |
| Glucofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und die Substanz unter Rühren in Lösung gebracht. Nach Zugabe der Lösungsmittel wird mit Wasser auf das Nennvolumen aufgefüllt.

Beispiel 10

Ampullen, enthaltend 20 mg Substanz der Formel I pro 10 ml

| Zusammensetzung: | |
|---|---|
| Substanz der Formel I | 20,000 mg |
| Natriumchlorid | 85,000 mg |
| Natriumdihydrogenphosphat x 2 $H_2O$ | 0,925 mg |
| Dinatriumhydrogenphosphat x 2 $H_2O$ | 1,320 mg |
| 0,1 N NaOH | ad pH 6 |
| Wasser für Injektionszwecke | ad 10 ml |

Herstellung:

Die obigen Bestandteile werden in Wasser gelöst und nach Filtration wird die Lösung in sterilisierte braune Ampullen abgefüllt.

Beispiel 11

Inhalationslösung, enthaltend 5 mg Substanz der Formel I pro 1 ml

| Zusammensetzung: | |
|---|---|
| Substanz der Formel I | 500 mg |
| Na-EDTA | 50 mg |
| Benzalkoniumchlorid | 25 mg |
| Natriumchlorid | 880 mg |
| Destilliertes Wasser ad | 100 mg |

Herstellung:

60 % der Wassermenge werden vorgelegt, darin nacheinander Na-EDTA, Benzalkoniumchlorid, Natriumchlorid und Substanz klar gelöst und mit dem restlichen Wasser aufgefüllt. Die Lösung wird in 20 ml Tropfflaschen abgefüllt.

**Ansprüche**

1. Neue Thienodiazepine der allgemeinen Formel

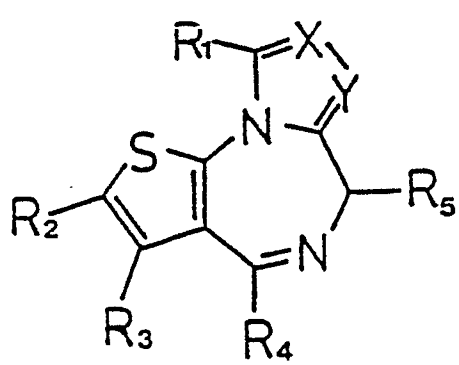

worin

$R_1$ Wasserstoff, eine verzweigte oder unverzweigte Alkylgruppe mit 1 bis 4 Kohlenstoffatom(en), bevorzugt Methyl, die gegebenenfalls durch Hydroxy oder Halogen substituiert sein kann, eine Cyclopropylgruppe, eine Cyclobutylgruppe, eine Cyclopentylgruppe, eine verzweigte oder unverzweigte Alkoxygruppe mit 1 bis 4 Kohlenstoffatomen, bevorzugt Methoxy, Halogen, bevorzugt Chlor oder Brom;

$R_2$ den Rest - G - $R_a$

worin

für n > O

$R_a$ Wasserstoff, Halogen, Hydroxy,

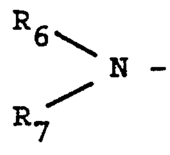 ,

wobei $R_6$ und $R_7$, die gleich oder verschieden sein können, Wasserstoff, Phenyl, substituiertes Phenyl, eine verzweigte oder unverzweigte Alkyl-, Alkenyl- oder Alkinylgruppe mit 1 - 10 Kohlenstoffatom(en), bevorzugt 1 - 4 Kohlenstoffatom(en), die gegebenenfalls durch Halogen, Hydroxy, Phenyl, substituiertes Phenyl, oder durch einen C-verknüpften Heterocyclus substituiert sein kann, wobei die Kohlenstoffkette durch Stickstoff (auch NH), Sauerstoff oder Schwefel unterbrochen sein kann, eine verzweigte oder unverzweigte Alkylcarbonylgruppe mit 1-6 Kohlenstoffatom(en), [gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann], eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl,

eine Alkylsulfonylgruppe mit 1 bis 4 Kohlenstoffatomen, einen gegebenenfalls substituierten $C_3$ bis $C_7$ Cycloalkylrest, einen gegebenenfalls substituierten $C_5$ -$C_7$-Cycloalkenylrest,

oder

$R_6$ oder $R_7$ ein gegebenenfalls ein- oder mehrfach durch verzweigtes oder unverzweigtes Alkyl mit 1 bis 4 Kohlenstoffatom(en) substituierter, gesättigter oder ungesättigter über ein Kohlenstoffatom gebundener 5-, 6- oder 7-gliedriger heterocyclischer Ring, oder $R_6$ und $R_7$ bilden zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatom(en), bevorzugt Methyl, substituiert sein kann;

$R_a$ eine Arylsulfonyloxygruppe, bevorzugt Tolylsulfonyloxy oder Phenylsulfonyloxy, gegebenenfalls ein- oder mehrfach durch verzweigte oder unverzweigte Alkyl- und/oder Alkoxygruppen mit 1 bis 4 Kohlenstoffatom(en) substituiert,

$R_a$ eine verzweigte oder unverzweigte Alkylsulfonyloxygruppe mit 1 bis 4 Kohlenstoffatom(en),

$R_a$ eine Arylcarbonyloxygruppe, bevorzugt Phenylcarbonyloxy, gegebenenfalls ein- oder mehrfach durch verzweigte oder unverzweigte Alkyl- und/oder Alkoxygruppen mit 1 bis 4 Kohlenstoffatom(en) substituiert, eine verzweigte oder unverzweigte Alkylcarbonyloxygruppe mit 1 bis 12, bevorzugt 1 bis 8 Kohlenstoffatom(en), wobei die Alkylkette durch Stickstoff, Sauerstoff oder Schwefel unterbrochen sein kann;

$R_a$ 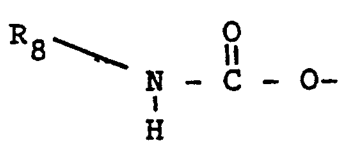 , 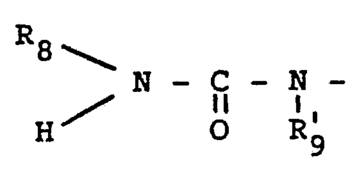

wobei $R_8$ eine verzweigte oder unverzweigte Alkyl-, Alkenyl- oder Alkinylgruppe mit 1 bis 4 Kohlenstoffatomen, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatom(en) substituierte Arylgruppe,

$R_9'$ Wasserstoff oder eine verzweigte oder unverzweigte Alkylgruppe mit 1 bis 4 Kohlenstoffatom(en),

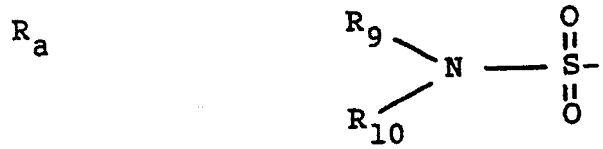

wobei $R_9$ und $R_{10}$, die gleich oder verschieden sein können, Wasserstoff, eine verzweigte oder unverzweigte Alkylgruppe mit 1 bis 10 Kohlenstoffatom(en), bevorzugt 1 bis 4 einen gegebenenfalls substituierten $C_3$ - $C_7$-Cycloalkylrest, einen gegebenenfalls substituierten $C_5$ - $C_7$-Cycloalkenylrest, oder $R_9$ und $R_{10}$ zusammen mit dem Stickstoffatom einen gegebenenfalls ein- oder mehrfach durch verzweigte oder unverzweigte Alkylgruppen mit 1 bis 4 Kohlenstoffatom(en) substituierten 5-, 6-oder 7-Ring, der als weitere Heteroatome Stickstoff, Sauerstoff oder Schwefel enthalten kann, wobei jedes weitere Stickstoffatom durch eine Alkylgruppe mit 1 bis 4 Kohlenstoffatom(en), bevorzugt Methyl, substituiert ist;
$R_a$ eine verzweigte oder unverzweigte Alkoxygruppe mit 1 bis 4 Kohlenstoffatomen;
eine Aryloxygruppe, bevorzugt Phenyloxy oder substituiertes Phenyloxy;
$R_a$ einen C-verknüpften 5 bis 7-gliedrigen nicht aromatischen Heterocyclus;
$R_a$ einen Imidorest, einen Benzimidazoylrest, ein cyclisches Amid oder Imid, das 5, 6 oder 7 Ringglieder aufweisen kann und in dem gegebenenfalls ein Kohlenstoffatom durch ein weiteres Heteroatom - wie z.B. Stickstoff, Sauerstoff oder Schwefel - ersetzt sein kann und das gegebenenfalls durch einen oder mehrere Arylrest(e) anneliert sein kann;
$R_a$ für n größer gleich 0: Wasserstoff, Halogen,
-CH = O, -CN, COOH;
$R_a$ Alkyloxycarbonylgruppe mit 1 - 10, bevorzugt 1 -4 Kohlenstoffatom(en), gegebenenfalls substituiert durch Hydroxy oder Halogen, bevorzugt Chlor, oder substituiert durch eine Aminogruppe, die gegebenenfalls ein- oder zweifach durch eine verzweigte oder unverzweigte Alkylgruppe mit 1 bis 6 Kohlenstoffatom(en) substituiert sein kann, wobei die Alkylgruppe wiederum durch Halogen oder Hydroxy substituiert sein kann;
$R_a$ gegebenenfalls substituiertes Phenyloxycarbonyl,
$R_a$ einen Rest der allgemeinen Formel

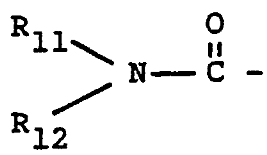,

worin $R_{11}$ und $R_{12}$, die gleich oder verschieden sein können, Wasserstoff, Phenyl, substituiertes Phenyl, eine verzweigte oder unverzweigte Alkyl-, Alkenyl- oder Alkinylgruppe mit 1 bis 10 Kohlenstoffatom(en), bevorzugt 1 bis 4 Kohlenstoffatom(en), die gegebenenfalls [durch Halogen, Hydroxy, Nitro, Amino, eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann, Morpholin, Piperazin, N-Alkylpiperazin,
oder
im Fall von $R_{11}$ = Wasserstoff oder Alkyl und Y = C-$R_1$ oder Y Stickstoff und X C-Alkyl, $R_{12}$ durch eine Estergruppe oder ein Säureamid der allgemeinen Formel

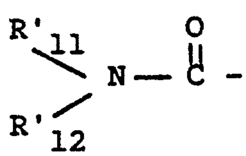

worin $R'_{11}$ und $R'_{12}$ dieselbe Bedeutung wie $R_{11}$ und $R_{12}$ - jedoch mit Ausnahme eines Säureamids haben können - substituiert sein kann],
$R_{11}$ oder $R_{12}$ ein gegebenenfalls ein- oder mehrfach durch verzweigtes oder unverzweigtes Alkyl mit 1 bis 4 Kohlenstoffatom(en) substituierter, gesättigter oder ungesättigter über ein Kohlenstoffatom gebundener 5-, 6- oder 7-gliedriger heterocyclischer Ring,

$R_{11}$ oder $R_{12}$ einen gegebenenfalls substituierten $C_3$ bis $C_7$-Cycloalkylrest, einen gegebenenfalls substituierten $C_5$ bis $C_7$-Cycloalkenylrest,

oder

$R_{11}$ und $R_{12}$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatom(en) substituierten 5-, 6-oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatom(en), bevorzugt Methyl, substituiert sein kann;

$R_a$ einen Rest der allgemeinen Formel

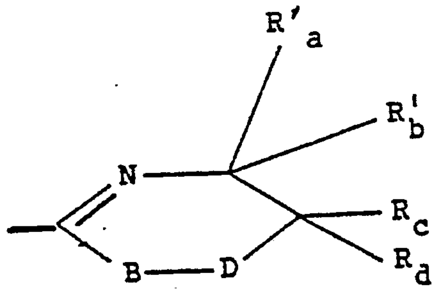

/

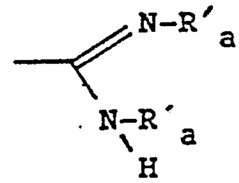

worin

B Sauerstoff, Schwefel, NH oder N-($C_1$-$C_6$-Alkyl),

D den Rest $(CR_eR_f)_n$, wobei n 0 bis 3 sein kann,

$R'_a$ Wasserstoff, gegebenenfalls durch eine Hydroxy- oder Aminogruppe substituiertes Alkyl mit 1 bis 6 Kohlenstoffatomen, $C_1$ bis $C_4$ Alkoxycarbonyl, Dialkylaminocarbonyl,

$R_b$, $R_c$, $R_d$, $R_e$, $R_f$ Wasserstoff, gegebenenfalls durch eine Hydroxy- oder Aminogruppe substituiertes Alkyl mit 1 bis 6 Kohlenstoffatomen, oder Phenyl;

$R_a$ Phenyl, oder substituiertes Phenyl;

G eine verzweigte oder unverzweigte Alkyl-, Alkenyl-oder Alkinylgruppe mit n Kohlenstoffatomen, wobei G gegebenenfalls zusätzlich durch einen Arylrest oder zusätzlich durch $R_a$ substituiert sein kann;

n eine der Zahlen 0, 1, 2, 3, 4, 5, 6, 7, 8, 9 oder 10, wobei im Falle G einen Alkenyl- oder Alkinylrest bedeutet n > 1 ist und bevorzugt die Bedeutung 2, 3 und 4 besitzt;

$R_3$ Wasserstoff, Phenyl, substituiertes Phenyl oder eine verzweigte oder unverzweigte Alkylgruppe mit 1 bis 4 Kohlenstoffatom(en), bevorzugt Methyl;

oder

$R_2$ und $R_3$ zuammen einen Rest der allgemeinen Formel

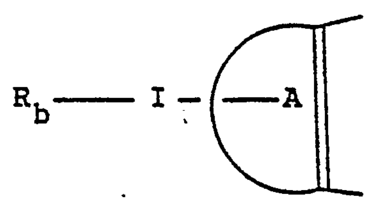

worin

A einen ankondensierten einfach ungesättigten 5-, 6-oder 7-gliedrigen Ring, wobei im Fall von m = 0 und $R_b$ = Wasserstoff ein Kohlenstoffatom durch C=0 ersetzt werden kann,

oder A einen ankondensierten Ring der Formel

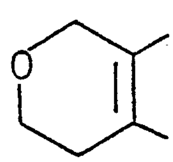

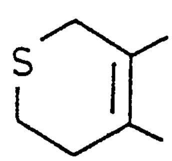

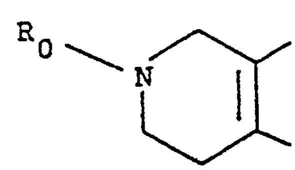

bedeutet,

wobei $R_0$ eine verzweigte oder unverzweigte Alkylgruppe mit 1 bis 18, bevorzugt mit 1 bis 4, Kohlenstoffatom(en), eine verzweigte oder unverzweigte Alkenyl- oder Alkinylgruppe mit 2 bis 18, bevorzugt 2 bis 4 Kohlenstoffatomen, eine Alkylcarbonyl- oder Alkylthiocarbonylgruppe mit 1 bis 18 bevorzugt 1 bis 4, Kohlenstoffatomen in der Alkylkette oder eine Arylcarbonyl- oder Arylthiocarbonylgruppe, eine gegebenenfalls substituierte $C_3$ bis $C_6$ Cycloalkyl- oder Cycloalkylcarbonylgruppe, eine Alkenyl- oder Alkinylcarbonylgruppe mit 2 bis 18, bevorzugt 2 bis 4 Kohlenstoffatomen, eine Alkoxycarbonylgruppe mit 1 bis 18, bevorzugt 1 bis 4 Kohlenstoffatomen, eine gegebenenfalls substituierte Phenalkyl-Phenylalkenylgruppe oder Phenalkinylgruppe mit 1 bis 6, bevorzugt 2 bis 4 Kohlenstoffatomen in der Kohlenstoffkette, eine Alkoxycarbonylalkylgruppe mit bis zu 18, bevorzugt bis zu 8 Kohlenstoffatomen, besonders bevorzugt bis zu 4, eine Alkylcarbonylalkylgruppe mit bis zu 18, bevorzugt bis zu 8 Kohlenstoffatom(en), besonders bevorzugt bis zu 4, eine Alkylcarbonylaminoalkylcarbonylgruppe mit bis zu 18, bevorzugt bis 10 Kohlenstoffatomen oder eine Aminocarbonylalkylgruppe mit bis zu 18, bevorzugt bis 4 Kohlenstoffatomen in der Alkylkette oder Wasserstoff,

$R_o$ einen Rest $R_{13}R_{14}$ NCO-$(CH_2)_{0-3}$-, einen über -$(CH_2)_{0-3}$ verknüpften 5- oder 6-gliedrigen heterocyclischen Rest, ein über -$(CH_2)$-$_{0-3}$, -CO-$(CH_2)_{0-3}$, -CO-CH=$CH_2$-, verknüpfter $C_3$-$C_6$-Cycloalkylrest, bevorzugt Cyclopropyl;

I eine verzweigte oder unverzweigte Alkyl-, Alkenyl-oder Alkinylgruppe mit m Kohlenstoffatomen;

m 0, 1, 2, 3, 4, 5 oder 6;

$R_b$ Wasserstoff, Hydroxy, Amino, Formyl, Carboxy, Cyano, verzweigtes oder unverzweigtes Alkyloxycarbonyl mit 1 bis 18, bevorzugt 8, Kohlenstoffatom(en), wobei die Alkylkette gegebenenfalls durch Hydroxy, Amino, Nitro oder Halogen substituiert sein kann, eine gegebenenfalls substituierte Aryloxycarbonylgruppe;

$R_b$ einen Rest der allgemeinen Formel

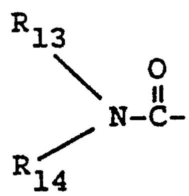

worin $R_{13}$ und $R_{14}$, die gleich oder verschieden sein können, Wasserstoff, Phenyl, substituiertes Phenyl, eine verzweigte oder unverzweigte Alkyl-, Alkenyl- oder Alkinylgruppe mit 1 bis 18 Kohlenstoffatomen, bevorzugt mit 1 bis 6, besonders bevorzugt 1 bis 4 Kohlenstoffatom(en), die gegebenenfalls durch Halogen, Hydroxy, Nitro, Amino, substituiertes Amino,$C_3$ - $C_6$-Cycloalkyl, Alkoxy, bevorzugt Methoxy oder im Fall von $R_{13}$ = Wasserstoff oder Alkyl, durch eine Esterfunktion oder durch ein Säureamid der allgemeinen Formel

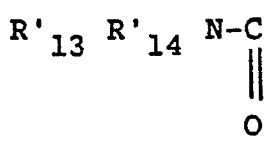

worin $R'_{13}$ und $R'_{14}$, dieselbe Bedeutung wie $R_{13}$ und $R_{14}$, jedoch mit Ausnahme eines Säureamids haben können, substituiert sein kann,

$R_{13}$ oder $R_{14}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoff verknüpfter 5-, 6-, oder 7-gliedriger heterocyclischer Ring, einen gegebenenfalls substituierten $C_3$- bis $C_7$-Cycloalkylrest, einen gegebenenfalls substituierten Cycloalkenylrest;

oder

$R_{13}$ und $R_{14}$ zusammen mit dem Stickstoffatom einen gesättigten oder ungesättigten gegebenenfalls ein- oder mehrfach durch verzweigte oder unverzweigte Alkylgruppen mit oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatom(en), bevorzugt Methyl, substituiert sein kann;

$R_b$ einen Rest der allgemeinen Formel

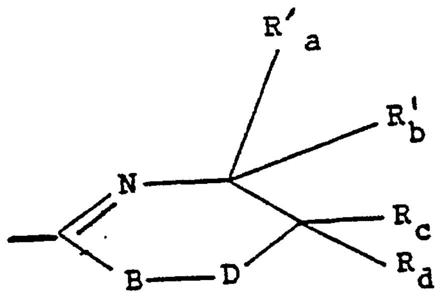 , 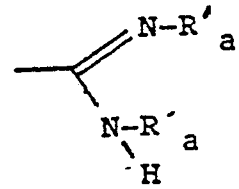

worin

B Sauerstoff, Schwefel, NH oder N-($C_1$-$C_6$-Alkyl),

D den Rest $(CR_eR_f)_n$, wobei n 0 bis 3 sein kann,

$R'_a$ Wasserstoff, gegebenenfalls durch eine Hydroxy- oder Aminogruppe substituiertes Alkyl mit 1 bis 6 Kohlenstoffatom(en), $C_1$ bis $C_4$ Alkoxycarbonyl, Dialkylaminocarbonyl,

$R_b$, $R_c$, $R_d$, $R_e$, $R_f$ Wasserstoff, gegebenenfalls durch eine Hydroxy- oder Aminogruppe substituiertes Alkyl mit 1 bis 6 Kohlenstoffatom(en), oder Phenyl,

$R_b$ 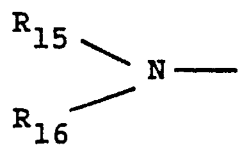

$R_{15}$ = Wasserstoff, $R_{16}$ = Wasserstoff, Alkylcarbonyl oder Alkoxycarbonyl mit 1 bis 18, bevorzugt 1 bis 6 Kohlenstoffatom(en), Aminocarbonyl, Alkylaminocarbonyl, Dialkylaminocarbonyl mit 1 bis 18 - bevorzugt 1 bis 6 Kohlenstoffatom(en) - in der Alkylkette;

$R_b$ Wasserstoff, Phenyl, substituiertes Phenyl;

$R_b$ Halogen, 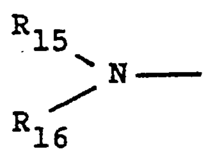 ,

wobei $R_{15}$ und $R_{16}$, die gleich oder verschieden sein können, Wasserstoff, Phenyl, substituiertes Phenyl, eine verzweigte oder unverzweigte Alkyl-, Alkenyl- oder Alkinylgruppe mit 1 - 18 Kohlenstoffatom(en), bevorzugt 1 bis 6 Kohlenstoffatom(en), die gegebenenfalls durch Halogen, Hydroxy,$C_3$ - $C_6$ Cycloalkyl oder einen C-verknüpften Heterocyclus substituiert sein kann, wobei die Kohlenstoffkette durch Stickstoff, Sauerstoff oder Schwefel unterbrochen sein kann,

eine verzweigte oder unverzweigte Alkylcarbonylgruppe mit 1-6 Kohlenstoffatom(en), gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatom(en) substituierte Aminogruppe, wobei die Alkylgruppe durch Halogen oder Hydroxy substituiert sein kann, eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatom(en),

oder

$R_{15}$ oder $R_{16}$ einen gegebenenfalls ein- oder mehrfach durch verzweigtes oder unverzweigtes Alkyl mit 1 bis 4 Kohlenstoffatom(en) substituierter, gesättigter oder ungesättigter über ein Kohlenstoffatom gebundener 5-, 6- oder 7-gliedriger heterocyclischer Ring,

$R_{15}$ oder $R_{16}$ einen gegebenenfalls substituierten $C_3$ bis $C_7$-Cycloalkylrest, einen gegebenenfalls substituierten $C_5$ bis $C_7$-Cycloalkenylrest

oder

$R_{15}$ und $R_{16}$ bilden zusammen mit dem Stickstoffatom einen gesättigten oder ungesättigten gegebenenfalls

ein- oder mehrfach durch verzweigte oder unverzweigte Alkylgruppen mit 1 bis 4 Kohlenstoffatom(en) substituierten 5-, 6- oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatom(en), bevorzugt Methyl, substituiert sein kann;

$R_b$ eine Arylsulfonyloxygruppe, bevorzugt Tolylsulfonyloxy oder Phenylsulfonyloxy, gegebenenfalls ein- oder mehrfach durch verzweigte oder unverzweigte Alkyl- und/oder Alkoxygruppen mit 1 bis 4 Kohlenstoffatom(en) substituiert;

$R_b$ eine verzweigte oder unverzweigte Alkylsulfonyloxygruppe mit 1 bis 4 Kohlenstoffatom(en);

$R_b$ eine Arylcarbonyloxygruppe, bevorzugt Tolylcarbonyloxy oder Phenylcarbonyloxy, gegebenenfalls ein oder mehrfach durch verzweigte oder unverzweigte Alkyl- und/oder Alkoxygruppen mit 1 bis 4 Kohlenstoffatom(en) substituiert;

$R_b$ eine verzweigte oder unverzweigte Alkylcarbonyloxygruppe mit 1 bis 18, bevorzugt 1 bis 8, Kohlenstoffatom(en), wobei die Alkylkette durch Stickstoff, Sauerstoff oder Schwefel unterbrochen sein kann;

$R_b$ 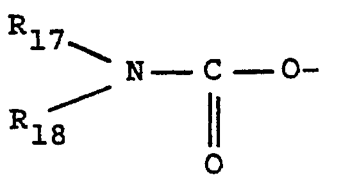 , 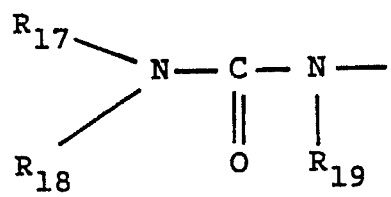

wobei $R_{17}$ Wasserstoff und $R_{18}$ eine Alkyl-, Alkenyl- oder Alkinylgruppe mit 2 bis 4 Kohlenstoffatomen, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatom(en) substituierte Arylgruppe, $R_{19}$ Wasserstoff oder eine verzweigte oder unverzweigte Alkylgruppe mit 1 bis 4 Kohlenstoffatom(en);

$R_b$ einen Imidorest,

ein cyclisches Amid oder Imid, das 5, 6 oder 7 Ringglieder aufweisen kann und in dem gegebenenfalls ein Kohlenstoffatom durch ein weiteres Heteroatom - wie z.B. Stickstoff, Sauerstoff oder Schwefel - ersetzt sein kann und das gegebenenfalls durch einen oder mehrere Arylrest(e) anneliert sein kann,

einen Benzimidazolylrest;

$R_b$ einen verzweigten oder unverzweigten Alkyloxy-bzw. Alkylthiorest mit 1 bis 18, bevorzugt 1 bis 4 Kohlenstoffatom(en), einen gegebenenfalls substituierten Phenyloxy- oder Phenylthiorest, einen über Sauerstoff oder Schwefel verknüpften, gesättigten oder ungestättigten 5- oder 6-gliedrigen heterocyclischen Ring;

$R_4$ einen Rest der allgemeinen Formel

$-S_K - W_L - T_M - V_N - U_M - P_N - Z_\alpha$

worin

S eine Einfachbindung, verzweigtes oder unverzweigtes Alkyl mit 1 bis 8 - bevorzugt 1 bis 4 - Kohlenstoffatom(en), gegebenenfalls durch Halogen und/oder Hydroxy substituiert, verzweigtes oder unverzweigtes Alkenyl mit 2 bis 8 Kohlenstoffatomen - bevorzugt mit 2 bis 4 Kohlenstoffatomen, gegebenenfalls durch Halogen und/oder Hydroxy substituiert, verzweigtes oder unverzweigtes Alkinyl mit 2 bis 8 Kohlenstoffatomen - bevorzugt mit 2 bis 4 Kohlenstoffatom(en), gegebenenfalls durch Halogen und/oder Hydroxy substituiert;

W einen gegebenenfalls substituierter aromatischer Ring mit 5 bis 10 Kohlenstoffatomen, der gegebenenfalls ein oder mehrere Heteroatom(e) aus der Gruppe Stickstoff, Sauerstoff oder Schwefel enthalten kann,

einen gegebenenfalls substituierten carbocyclischen Ring mit 3 bis 7 Kohlenstoffatomen, der auch eine Doppelbindung enthalten kann;

T verzweigtes oder unverzweigtes Alkyl gegebenenfalls substituiert durch Halogen oder Hydroxy mit 1 bis 8 - bevorzugt 1 bis 4 -Kohlenstoffatom(en); verzweigtes oder unverzweigtes Alkenyl mit 2 bis 8 Kohlenstoffatomen - bevorzugt 2 bis 4 Kohlenstoffatomen - gegebenenfalls durch Halogen und/oder Hydroxy substituiert; verzweigtes oder unverzweigtes Alkinyl mit 2 bis 8 Kohlenstoffatomen - bevorzugt 2 bis 4 Kohlenstoffatomen - gegebenenfalls durch Halogen und/oder Hydroxy substituiert - einen Rest der Formel

$- (F)_{0-1} - E - (F)_{0-1} -$

$E = O, S, N-R_9,$

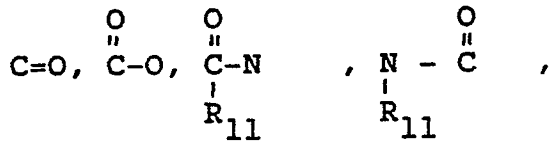

worin $R_6$ wie zuvor definiert, bevorzugt Wasserstoff, $C_1$ - $C_4$-Alkyl, oder

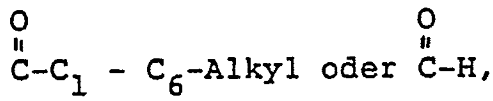

$R_{11}$ wie zuvor definiert, bevorzugt Wasserstoff oder $C_1$ - $C_4$-Alkyl; oder E bedeutet

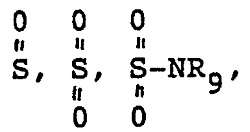

$R_9$ wie zuvor definiert ist,

F eine verzweigte oder unverzweigte gegebenenfalls substituierte Alkylgruppe mit 1 bis 8 - bevorzugt 1 bis 4 -Kohlenstoffatom(en), eine gegebenenfalls substituierte Alkenylgruppe mit 2 bis 8 -bevorzugt 2 bis 4 - Kohlenstoffatomen, eine gegebenenfalls substituierte Alkinylgruppe mit 2 bis 8 - bevorzugt 2 bis 4 Kohlenstoffatomen;

V einen gegebenenfalls substituierten aromatischen Ring mit 5 bis 10 Kohlenstoffatomen, der gegebenenfalls ein oder mehrere Heteroatome aus der Gruppe Stickstoff, Sauerstoff oder Schwefel enthalten kann, einen gegebenenfalls substituierten carbocyclischen Ring mit 3 bis 7 Kohlenstoffatomen, der auch eine Doppelbindung enthalten kann,

ein cyclisches Amid oder Imid, das 5, 6 oder 7 Ringglieder aufweisen kann und in dem gegebenenfalls ein Kohlenstoffatom durch ein weiteres Heteroatom - wie z.B. Stickstoff, Sauerstoff oder Schwefel - ersetzt sein kann und das gegebenenfalls durch einen oder mehrere Arylrest(e) anneliert sein kann;

U verzweigtes oder unverzweigtes Alkyl gegebenenfalls substituiert durch Halogen oder Hydroxy mit 1 bis 8 - bevorzugt 1 bis 4 -Kohlenstoffatom(en), verzweigtes oder unverzweigtes Alkenyl mit 2 bis 8 Kohlenstoffatomen - bevorzugt 2 bis 4 Kohlenstoffatomen - gegebenenfalls durch Halogen und/oder Hydroxy substituiert; verzweigtes oder unverzweigtes Alkinyl mit 2 bis 8 Kohlenstoffatomen - bevorzugt 2 bis 4 Kohlenstoffatomen - gegebenenfalls durch Halogen und/oder Hydroxy substituiert; einen Rest der Formel

$-(F)_{0-1} - E - (F)_{0-1} -$

E = O, S, N-$R_6$,

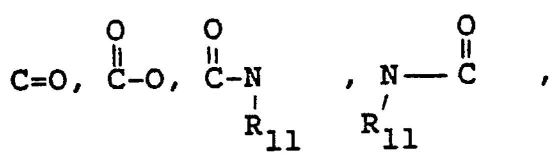

worin $R_6$ wie zuvor definiert, bevorzugt Wasserstoff, $C_1$ - $C_4$-Alkyl, oder

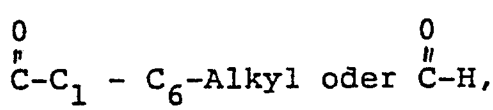

und $R_{11}$ wie zuvor defiiniert, bevorzugt Wasserstoff oder $C_1$ - $C_4$ -Alkyl; oder E bedeutet

$$\overset{O}{\overset{\|}{S}},\ \underset{\underset{O}{\|}}{\overset{O}{\overset{\|}{S}}},\ \overset{O}{\overset{\|}{S}}-NR_9,$$

worin $R_9$ wie zuvor definiert,

F eine verzweigte oder unverzweigte gegebenenfalls substituierte Alkylgruppe mit 1 bis 8 - bevorzugt 1 bis 4 - Kohlenstoffatom(en), eine gegebenenfalls substituierte Alkenylgruppe mit 2 bis 8 -bevorzugt 2 bis 4 - Kohlenstoffatomen, eine gegebenenfalls substituierte Alkinylgruppe mit 2 bis 8 - bevorzugt 2 bis 4 Kohlenstoffatomen;

P einen gegebenenfalls substituierten aromatischen Ring mit 6 bis 10 Kohlenstoffatomen, der gegebenenfalls ein oder mehrere Heteroatome aus der Gruppe Stickstoff, Sauerstoff oder Schwefel enthalten kann, einen gegebenenfalls substituierten carbocyclischen Ring mit 3 bis 7 Kohlenstoffatomen, der auch eine Doppelbindung enthalten kann, wobei W, V und/oder P ein- oder mehrfach durch Z substituiert sein kann, ein cyclisches Amid oder Imid, das 5, 6 oder 7 Ringglieder aufweisen kann und in dem gegebenenfalls ein Kohlenstoffatom durch ein weiteres Heteroatom wie z.B. Stickstoff, Sauerstoff oder Schwefel - ersetzt sein kann und das gegebenenfalls durch einen oder mehrere Arylrest(e) anneliert sein kann;

Z Wasserstoff, Halogen, Hydroxy, Nitro, Cyano, Trifluormethyl, gegebenenfalls durch Halogen oder Hydroxy substituiertes, verzweigtes oder unverzweigtes Alkyl mit 1 bis 8 - bevorzugt 1 bis 4 - Kohlenstoffatom(en), gegebenenfalls substituiertes, verzweigtes Alkoxy mit 1 bis 8 bevorzugt 1 bis 4 Kohlenstoffatom(en), einen Rest der Formel

$$-(F')_{0-1}-E'-(F')_{0-1}-H,\ (F')_{0-1}-NO_2,\ SO_2-C_1-C_4\text{-alkyl},$$

$$-(F')_{0-1}-N\begin{matrix}\diagup R_6\\ \diagdown R_7\end{matrix}\ ,-(F')_{0-1}-\underset{\underset{O}{\|}}{C}-N\begin{matrix}\diagup R_{11}\\ \diagdown R_{12}\end{matrix}$$

$$-(F')_{0-1}-\underset{\underset{O}{\|}}{\overset{\overset{OH}{|}}{C}}$$

$$-(F')_{0-1}-\underset{\underset{O}{\|}}{C}-(C_1-C_8)\text{-Alkyl},$$

bevorzugt $C_1 - C_4$-Alkyl,

worin F' eine verzweigte oder unverzweigte Alkylgruppe mit 1 bis 4 Kohlenstoffatom(en), bevorzugt 1 bis 2 Kohlenstoffatom(en) oder F' hat dieselbe Bedeutung wie F und E' = E oder
E' = O, S, N-$R_6$,

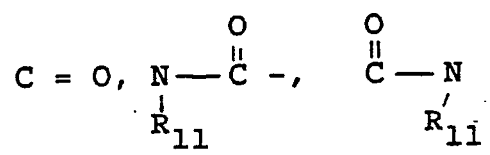

wobei bei $\alpha > 1$ alle Z gleich, teilweise gleich oder verschieden sein können,
K = 0, 1,
L = 0, 1, 2,
M = 0, 1,
N = 0, 1
und $\alpha$ = 1, 2, 3, 4, 5 bedeuten können und E die unter T angegebene Bedeutung hat, wobei die Summe K + L + M + N ungleich 0 ist und
$-S_K - W_L - T_M - V_N - U_M - P_N - Z_\alpha$
insgesamt nicht einen Phenylrest, einen Phenylrest, der mit Halogen, $CH_3$, $CF_3$ und $NO_2$ substituiert ist, einen Pyridyl- oder einen Thienylrest bedeuten kann.
$R_5$ Wasserstoff, $CF_3$, Hydroxy, verzweigtes oder unverzweigtes Alkyl mit 1 bis 6, bevorzugt 1 bis 4 Kohlenstoffatom(en), besonders bevorzugt Methyl, gegebenenfalls ist die Alkylgruppe durch Hydroxy, Halogen, $C_1$- bis $C_4$-Alkylsulfonyloxy, bevorzugt Methylsulfonyloxy,

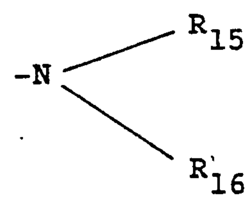

worin $R_{15}$ und $R_{16}$ die zuvor genannte Bedeutung aufweisen können, oder

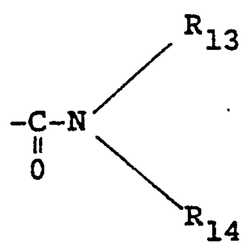

worin $R_{13}$ und $R_{14}$ die zuvor genannte Bedeutng aufweisen können, substituiert; Alkylcarbonyloxy mit 1 bis 4 Kohlenstoffatom(en) in den verzweigten oder unverzweigten Alkylkette, Alkoxycarbonyl mit 1 bis 4 Kohlenstoffatom(en) in der verzweigten oder unverzweigten Alkylkette, Carboxyalkyl mit 1 bis 4 Kohlenstoffatom(en) in der verzweigten oder unverzweigten Alkylkette, Alkoxycarbonylalkyl mit 1 bis 4 Kohlenstoffatom(en) in den verzweigten oder unverzweigten Alkylresten
X/Y unabhängig voneinander C-$R_1$, oder N, aber nicht beide C-$R_1$, mit $R_1$ bevorzugt Wasserstoff oder Methyl,
oder Y die Gruppe C-COOR′, wobei R′ Alkyl oder Wasserstoff bedeutet und X Stickstoff bedeutet; gegebenenfalls in Form ihrer Racemate, ihrer Enantiomere, ihrer Diastereomere und ihrer Gemische sowie gegebenenfalls ihre physiologisch unbedenklichen Säureadditionssalze.
2. Neue Thienodiazepine der allgemeinen Formel I nach Anspruch 1, worin
$R_2$ und $R_3$ einen Rest wie für die allgemeine Formel 1 eingangs definiert und worin
$R_1$ Ethyl, Trifluormethyl, Methoxy, Ethoxy oder Halogen, besonders bevorzugt Chlor oder Brom; besonders bevorzugt Methyl;
$R_a$ Wasserstoff, Chlor, Brom, Jod, Hydroxy, $C_1$-$C_4$-Alkoxycarbonyl, Phenyl, substituiertes Phenyl; $C_3$-$C_6$-Cycloalkyl;
wobei in $R_4$ bevorzugt
S eine Einfachbindung (K = 0), verzweigtes oder unverzweigtes $C_1$-$C_4$-Alkyl, verzweigtes oder unverzweig-

tes $C_2$-$C_4$-Alkenyl,
verzweigtes oder unverzweigtes $C_2$-$C_4$-Alkinyl,
W einen Phenylring, einen Pyridinring, einen Thiophenring - gegebenenfalls mit Halogen, einer Hydroxygruppe, einen Cyclopropylrest, einem $C_1$-$C_4$-Alkylrest, einem $C_1$-$C_4$-Alkoxyrest untereinander gleich oder verschieden ein- oder mehrfach substituiert;
T eine Alkylkette mit bis zu 6 Gliedern, bevorzugt Ethyl, eine Alkenylkette mit 2 bis 4 Gliedern bevorzugt Vinyl, eine Alkinylkette mit 2 bis 4 Gliedern bevorzugt C≡C, die gegebenenfalls durch ein Heteroatom - wie Sauerstoff, Schwefel oder Stickstoff unterbrochen sein können,

oder 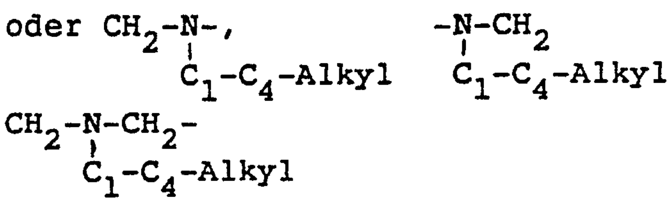

oder
den Rest 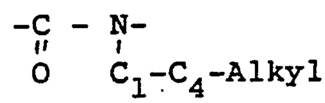 ;

V eine Phenylgruppe, eine Thienylgruppe oder eine $C_3$-$C_6$-Cycloalkylgruppe, eine substituierte Phenylgruppe,
U bevorzugt eine Einfachbindung (M = O),
oder $-CH_2-CH_2-$
wenn P eine Phenylgruppe bedeutet;
P eine Phenylgruppe; bevorzugt $P_N$ = null
Z Wasserstoff, verzweigtes oder unverzweigtes $C_1$-$C_4$-Alkyl oder $C_1$-$C_4$-Alkoxy, Trifluormethyl, Halogen - bevorzugt Chlor oder Brom, CN, $NO_2$, Cyano, COH, COOH, $COOC_1$ - $C_4$-Alkyl, $CONH_2$, $CON(C_1$ - $C_4$-$Alkyl)_2$, Cyclopropyl, Amino, $C_1$-$C_4$-Alkylamino, $C_1$-$C_4$-Dialkylamino, Hydroxymethyl, $SO_2$-$C_1$-$C_4$-Alkyl, $SO_2$-$C_1$-$C_4$-Hydroxyalkyl, Hydroxy, SH, S-$C_1$-$C_4$-Alkyl,
wobei bei $\alpha > 1$ alle Z gleich, teilweise gleich oder verschieden sein können,
K = 0, 1, bevorzugt 0
L = 0, 1, 2, bevorzugt 1
M = 0, 1, bevorzugt 1
N = 0, 1, bevorzugt 1, aber $P_N$ bevorzugt 0
und $\alpha$ = 1, 2, 3, bedeuten können, wobei die Summe K + L + M + N ungleich 0 ist und
$- S_K - W_L - T_M - V_N - U_M - P_N - Z_\alpha$
insgesamt nicht einen Phenylrest, einen Phenylrest, der mit Halogen, $CH_3$, $CF_3$ und $NO_2$ substituiert ist, einen Pyridyl- oder einen Thienylrest bedeutet.
$R_5$ Wasserstoff, Hydroxy, Methyl oder Trifluormethyl;
X,Y unabhängig voneinander C-$R_1$ oder N, aber nicht gleichzeitig beide C-$R_1$, bevorzugt beide N oder X = C-$R_1$ ($R_1$ = H, Methyl) Y = N,
G wie zuvor definiert ist,
n eine der Zahlen 1, 2, 3, 4, 5, 6, 7, 8, 9 oder 10, bevorzugt 0, 1 oder 2, bedeuten können sowie gegebenenfalls ihre physiologisch unbedenklichen Säureadditionssalze.
3. Neue Thienodiazepine der allgemeinen Formel nach Anspruch I, worin $R_2$ und $R_3$ ein Rest der allgemeinen Formel

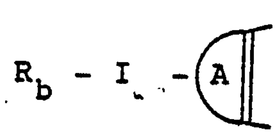

bilden, worin A ein ankondensierter einfach ungesättigter 5- oder 6-gliedriger Ring bedeutet, oder einen Rest der allgemeinen Formel

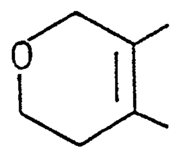

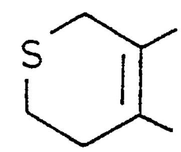

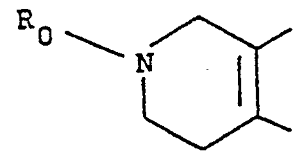

worin $R_o$ = Wasserstoff, $C_1$-$C_4$-Alkyl, $C_3$ bis $C_6$-Cycloalkyl, $C_3$ bis $C_6$-Cycloalkylcarbonyl
I eine unverzweigte Alkylgruppe mit m Kohlenstoffatomen
m O, 1, 2, 3 oder 4;
X/Y unabhängig voneinander C-$R_1$ oder N, aber nicht beide C-$R_1$, bevorzugt beide N oder X C-$R_1$ und Y N ($R_1$ = H, Methyl);
oder
Y C-COOR′, wobei R′ Wasserstoff oder Niederalkyl und X Stickstoff bedeutet;
$R_1$ Wasserstoff, Hydroxymethyl, Chlormethyl, Trifluormethyl, Cyclopropyl, Ethyl, Methoxy, Ethoxy, Chlor oder Brom, bevorzugt Methyl;
$R_b$ Wasserstoff, Hydroxy, Amino, Aminocarbonyl, Carboxy, Cyano, Alkyloxycarbonyl mit 1 bis 6 Kohlenstoffatom(en), ein Säureamid der Formel $R_{13}R_{14}NCO$, worin $R_{13}$ und $R_{14}$ ein $C_1$ bis $C_4$-Alkylrest oder $R_{13}$ und $R_{14}$ zusammen mit dem Stickstoffatom einen gegebenenfalls substituierten 6-gliedrigen Heterocyclus, der als weiteres Heteroatom Sauerstoff, Schwefel oder Stickstoff enthalten kann,
worin in $R_4$ besonders bevorzugt
S eine Einfachbindung (K = 0), eine Methylengruppe oder eine Ethylengruppe;
W einen Phenylring, einen Thiophenring, der gegebenenfalls mit Halogen, einer Hydroxygruppe, einem $C_1$-$C_4$-Alkylrest, einem $C_1$-$C_4$-Alkoxyrest, einen Cyclopropylrest, untereinander gleich oder verschieden, ein- oder mehrfach substituiert sein kann;
T eine Alkylkette mit bis zu 4 Gliedern, eine Alkenylkette mit 2 bis 4 Gliedern, eine Alkinylkette mit 2 bis 4 Gliedern, die gegebenenfalls durch ein Heteroatom wie Sauerstoff oder Schwefel unterbrochen ist;

oder $CH_2$-N-, mit N–$C_1$-$C_4$-Alkyl; -N-$CH_2$ mit N–$C_1$-$C_4$-Alkyl

$CH_2$-N-$CH_2$- mit N–$C_1$-$C_4$-Alkyl

oder

den Rest -C(=O) - N(–$C_1$-$C_4$-Alkyl)- ;

V eine Phenylgruppe; eine substituierte Phenylgruppe, $C_3$-$C_6$-Cycloalkylgruppe, eine Thienylgruppe
U bevorzugt eine Einfachbindung (M = O), oder
-$CH_2$-$CH_2$-
wenn P eine Phenygruppe bedeutet;
P eine Phenylgruppe;
Z Wasserstoff, verzweigtes oder unverzweigtes $C_1$-$C_4$-Alkyl oder $C_1$-$C_4$-Alkoxy, Trifluormethyl, Halogen - bevorzugt Chlor oder Brom, CN, $NO_2$, Cyano, COH, COOH, $COOC_1$-$C_4$-Alkyl, $CONH_2$, $CON(C_1$-$C_4$-$Alkyl)_2$, Cyclopropyl, Amino, $C_1$-$C_4$-Alkylamino, $C_1$-$C_4$-Dialkylamino, Hydroxymethyl, $SO_2$-$C_1$-$C_4$-Alkyl, $C_1$-$C_4$-Dialkylamino, Hydroxymethyl, $SO_2$-$C_1$-$C_4$-Alkyl, $SO_2$-$C_1$-$C_4$-Hydroxyalkyl, Hydroxy, SH, S-$C_1$-$C_4$-Alkyl,
und
K = 0, 1, bevorzugt O,
L = 0, 1, bevorzugt 1,
M = 0, 1, bevorzugt 1,
N = 0, 1, bevorzugt 1, aber $P_N$ bevorzugt 0

und $\alpha$ = 1, 2, 3,
bedeuten kann, wobei die Summe K + L + M + N ungleich 0 ist und
$R_5$ Wasserstoff, Hydroxy, Methyl oder Trifluormethyl bedeuten kann, sowie gegebenenfalls ihre physiologisch unbedenklichen Säureadditionssalze.
4. Pharmazeutische Zubereitungen, gekennzeichnet durch einen Gehalt an einer Verbindung nach einem der Ansprüche 1 bis 3.
5. Verwendung von Verbindungen nach einem der Ansprüche 1 bis 3 bei der Herstellung von,pharmazeutischen Zubereitungen zur Behandlung von Erkrankungen, die durch endogen gebildetes PAF induziert werden.
6. Verwendung von Verbindungen nach einem der Ansprüche 1 bis 3 als Arzneimittel.
7. Neue Diazepinthione der allgemeinen Formel

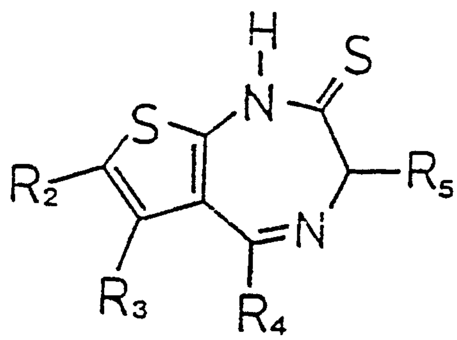

worin $R_2$, $R_3$, $R_4$ und $R_5$ wie in Anspruch 1, 2 oder 3 definiert sind.
8) Neue Zwischenverbindungen der allgemeinen Formel
$R_4$-$CH_2$-CN
worin $R_4$ wie in Anspruch 1, 2 oder 3 definiert ist,
9) Verfahren zur Herstellung von Verbindungen der allgemeinen Formel I wie in Anspruch 1, 2 oder 3 definiert, dadurch gekennzeichnet, daß man eine Verbindung der allgemeinen Formel

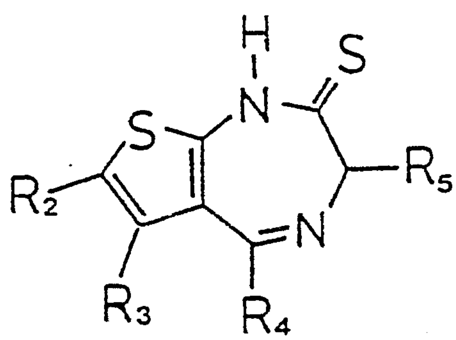

worin $R_2$, $R_3$, $R_4$ und $R_5$ wie zuvor definiert sind,
A) für den Fall, daß X und Y Stickstoff bedeutet
a) mit einem Säurehydrazid der allgemeinen Formel
$R_1$-CONHNH$_2$ III
umsetzt,
b) oder aber mit Hydrazin in eine Verbindung der allgemeinen Formel

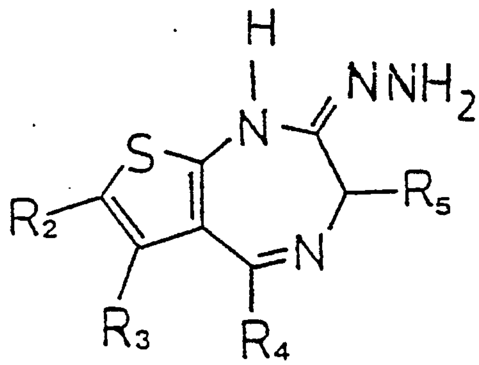

überführt und anschließend mit einem Säurehalogenid der allgemeinen Formel $R_1$-CO-Hal oder mit einem Orthoester der allgemeinen Formel $R_1$-C(OR′)$_3$ worin R′ eine niedere Alkylgruppe bedeutet, umsetzt, oder

B) Für den Fall, daß X C-H, C-Alkyl und Y Stickstoff

a) mit einem Aminoalkin der allgemeinen Formel $R_1'$ -C≡C - $CH_2$-$NH_2$ worin $R'_1$ Wasserstoff oder eine niedere Alkylgruppe bedeutet

oder aber

b) mit einem α-Aminoaldehyd-alkylacetal oder α-Aminoketon-alkylketal der allgemeinen Formel $H_2NCH_2$-$CR_1(OR')_2$

worin $R_1$ Wasserstoff oder eine Alkylgruppe mit 1 bis 4 Kohlenstoffatomen und R′ eine niedere Alkylgruppe bedeutet, umsetzt, anschließend gewünschtenfalls die erhaltenen Verbindungen mit $R_1$ = Wasserstoff in Gegenwart einer Base mit einem Halogenierungsmittel, wie z.B. mit Chlor oder Brom, zu einer Verbindung der allgemeinen Formel Ia mit $R_1$ Halogen, wie z.B. Chlor oder Brom umsetzt; anschließend gewünschtenfalls die Halogenverbindung in eine Verbindung der allgemeinen Formel Ia mit $R_1$ = Alkoxy mit 1 bis 4 Kohlenstoffatomen durch Reaktion mit dem entsprechenden Alkoholat überführt; und anschließend gegebenenfalls die so erhaltenen Verbindungen mittels an sich bekannter Trennverfahren in ihre optisch aktiven Verbindungen spaltet,

und gegebenenfalls die so erhaltenen Verbindungen in ihre unbedenklichen Säureadditionssalze überführt.

10) Analogieverfahren zur Herstellung von Verbindungen der allgemeinen Formel I, die in der Seitenkette -G-$R_a$ oder -I -$R_b$ ein Säureamid aufweisen, dadurch gekennzeichnet, daß man eine Verbindung der allgemeinen Formel I, worin $R_a$ oder $R_b$ den Rest COOH bedeuten, mit einem Amin der Formel $HNR_{11}R_{12}$ oder $HNR_{13}R_{14}$ in Gegenwart von Sulfonyldiimidazol oder Carbonyldiimidazol umsetzt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 90113130.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 3 724 031 (BOEHRINGER INGELHEIM) * Ansprüche 1,3,4,8-10; Seite 21, Syntheseschema * -- | 1-5,7-10 | C 07 D 495/14<br>C 07 D 495/04<br>C 07 C 255/40<br>A 61 K 31/55<br>//(C 07 D 495/14<br>C 07 D 333:00<br>C 07 D 243:00<br>C 07 D 235:00)<br>(C 07 D 495/14<br>C 07 D 333:00<br>C 07 D 249:00<br>C 07 D 243:00) |
| A | DE - A1 - 3 701 344 (BOEHRINGER INGELHEIM) * Ansprüche 1,4,8-13; Seite 20, Syntheseschema * -- | 1-5,7-10 | |
| A | DE - A1 - 3 624 779 (BEOHRINGER INGELHEIM) * Anspruch 1; Seite 5, Formelschema * ---- | 1-5,7-10 | |

RECHERCHIERTE SACHGEBIETE (Int. Cl.4)

C 07 D 495/00
C 07 C 255/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5,7-10
Unvollständig recherchierte Patentansprüche: -
Nicht recherchierte Patentansprüche: 6
Grund für die Beschränkung der Recherche:

Verfahren zur therapeutischen Behandlung oder tierischen Körpers; EPÜ Art. 52(4))

| Recherchenort | Abschlußdatum der Recherche | Prüfer |
|---|---|---|
| WIEN | 08-10-1990 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument